# EUROPEAN PATENT SPECIFICATION

(11) **EP 1 009 303 B1**
(45) Date of publication and mention of the grant of the patent: **10.06.2009**
(21) Application number: 98933279.6
(22) Date of filing: 08.07.1998
(51) Int. Cl.: A61B 18/04, A61N 5/04, A61N 7/02, A61B 17/22

(54) **CIRCUMFERENTIAL ABLATION DEVICE ASSEMBLY**
VORRICHTUNG ZUR ZIRKUMFERENZIELLEN ABLATION
DISPOSITIF D'ABLATION CIRCONFERENTIELLE

(30) Priority: 08.07.1997 US 889835; 03.02.1998 US 73527; 03.02.1998 US 73477; 08.07.1997 US 889798
(43) Date of publication of application: 21.06.2000
(73) Proprietor: The Regents of The University of California, Oakland, CA 94607-5200 (US); Atrionix, Inc., Palo Alto, CA 94303 (US); EMORY UNIVERSITY, Atlanta, GA 30322 (US)
(72) Inventor: DIEDERICH, Chris, J., Novato, CA 94945 (US); LESH, Michael, D., Mill Valley, CA 94941 (US); PEACOCK, James, C., III, San Carlos, CA 94070 (US); ROSS, Michael, Ronald, Hillsborough, CA 94010 (US); LANGBERG, Jonathan, J., Atlanta, GA 30306 (US)
(74) Representative: Fiener, Josef
(86) International application number: PCT/US1998/014220
(87) International publication number: WO 1999/002096

(56) References cited:
- EP-A- 0 472 368
- EP-A- 0 711 573
- US-A- 5 620 479
- US-A- 5 687 723

## Description

### TECHNICAL FIELD

The present invention is a surgical device. More specifically, it is a device assembly adapted to form a circumferential conduction block along a circumferential region of tissue located between a substantial portion of a pulmonary vein, such as a portion which includes an arrhythmogenic focus, and a substantial portion of a posterior left atrial wall, such as a portion including the other pulmonary veins.

### BACKGROUND

Many abnormal medical conditions in humans and other mammals have been associated with disease and other aberrations along the lining or walls which define several different body spaces. In order to treat such abnormal wall conditions of the body spaces, medical device technologies adapted for delivering specific forms of ablative energy to specific regions of targeted wall tissue from within the associated body space have been developed and disclosed.

The terms "body space," including derivatives thereof, is herein intended to mean any cavity or lumen within the body which is defined at least in part by a tissue wall. For example, the cardiac chambers, the uterus, the regions of the gastrointestinal tract, and the arterial or venous vessels are all considered illustrative examples of body spaces within the intended meaning.

The term "body lumen," including derivatives thereof, is herein intended to mean any body space which is circumscribed along a length by a tubular tissue wall and which terminates at each of two ends in at least one opening that communicates externally of the body space. For example, the large and small intestines, the vas deferens, the trachea, and the fallopian tubes are all illustrative examples of body lumens within the intended meaning. Blood vessels are also herein considered body lumens, including regions of the vascular tree between their branch points. More particularly, the pulmonary veins are body lumens within the intended meaning, including the region of the pulmonary veins between the branched portions of their ostia along a left ventricle wall, although the wall tissue defining the ostia typically presents uniquely tapered lumenal shapes.

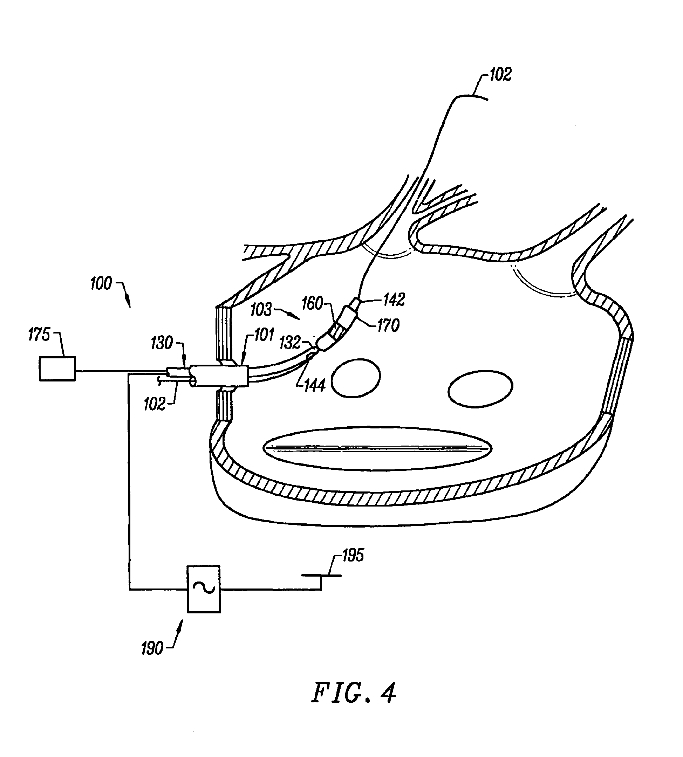

Atherosclerosis, a vascular disease characterized by abnormal deposits upon vessel walls or thickening thereof, is an example of an abnormal wall condition. The dangers related to flow blockages or functional occlusions resulting from the disease have made atherosclerosis the focus of many disclosed devices. Such devices as disclosed in EP-A-0 472 368 can be categorized by their structures and tissue treatment mechanisms. These categories may include direct contact electrode devices, resistance heating devices, light transmission/conversion to-heat devices, hot fluid lumen devices, and radio frequency (RF) heated devices. The catheter of EP-A-0 472 368 has a plurality of helically-shaped cutting wires that can be heated to enhance the cutting action and to melt the obstructing material. As shown in Figures 4-6, the catheter is first pushed beyond the obstruction, and then pulled back. While the catheter is pulled back, the cutting wires undercut the obstruction and separate the obstructing matter from the vessel. Therefore, while the pending application aims to alter the tissue properties of a tissue wall to form a conduction block along that tissue, the prior art catheter instead operates very differently to mechanically remove occlusive matter that resides within an inner lumen that is surrounded by a blood vessel wall.

Several direct (or nearly direct) contact electrode devices have been disclosed. U.S. Patent No. 4,998,933 to Eggers et al. describes a catheter designed for thermal angioplasty which utilizes a heated electrode in direct contact with surrounding tissue or plaque deposits as a mechanism for treating the diseased body lumen walls. U.S. Patent Nos. 4,676,258 to InoKuchi et al. and 4,807,620 to StruI et al. disclose devices designed to treat surrounding tissues using heat generated by two electrodes within the device and an RF power source.

U.S. Patent Nos. 4,672,962 to Hershenson and 5,035,694 to Kasprzyk et al. disclose devices which may be categorized as resistance heating probes, In each of these devices, current flowing through a conductive material at the end of the device provides heat which is transmitted to surrounding tissues for treatment of atherosclerosis and other diseases. Current is transmitted in each of these devices by electrically conductive materials. In contrast, U.S. Patent No. 5,226,430 to Spears et al. discloses a device which uses light transmitting fiber to transmit energy to a heat generating element at the tip of the device. The heat generating element in that device transmits heat energy to a surrounding balloon structure which is in contact with surrounding tissue. In further contrast, U.S. Patent No. 4,790,311 to Ruiz discloses an angioplasty catheter system wherein a heat generating electrode, at the tip of the device is heated using the transmission of RF energy. This device may be categorized as an RF heated device.

U.S. Patent Nos. 5,190,540 and 5,292.321 to Lee can be categorized as hot fluid lumen devices. In the 540 disclosure, Lee describes a balloon catheter designed for remodeling a body lumen. This device utilizes a multi-lumen catheter which is capable of delivering heated fluid to an expandable balloon lumen, thereby expanding the balloon geometrically and heating tissue which is in contact with the balloon. In the '321 disclosure, Lee describes a lumen of an expandable balloon is filled with thermoplastic material which is designed to become softer and more moldable when heated by a heating element.

Endometriosis, another abnormal wall tissue condition, is associated with the endometrial cavity of the female. This medical condition, characterized by dangerously proliferative uterine wall tissue along the surface of the endometrial cavity, has been treated by delivering energy to the tissue. U.S. Patent No. 5,449,380 to Chin discloses a medical device for delivering energy to the wall tissue of a diseased endometrial cavity using a balloon lumen with heated fluid circulating therein. Other devices, such as those disclosed in U.S. Patent Nos. 5,505,730 to Edwards; 5,558,672 to Edwards et al. and 5,562,720 to Stem et al. are intended to treat particular tissues using heat generated by the flow of RF current between electrodes.

Diseased or structurally damaged blood vessels may.bring about various abnormal wall conditions. The inducement of thrombosis and control of hemorrhaging within certain body lumens such as vessels have been the focus of several disclosed devices which use catheter-based heat sources for cauterizing damaged tissues. In U.S. Patent No. 4,449,528, for example, Auth et al. disclose a thermal cautery probe designed for heating specific layers of tissue without producing deep tissue damage. The mechanism of heat generation in this device is a resistive coil within the cautery probe which is electrically connected to a power source. In U.S. Patent No. 4,662,368, Hussein et al. disclose a device designed for localized heat application within a body lumen. In this device, energy for heat generation is delivered to the tip of the device in the form of light by a flexible fiber. Heat from an element which converts light energy to heat energy is then conducted to the adjacent tissue. In U.S. Patent No. 4,522,205, Taylor et al. disclose a device designed for inducing thrombosis in a blood vessel comprising an array of electrodes mounted onto an expandable balloon which may be delivered by a catheter such that direct current flows through electrodes which are in contact with adjacent tissues in order to precipitate thrombosis.

Maintenance of patency in diseased body lumens such as blood vessels has been the focus of several disclosed devices, such as for example cardiovascular stent devices. U.S. Patent No. 5,078,736 to Behl, for example, discloses an apparatus for maintaining patency in the body passages comprising a stent structure which may be connected to a radiofrequency power source. In addition to mechanically supporting a body lumen, this device is intended to provide for thermal disruption of the adjacent tissues in order to inhibit reocclusion of the body lumen. U.S. Patent No. 5,178,618 to Kandarpa discloses a device which is intended to recanalize an occluded vessel prior to mechanically supporting a body lumen region.

In addition to the references just described above, other examples of devices and methods which are intended to ablate tissues within various body spaces are disclosed in the following additional references: U.S. Patent No. 5,295,484 to Marcus et al. ; U.S. Patent No. 5,324,255 to Passafaro et al. ; U.S. Patent No. 5,391,197 to Burdette et al. ; U.S. Patent No. 5,447,509 to Mills et al. ; U.S. Patent No. 5,474,530 to Passafaro et al. ; U.S. Patent No. 5,571,088 to Lennox et al. ; U.S. Patent No. 5,575,772 to Lennox ; U.S. Patent No. 5,630,837 to Crowley; U.S. Patent No. 5,606,974 to Castellano et al.; and U.S Patent No. 5,620,479 to Diederich.

### Atrial Fibrillation

Cardiac arrhythmias, and atrial fibrillation in particular, persist as common and dangerous medical ailments, especially in the aging population. In patients with normal sinus rhythm, the heart, which is comprised of atrial, ventricular, and excitatory conduction tissue, is electrically excited to beat in a synchronous, patterned fashion. In patients with cardiac arrhythmia, abnormal regions of cardiac tissue do not follow the synchronous beating cycle associated with normally conductive tissue in patients with sinus rhythm. Instead, the abnormal regions of cardiac tissue aberrantly conduct to adjacent tissue, thereby disrupting the cardiac cycle into an asynchronous cardiac rhythm. Such abnormal conduction has been previously known to occur at various regions of the heart, such as, for example, in the region of the sino-atrial (SA) node, along the conduction pathways of the atrioventricular (AV) node and the Bundle of His, or in the cardiac muscle tissue forming the walls of the ventricular and atrial cardiac chambers.

Cardiac arrhythmias, including atrial arrhythmia, may be of a multiwavelet reentrant type, characterized by multiple asynchronous loops of electrical impulses that are scattered about the atrial chamber and are often self propagating. In the alternative or in addition to the multiwavelet reentrant type, cardiac arrhythmias may also have a focal origin, such as when an isolated region of tissue in an atrium fires autonomously in a rapid, repetitive fashion. Cardiac arrhythmias, including atrial fibrillation, may be generally detected using the global technique of an electrocardiogram (EKG). More sensitive procedures of mapping the specific conduction along the cardiac chambers have also been disclosed, such as, for example, in US Patents Nos. 4,641,649 to Walinsky et al. and WO 96/32897 to Desai.

A host of clinical conditions may result from the irregular cardiac function and resulting hemodynamic abnormalities associated with atrial fibrillation, including stroke, heart failure, and other thromboembolic events. In fact, atrial fibrillation is believed to be a significant cause of cerebral stroke, wherein the abnormal hemodynamics in the left atrium caused by the fibrillatory wall motion precipitate the formation of thrombus within the atrial chamber. A thromboembolism is ultimately dislodged into the left ventricle, which thereafter pumps the embolism into the cerebral circulation where a stroke results. Accordingly, numerous procedures for treating atrial arrhythmias have been developed, including pharmacological, surgical, and catheter ablation procedures.

### Conventional Atrial Arrhythmia Treatments

Several pharmacological approaches intended to remedy or otherwise treat atrial arrhythmias have been disclosed, such as, for example, in US Patent No. 4,673,563 to Berne et al.; US Patent No. 4,569,801 to Molloy et al.; and also by Hindricks, et al. in "Current Management of Arrhythmias" (1991). However, such pharmacological solutions are not generally believed to be entirely effective in many cases, and may in some cases result in proarrhythmia and long term inefficacy.

Several surgical approaches have also been developed with the intention of treating atrial fibrillation. One particular example is known as the "maze procedure," as is disclosed by Cox, JL et al. in "The surgical treatment of atrial fibrillation. I. Summary" Thoracic and Cardiovascular Surgery 101(3), pp. 402-405 (1991); and also by Cox, JL in "The surgical treatment of atrial fibrillation. IV. Surgical Technique", Thoracic and Cardiovascular Surgery 101(4), pp. 584-592 (1991). In general, the "maze" procedure is designed to relieve atrial arrhythmia by restoring effective atrial systole and sinus node control through a prescribed pattern of incisions about the tissue wall. In the early clinical experiences reported, the "maze" procedure included surgical incisions in both the right and the left atrial chambers. However, more recent reports predict that the surgical "maze" procedure may be substantially efficacious when performed only in the left atrium, such as is disclosed in Sueda et al., "Simple Left Atrial Procedure for Chronic Atrial Fibrillation Associated With Mitral Valve Disease". The Annals of Thoracic Surgery 62(6), pp. 1796-1800 (1996).

The "maze procedure" as performed in the left atrium generally includes forming vertical incisions from the two superior pulmonary veins arid terminating in the region of the mitral valve annulus, traversing the inferior pulmonary veins en route. An additional horizontal line also connects the superior ends of the two vertical incisions. Thus, the atrial wall region bordered by the pulmonary vein ostia is isolated from the other atrial tissue. In this process, the mechanical sectioning of atrial tissue eliminates the precipitating conduction to the atrial arrhythmia by creating conduction blocks within the aberrant electrical conduction pathways.

While the "maze" procedure as reported by Cox and others, and also other surgical procedures, have met some success in treating patients with atrial arrhythmia, its highly invasive methodology is believed to be prohibitive in most cases. However, these procedures have provided a guiding principle that mechanically isolating faulty cardiac tissue may successfully prevent atrial arrhythmia, and particularly atrial fibrillation caused by perpetually wandering reentrant wavelets or focal regions of arrhythmogenic conduction.

### Modern Catheter Treatments for Atrial Arrhythmia

Success with surgical interventions through atrial segmentation, particularly with regard to the surgical "maze" procedure just described, has inspired the development of less invasive catheter-based approaches to treat atrial fibrillation through cardiac tissue ablation. Examples of such catheter-based devices and treatment methods have generally targeted atrial segmentation with ablation catheter devices and methods adapted to form linear or curvilinear lesions in the wall tissue which defines the atrial chambers, such as are disclosed in the following US Patents: US Patent No. 5,617,854 to Munsif; US Patent No. 4,898,59I to Jang et al.; US Patent No. 5,487,385 to Avitall; and US Patent No. 5,582,609 to Swanson.

Additional examples of catheter-based tissue ablation in performing less-invasive cardiac chamber segmentation procedures are also disclosed in the following articles: "Physics and Engineering of Transcatheter Tissue Ablation", Avitall et al., Journal of American College of Cardiology, Volume 22, No. -3:921-937 (1993); and "Right and Left Atrial Radiofrequency Catheter Therapy of Paroxysmal Atrial Fibrillation," Haissaguerre, et al., Journal of Cardiovascular Electrophysiology 7(12), pp. 1132-1144 (1996).

Furthermore, the use of particular guiding sheath designs for use in ablation procedures in both the right and/or left atrial chambers are disclosed in US Patents Nos. 5,427,119; 5,497,119; 5,564,440; 5,575,766 to Swartz et al.. In addition, various energy delivery modalities have been disclosed for forming such atrial wall lesions, and include use of microwave, laser, and more commonly, radiofrequency energies to create conduction blocks along the cardiac tissue wall, as disclosed in US Patent Nos. WO 93/20767 to Stem et al.; 5,104,393 to Isner et al.; and 5,575,766 to Swartz et al, respectively.

In addition to attempting atrial wall segmentation with long linear lesions for treating atrial arrhythmia, ablation catheter devices and methods have also been disclosed which are intended to ablate arrhythmogenic tissue of the left-sided accessory pathways, such as those associated with the Wolff Parkinson-White syndrome, through the wall of an adjacent region along the coronary sinus.

For example, Fram et al., in "Feasibility of RF Powered Thermal Balloon Ablation of Atrioventricular Bypass Tracts via the Coronary Sinus: In vivo Canine Studies," PACE, Vol. 18, p 1518-1530 (1995), disclose attempted thermal ablation of left-sided accessory pathways in dogs using a balloon which is heated with bipolar radiofrequency electrodes positioned within the balloon. A 10 French (3.3 mm) guiding catheter and a 0.035" (0.889 mm) wire were provided in an assembly adapted to advance the ablation catheter into the coronary sinus from the jugular vein. Thermal ablation procedures were performed in the posterospetal coronary sinus and in the left free-wall coronary sinus with thermal inflations at either 70deg, 80deg, or 90deg for either 30 or 60 seconds. In all cases balloon occlusion was confirmed using distal dye injection. A compliant silicone balloon was used which had a diameter range of 5-20mm and a length range of 8-23mm over a final inflation pressure range of 0.4 to 1.5 atms (4.053×10⁴ Pa-15.199×10⁴ Pa). Fram et al. discloses that the lesion depth of some population groups may be sufficient to treat patients with Wolff-Parkinson-White syndrome.

Additional examples of cardiac tissue ablation from the region of the coronary sinus for the purpose of treating particular types of cardiac arrhythmias are disclosed in: "Long-term effects of percutaneous laser balloon ablation from the canine coronary sinus", Schuger CD et al., Circulation (1992) 86:947-954; and "percutaneous laser balloon coagulation of accessory pathways", McMath LP et al., Diagn Ther Cardiovasc Interven 1991; 1425:165-171.

### Focal Arrhythmias Originating from Pulmonary Veins

Certain particular modes of atrial fibrillation are believed to be focal in nature, caused by the rapid and repetitive firing of an isolated center within the atrial cardiac muscle tissue. These foci may act as either a trigger of atrial fibrillation or may sustain the fibrillation. Recent studies have suggested that focal arrhythmia often originates from a tissue region along the pulmonary veins extending from the left atrium, and even more particularly in the superior pulmonary veins.

Less-invasive percutaneous catheter ablation techniques have been disclosed which use end-electrode catheter designs with the intention of ablating and thereby treating focal arrhythmias in the pulmonary veins. These ablation procedures are typically characterized by the incremental application of electrical energy to the tissue to form focal lesions designed to ablate the focus and thereby terminate the focal trigger of arrhythmia.

One example of a focal ablation method intended to destroy such arrhythmogenic foci and thereby treat focal arrhythmia originating from a pulmonary vein is disclosed by Haissaguerre, et al. in "Right and Left Atrial Radiofrequency Catheter Therapy of Paroxysmal Atrial Fibrillation" in Journal of Cardiovascular Electrophysiology 7(12), pp. 1132-1144 (1996). Haissaguerre, et al. disclose radiofrequency catheter ablation of drug-refractory paroxysmal atrial fibrillation using linear atrial lesions complemented by focal ablation targeted at arrhythmogenic foci in a screened patient population. The site of the arrhythmogenic foci were generally located just inside the superior pulmonary vein, and were ablated using a standard 4mm tip single ablation electrode.

In another focal ablation example, Jais et al. in "A focal source of atrial fibrillation treated by discrete radiofrequency ablation" Circulation 95:572-576 (1997) applies an ablative technique to patients with paroxysmal arrhythmias originating from a focal source. At the site of arrhythmogenic tissue, in both right and left atria, several pulses of a discrete source of radiofrequency energy were applied in order to eliminate the fibrillatory process.

There is still a need for a circumferential ablation device assembly and method adapted to electrically isolate a substantial portion of a posterior left atrial wall from an arrhythmogenic focus along a pulmonary vein. In particular there is still a need for such an assembly and method which provides a circumferential ablation member secured to the distal end of an elongate catheter body and which includes an ablation element adapted to form a circumferential conduction block along a circumferential region of tissue which either includes the arrhythmogenic focus or is between the arrthythmogenic focus and the substantial portion of the posterior left atrium wall.

There is also still a need for a circumferential ablation device assembly and method for forming a circumferential conduction block which is adapted to couple an ablation element to a circumferential region of tissue while substantially isolating ablative energy from the ablation element to the circumferential region of tissue by shielding adjacent regions of tissue adjacent to the circumferential region from coupling to the ablation element.

There is also still a need for an ablation device assembly and method adapted to connect multiple linear lesions that are each formed along regions of tissue extending between a common pulmonary vein and multiple other pulmonary veins adjacent to the common pulmonary vein in a less-invasive "maze"-type procedure for treating atrial arrhythmias.

There is also still a need for a tissue ablation device assembly with an ablation catheter assembly having an elongate body with an anchor adjacent to a linear ablation element, wherein the anchor is formed by a stylet slideably engaged within a stylet passageway along the elongate body such that the stylet when advanced within the stylet passageway to a predetermined location is adapted to push a portion of the elongate body adjacent to the linear ablation element into a pulmonary vein through its ostium along the posterior left atrial wall.

There is also still a need for a tissue ablation device assembly and a corresponding kit which provides a circumferential ablation member which may be selectively chosen from a kit of ablation, members based upon a measured diameter of a pulmonary vein in order to ablate a particular circumferential region of tissue between a substantial portion of the pulmonary vein and a substantial portion of a posterior left atrial wall.

### SUMMARY OF THE INVENTION

This object is achieved by a tissue ablation device assembly as defined in claim 1.

A circumferential ablation device assembly is adapted to form a circumferential conduction block which electrically isolates a substantial portion of a posterior left atrial wall of a left atrium in a patient from an arrhythmogenic focus located along a pulmonary vein that extends from the posterior left atrial wall at a pulmonary vein ostium. The assembly includes a circumferential ablation device having an elongate body and a circumferential ablation member secured to the distal end portion of the elongate body. The circumferential ablation member includes an ablation element which is adapted to couple to and ablate a circumferential region of tissue that either includes the arrhythmogenic focus along the pulmonary vein, or which is located between the arrhythmogenic focus along the pulmonary vein.

According to one mode of the assembly, a kit of multiple such circumferential ablation devices is provided with each circumferential ablation device in the kit adapted to ablate a circumferential region of tissue which circumscribes a space having a unique diameter. A particular one of the circumferential ablation devices may be chosen for use in forming a circumferential conduction block in relation to a particular pulmonary vein in a patient based upon reviewing a measured diameter associated with the circumferential region of tissue desired to be ablated.

In one aspect of this mode, the particular circumferential ablation device is chosen based upon a measured diameter from an X-ray or fluoroscopic view of the circumferential region. In another aspect of this mode, the particular circumferential ablation device is chosen based upon a transesophageal ultrasound view of the circumferential region.

According to another mode of the assembly, the circumferential ablation member includes an expandable member along the distal end portion of the elongate body and that is adjustable from a radially collapsed position to a radially expanded position such that an outer surface along the working length contacts or engages the circumferential region of tissue to be ablated. The circumferential ablation element is coupled to the outer surface and also to the circumferential region of tissue in order to ablate the tissue.

In one aspect of this mode, the working length is adapted to conform to a pulmonary vein ostium when adjusted from a radially collapsed position to a radially expanded position.

In one further embodiment of this aspect, the working length when expanded includes a taper with a distally reducing outer diameter from a proximal region to a distal region. In one beneficial variation of this embodiment, the taper is pear-shaped with a contoured surface between the proximal and distal regions.

In another embodiment of this aspect, the expandable member is radially compliant and is adapted to conform to the pulmonary vein ostium when the working length is expanded to the radially expanded position in the left atrium. In one beneficial variation of this embodiment, the expandable member is adapted to conform to the pulmonary vein ostium by expanding it to the expanded position within the atrium and thereafter forcing the member retrogradedly into the pulmonary vein and against the pulmonary vein ostium.

In another mode of the assembly, the circumferential ablation member includes first and second end portions that border opposite sides of a middle region along the elongate body relative to the longitudinal axis. The ablation element is coupled to the middle region, and the first and second end portions are adapted to substantially isolate ablative energy from the ablation element and to the circumferential region of tissue by substantially shielding surrounding tissue from coupling to the ablation element.

A further mode of the assembly combines the expandable member mode and isolated coupling modes just described for the circumferential ablation member and ablation element. The expandable member includes a working length with first and second end portions and a circumferential band which circumscribes an outer surface of the working length between the first and second end portions. When the expandable member is in the radially expanded position, the circumferential band is adapted to contact or engage the circumferential region of tissue whereas the first and second end portions are adapted to engage adjacent regions of tissue bordering either side of the circumferential region of tissue. The ablation element is adapted to couple to the circumferential band in the expanded position to thereby form the middle region for ablating the circumferential region of tissue, whereas the first and second end portions or shields are adapted to isolate ablative energy from the ablation element to the circumferential region of tissue adjacent to the circumferential band.

In one aspect of this mode, the circumferential band has a length which is substantially shorter than the working length of the expandable member, and may be less than two-thirds or even one-half the working length of the expandable member.

In another aspect of this mode, the working length is adjustable between a plurality of radially expanded positions each having a different expanded outer diameter in the region of the circumferential band. The circumferential band of the ablation element is adapted to ablate a continuous circumferential lesion pattern in tissue surrounding the circumferential band over the range of expanded outer diameters. In one mode of this variation, the circumferential band has a secondary shape along the outer surface of the working length, such as a modified step, serpentine, or sawtooth shape.

In another aspect of this mode, the ablation element includes an RF electrode in an RF ablation circuit. In a particular variation of this aspect, the ablation electrode includes a porous membrane along the equatorial or other circumferential band which is adapted to pass electrically conductive fluid from the conductive fluid chamber formed by the expandable member and into tissue adjacent to the band, the fluid conducting current to the tissue in an RF ablation circuit.

In another aspect of this mode, a thermal conductor is coupled to the circumferential band and is adapted to emit thermal energy into the circumferential region of tissue adjacent to the circumferential band.

In another aspect of this mode, the ablation element includes an ultrasound transducer which is ultrasonically coupled to the circumferential band and also to the circumferential region of tissue adjacent to the circumferential band.

In one beneficial embodiment of this aspect, the ultrasound transducer is fixed to the distal end portion of the elongate body and within the expandable member along the circumferential band and is further adapted to emit a circumferential pattern of ultrasonic energy which couples to the circumferential band to thereby ablate circumferential region of tissue adjacent thereto.

In one variation of this ultrasound embodiment, the ablation element further includes a thermal conductor along the circumferential band which is adapted to absorb the ultrasonic energy from the transducer and to thereby heat and conduct thermal energy into the circumferential region of tissue.

In another detailed variation of the ultrasound embodiment, the ultrasound transducer is also adapted to ultrasonically couple to first and second end portions bordering either side of the circumferential band along the working length, which end portions are adapted to engage adjacent regions of tissue bordering opposite sides of the circumferential region of tissue to be ablated. Ultrasonic insulators are provided over the end portions in order to form shields to isolate the transmission of ultrasonic energy from the ultrasound transducer to the circumferential region of tissue adjacent to the circumferential band.

In another aspect of this mode, the ablation element is coupled to a substantial portion of the working length of the expandable member including the first and second end portions in addition to the circumferential band. According to this aspect, however, insulators are provided along the first and second end regions to thereby form the first and second shields adapted to prevent the ablation element from coupling to and ablating the adjacent regions of tissue. The insulators leave only the circumferential band unshielded to form the middle region which is thereby adapted to ablate the circumferential region of tissue.

In another mode of the assembly, the circumferential ablation member is adapted such that the ablation element couples to and ablates a circumferential region of tissue in a left posterior atrial wall which surrounds a pulmonary vein ostium to thereby isolate the substantial portion of the posterior left atrial wall.

In one aspect of this mode, the circumferential ablation member includes an expandable member with a tapered outer surface adapted to conform to the pulmonary vein ostium such that a circumferential band that circumscribes the outer surface is adapted to engage the circumferential region of tissue surrounding the pulmonary vein ostium. The ablation element is adapted to couple to the circumferential band and also to the circumferential region of tissue in order to ablate the tissue.

According to another mode of the assembly, the distal end portion of the elongate member includes a circumferential ablation member such as according to the modes just described, and also includes a linear ablation member having an elongate ablation element length and linear ablation element which is adapted to form a continuous linear lesion in tissue adjacent thereto when the linear ablation element is coupled to an ablation actuator.

In one aspect of this mode, the circumferential ablation member includes an expandable member such as according to the modes just described and which forms a first anchor adapted to secure a first linear ablation member end at a first location at or adjacent to a pulmonary vein ostium along a left atrium wall. A second anchor is also provided adjacent to a second, opposite end of the linear ablation member end and is adapted to secure the second linear ablation member end to a second location along the left atrium wall.

In one beneficial variation of this aspect, the second anchor is formed by a stylet slideably engaged within a stylet passageway along the elongate body and which is adapted to push the elongate body into another pulmonary vein, wherein the second location is at or adjacent to the second pulmonary vein.

A tissue ablation device assembly is also provided that includes an ablation catheter having an elongate body with an anchor adjacent to a linear ablation element, wherein the anchor is formed by a stylet slideably engaged within a stylet passageway along the elongate body such that the stylet when advanced within the stylet passageway to a predetermined location is adapted to push a portion of the elongate body adjacent to the linear ablation element into a pulmonary vein through its ostium along the posterior left atrial wall.

In one aspect of this mode, the assembly includes two anchors each adjacent to opposite end portions of the linear ablation element. A first anchor is adjacent to a distal end of the linear ablation element and is adapted to engage a first pulmonary vein. The anchor formed by the stylet is a second anchor adjacent a proximal end of the linear ablation element and is adapted to engage a second pulmonary vein. In one embodiment of this aspect, an expandable member is provided along the distal end portion of the elongate body distally adjacent the distal end of the linear ablation element and is adapted to radially engage the pulmonary vein wall when expanded. In one beneficial variation of this embodiment, the expandable member forms in part a circumferential ablation member such as according to the particular modes just described.

Another tissue ablation device assembly is also provided which includes an elongate body with a linear ablation element secured to its distal end portion that is adapted to ablate a region of tissue extending between first and second locations along a posterior left atrial wall. An anchor is located along the distal end portion adjacent to a distal end portion of the linear ablation element and includes a guidewire tracking member adapted to slideably engage and track over a guidewire positioned within a pulmonary vein through its ostium, thereby securing the distal end of the linear ablation element to a first location along the posterior left atrial wall. A stylet is engaged within a stylet passageway along the elongate body such that the stylet when advanced within the stylet passageway is adapted to secure the proximal end portion of the linear ablation element to a second predetermined location along the posterior left atrial wall.

### BRIEF DESCRIPTION OF THE DRAWINGS

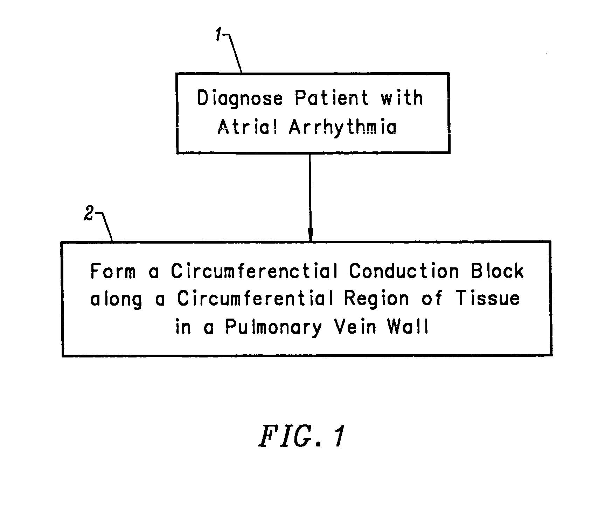

Figure 1 diagrammatically shows sequential, general steps for treating atrial arrhythmia according to the method of using the present invention.

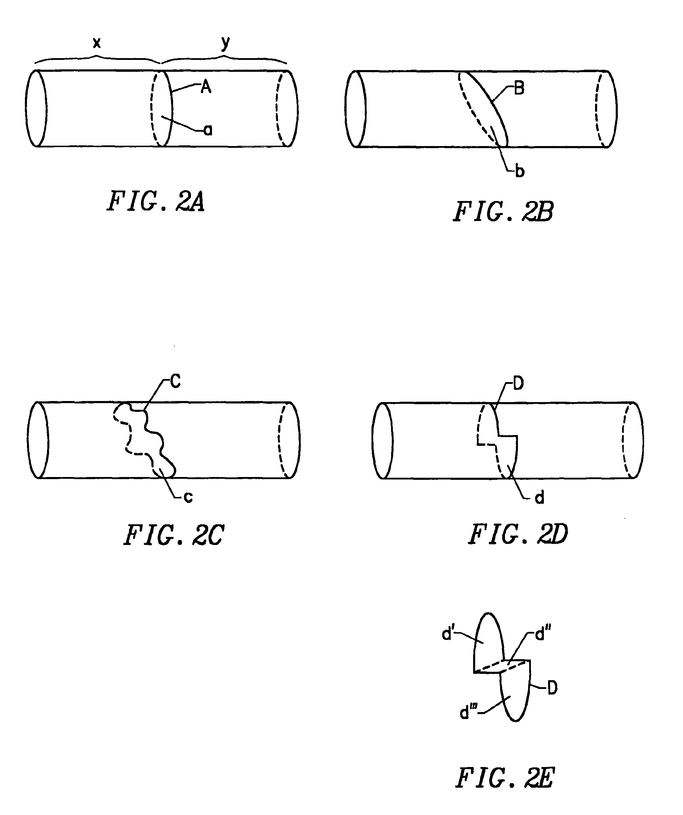

Figures 2A-E show schematic, perspective views of various exemplary circumferential conduction blocks formed in pulmonary vein wall tissue with the circumferential ablation device assembly of the present invention.

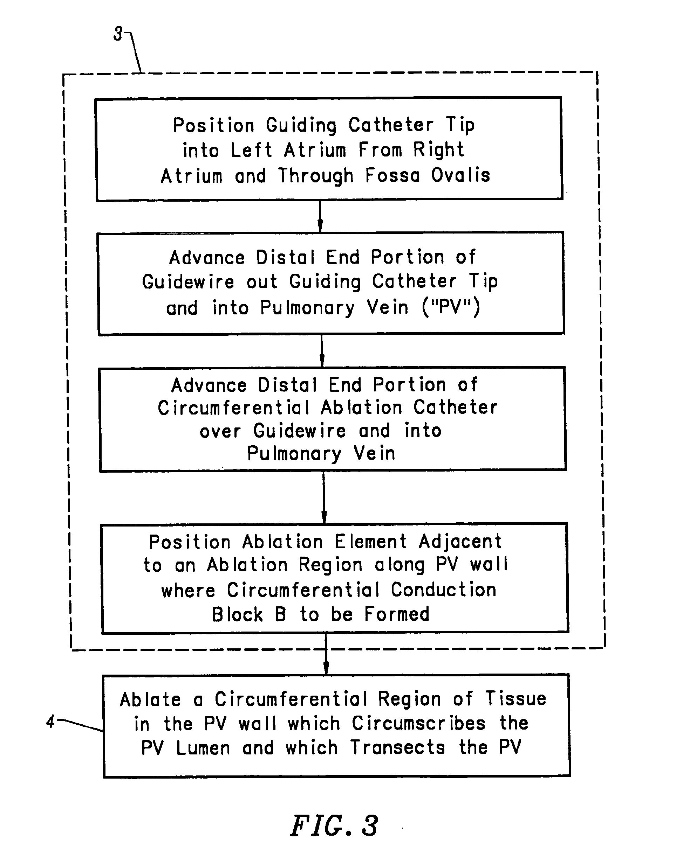

Figure 3 shows a flow diagram of a method for using the circumferential ablation device assembly of the present invention.
Figure 4 shows a perspective view of a circumferential ablation device assembly during use in a left atrium subsequent to performing transeptal access and guidewire positioning steps according to the method of Figure 3.

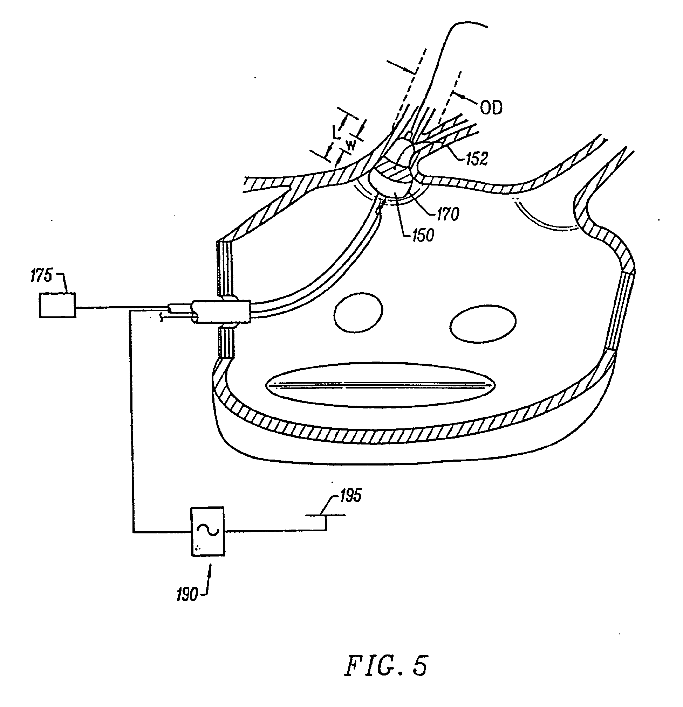

Figure 5 shows a similar perspective view of the circumferential ablation device assembly shown in Figure 4, and further shows a circumferential ablation catheter during use in ablating a circumferential region of tissue along a pulmonary vein wall to form a circumferential conduction block in the pulmonary vein according to the method of Figure 3.

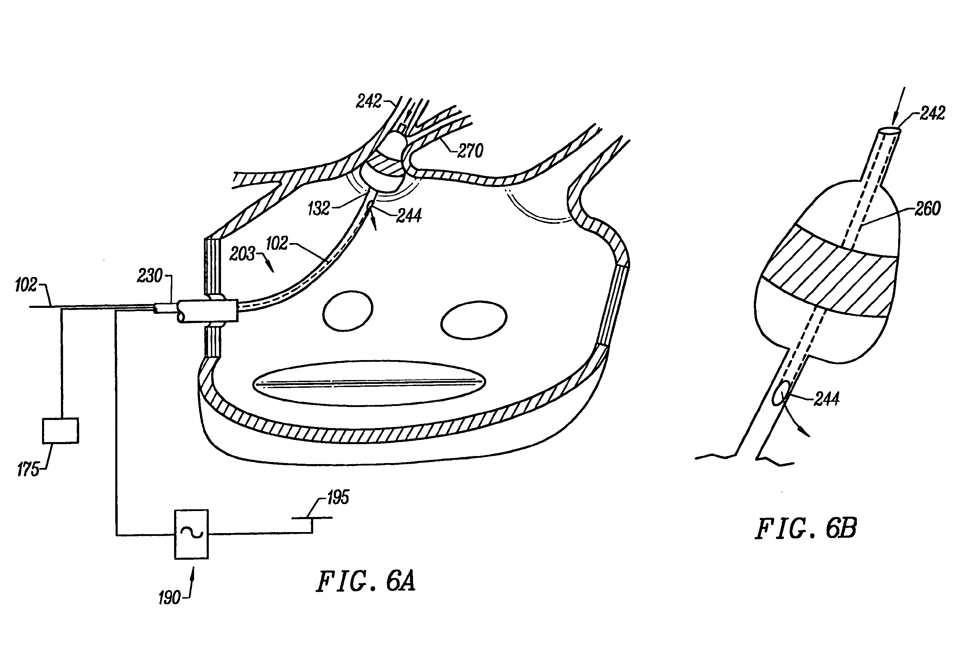

Figure 6A shows a similar perspective view as shown in Figure 5, although showing a further circumferential ablation catheter variation which is adapted to allow for blood perfusion from the pulmonary vein and into the atrium while performing the circumferential ablation method shown diagramatically in Figure 3.
Figure 6B is an enlarged partial view of the circumferential ablation catheter shown in Figure 6A, with a perfusion lumen shown in phantom.

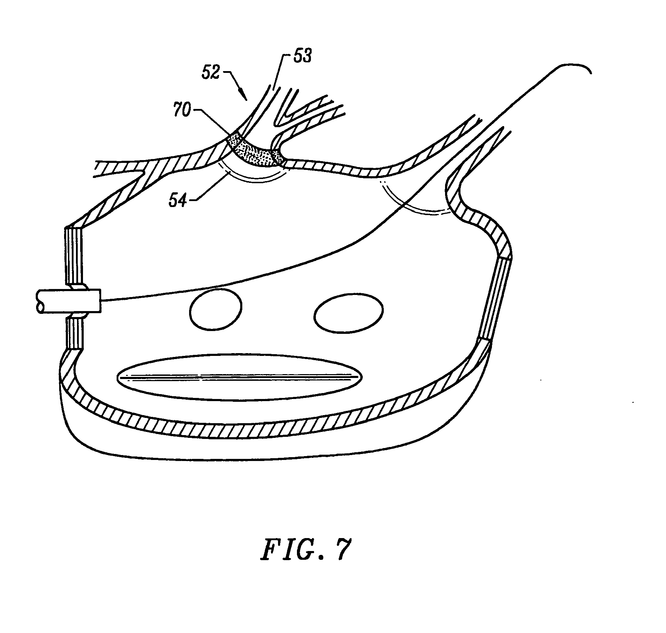

Figure 7 shows a similar perspective view of the left atrium as that shown in Figures 3-5, although showing a cross-sectional view of a circumferential lesion after being formed by circumferential catheter ablation according to the method of Figure 3.

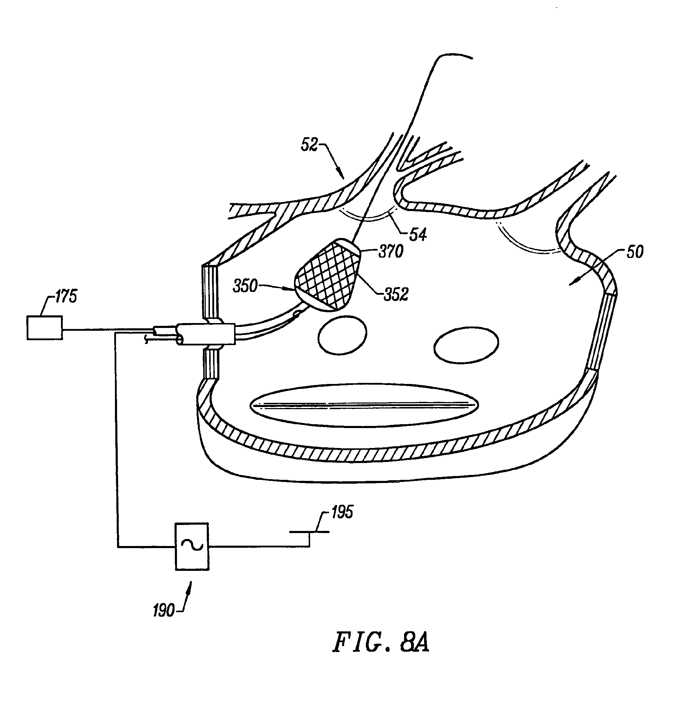

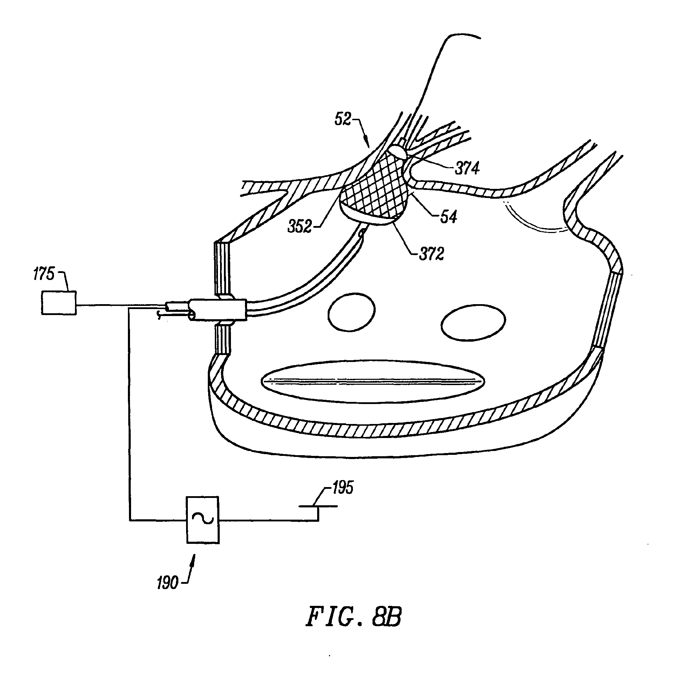

Figures 8A-B show perspective views of another circumferential ablation catheter variation during use in a left atrium according to the method of Figure 3, wherein Figure 8A shows a radially compliant expandable member with a working length adjusted to a radially expanded position while in the left atrium, and Figure 8B shows the expandable member after advancing it into and engaging a pulmonary vein ostium while in the radially expanded position.

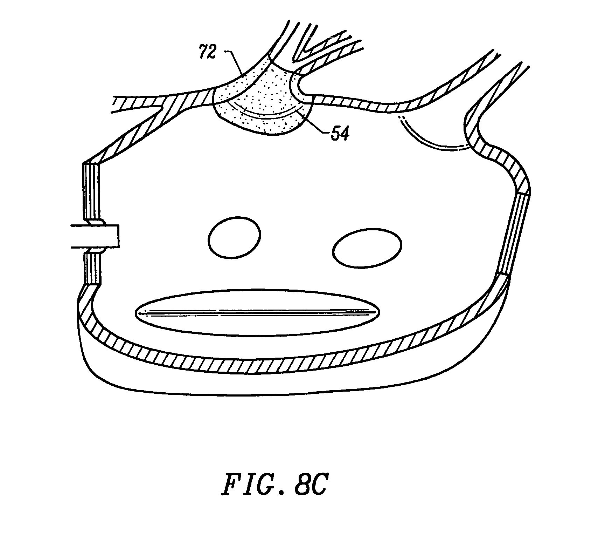

Figure 8C shows the same perspective view of the left atrium shown in Figures 8A-B, although shown after forming a circumferential conduction block according to the circumferential ablation procedure of Figure 3 and also after removing the circumferential ablation device assembly from the left atrium.

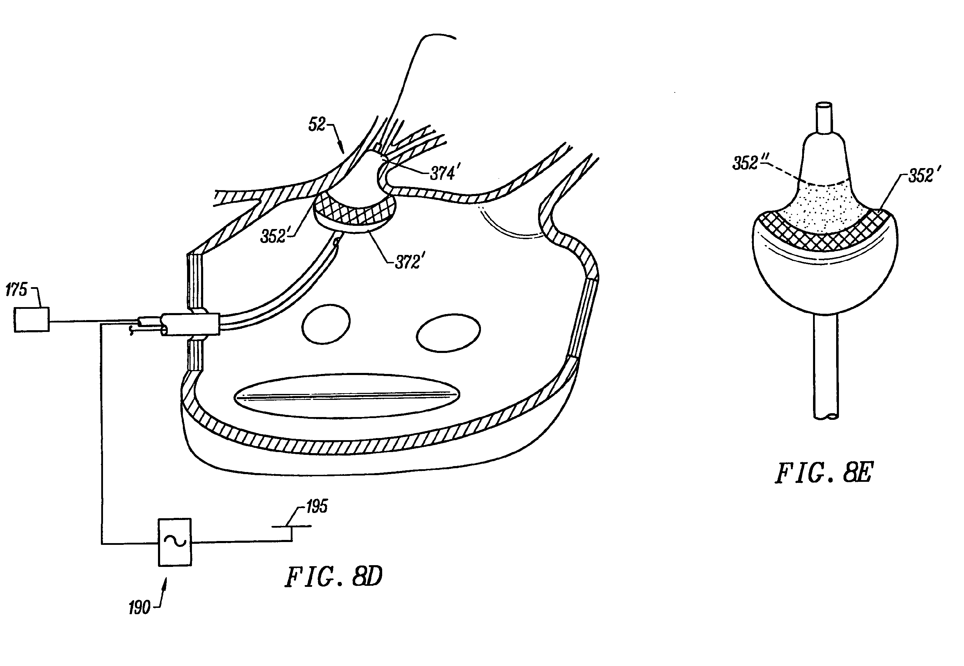

Figure 8D shows another circumferential ablation catheter during use in a left atrium, and shows an expandable member in a radially expanded position which is engaged within a pulmonary vein ostium such that a circumferential band of a circumferential ablation element circumscribing the expandable member is also engaged to a circumferential path of tissue along the left posterior atrial wall which surrounds the pulmonary vein ostium.
Figure 8E shows one particular expandable member and circumferential ablation element which is adapted for use according to the mode of use shown in Figure 8D.

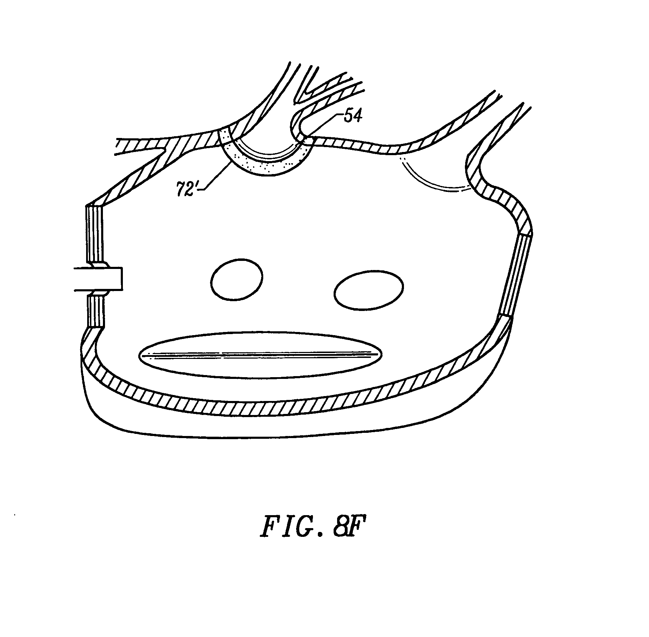

Figure 8F shows a resulting circumferential conduction block or lesion which may be formed with the assemblies shown in Figures 8D-E and according to the method of use shown in Figure 8D.

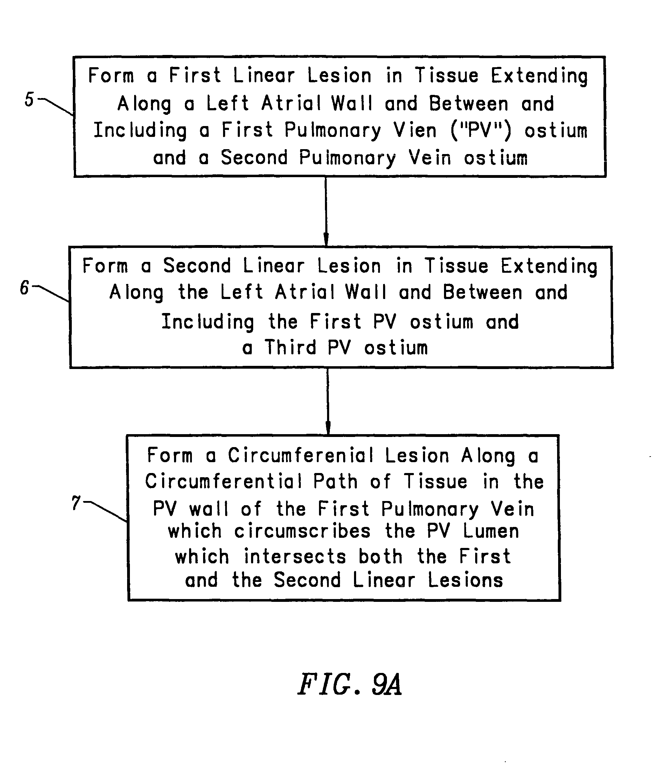

Figure 9A diagrammatically shows a method for using the circumferential ablation device assembly of the present invention by forming a circumferential conduction block in a pulmonary vein in combination with a method for forming long linear lesions between pulmonary vein ostia in a less-invasive "maze"-type procedure.

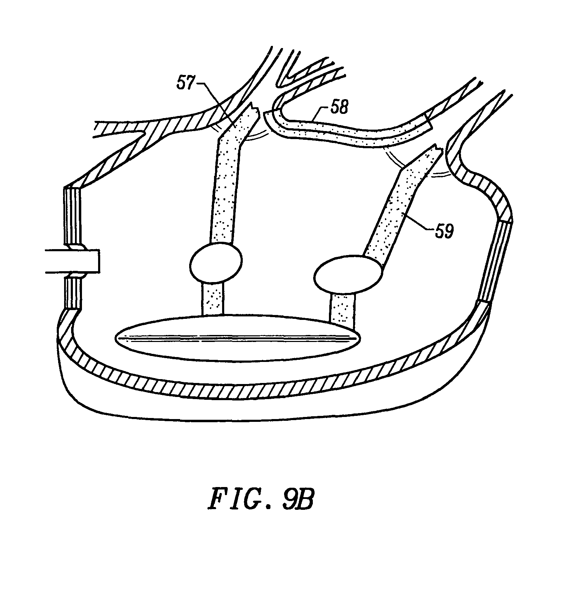

Figure 9B shows a perspective view of a segmented left atrium after forming several long linear lesions between adjacent pairs of pulmonary vein ostia according to the method of Figure 9A.

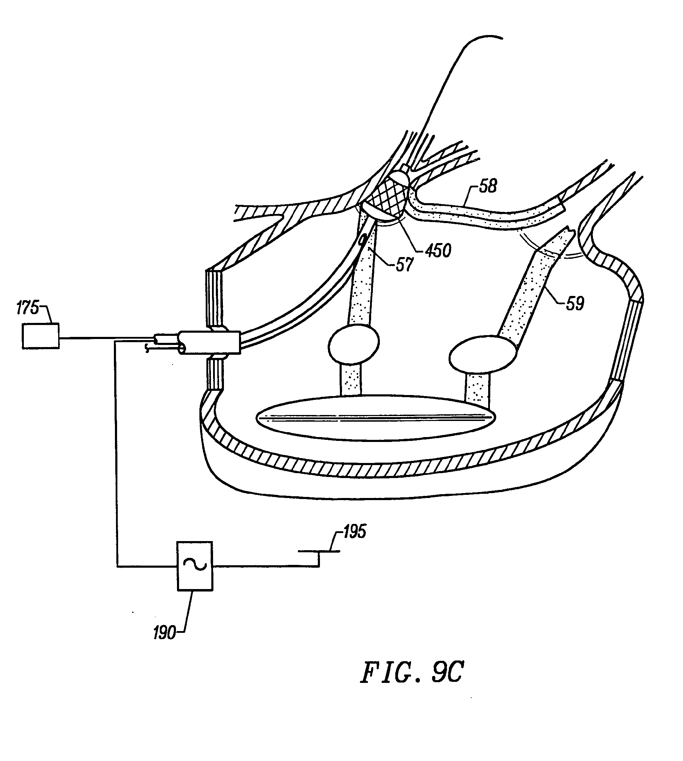

Figure 9C shows a similar perspective view as that shown in Figure 9B, although showing a circumferential ablation device assembly during use in forming a circumferential lesion in a pulmonary vein which intersects with two linear lesions that extend into the pulmonary vein, according to the method of Figure 9A.

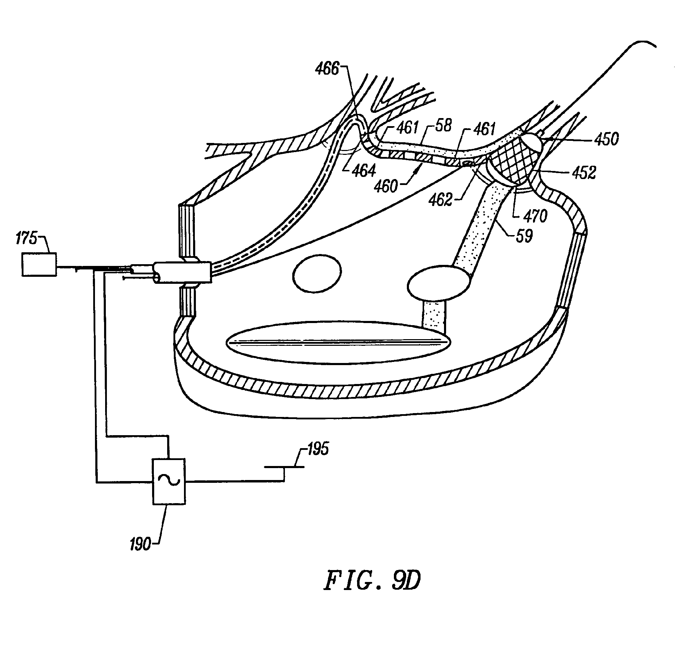

Figure 9D shows a perspective view of another ablation catheter which combines a linear ablation member extending between two anchors with a circumferential ablation member for use in forming a circumferential lesion which intersects with at least one linear lesion according to the method of Figure 9A.

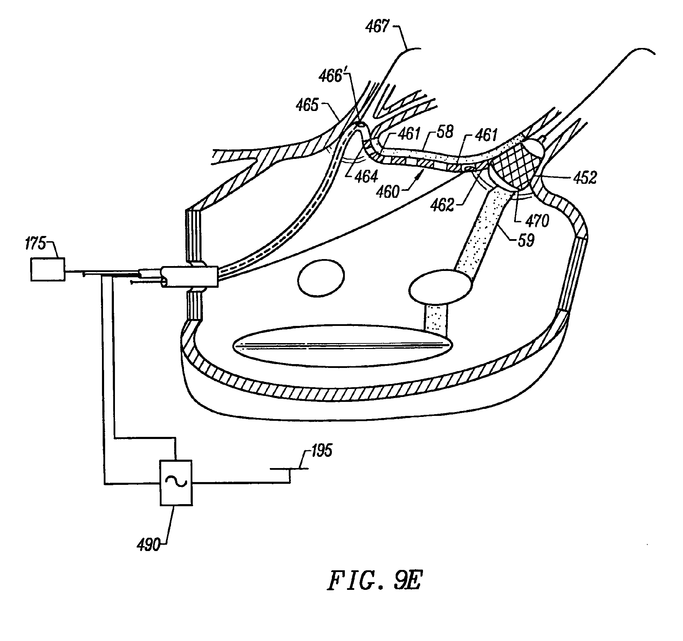

Figure 9E shows a perspective view of another circumferential ablation catheter for use in forming a circumferential lesion which intersects with at least one linear lesion according to the method of Figure 9A.

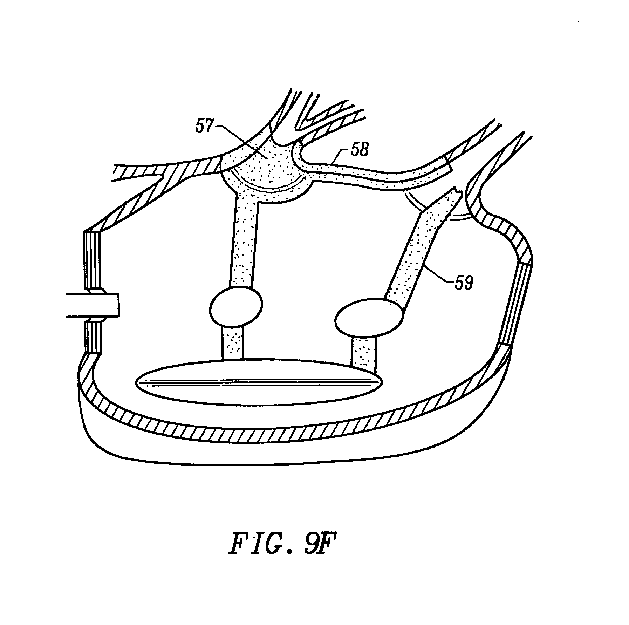

Figure 9F shows a perspective view of a segmented left posterior atrial wall with a lesion pattern which results from combining the formation of two linear lesions according to Figure 9B with the formation of a circumferential conduction block according to the methods and devices shown in Figures 8A-C.

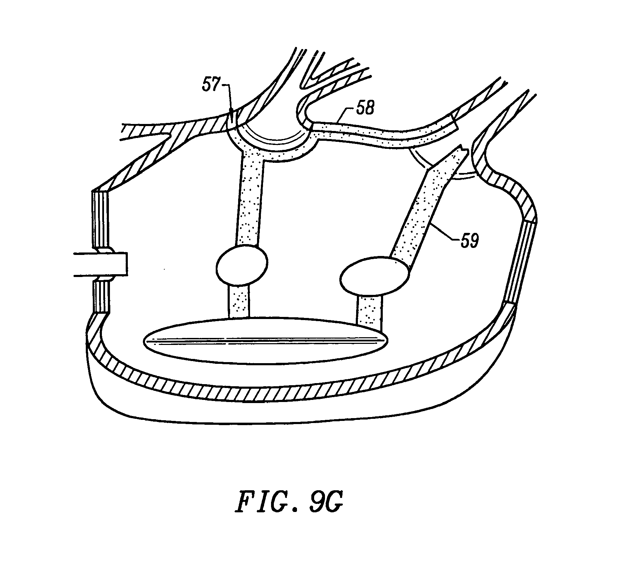

Figure 9G shows a perspective view of a segmented left posterior atrial wall with a lesion pattern which results from combining the formation of two linear lesions according to Figure 9B with the formation of a circumferential conduction block according to the methods and devices shown in Figures 8D-F.

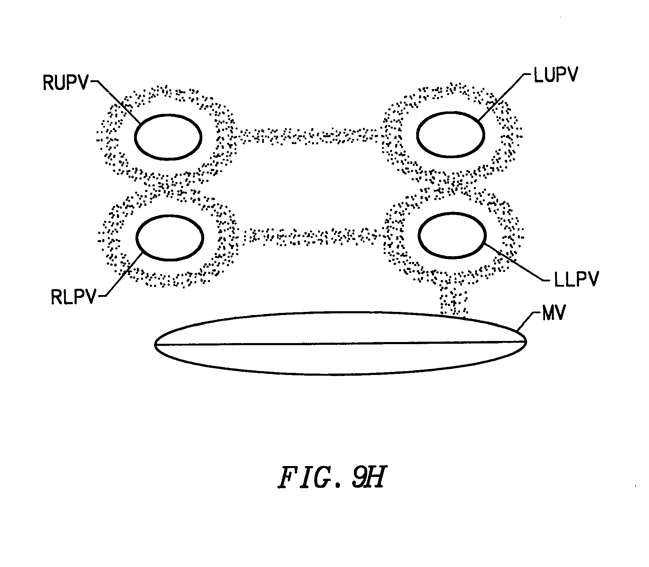

Figure 9H shows a schematic perspective view of a left posterior atrial wall with one complete lesion pattern in a variation of a less-invasive "maze"-type procedure wherein circumferential conduction blocks are formed along circumferential paths of tissue along a left posterior atrial wall such that each circumferential conduction block surrounds a pulmonary vein ostium, each pair of vertically adjacent circumferential conduction blocks intersects, and each pair of horizontally adjacent circumferential conduction blocks are connected with one of two linear lesions extending between the respective pair of horizontally adjacent pulmonary vein ostia.

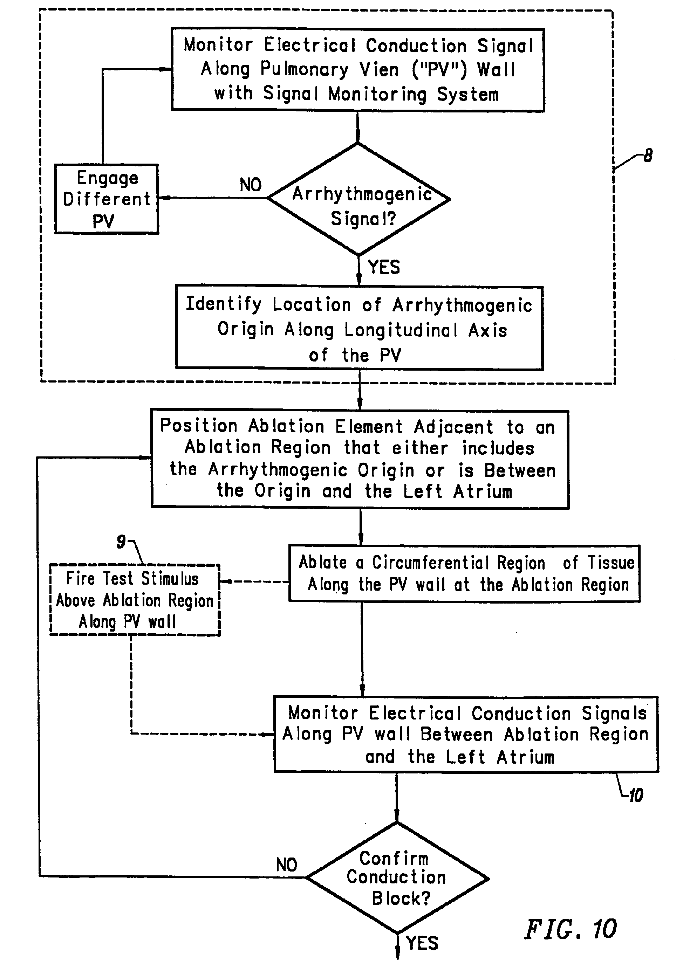

Figure 10 diagrammatically shows a further method for using the circumferential ablation device assembly of the present invention to form a circumferential conduction block in a pulmonary vein wall, wherein signal monitoring and "post-ablation" test elements are used to locate an arrhythmogenic origin along the pulmonary vein wall and to test the efficacy of a circumferential conduction block in the wall, respectively.

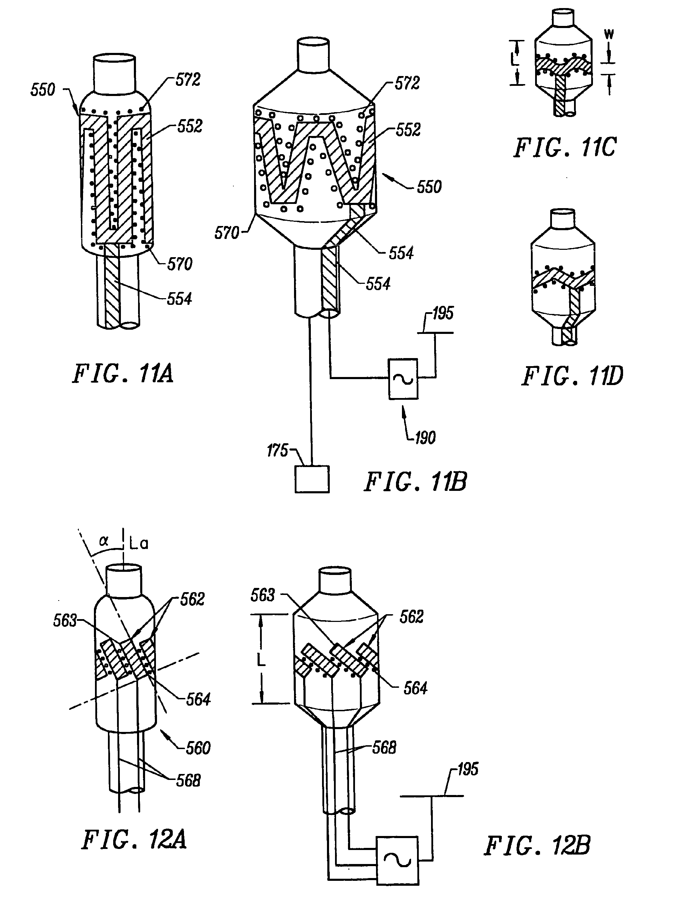

Figures 11A-B show perspective views of one circumferential ablation member variation for use in the circumferential ablation device assembly of the present invention, showing a circumferential ablation electrode circumscribing the working length of an expandable member with a secondary shape along the longitudinal axis of the working length which is a modified step shape, the expandable member being shown in a radially collapsed position and also in a radially expanded position, respectively.
Figures 11C-D show perspective views of two circumferential ablation electrodes which form equatorial or otherwise circumferentially placed bands that circumscribe the working length of an expandable member and that have serpentine and sawtooth secondary shapes, respectively, relative to the longitudinal axis of the expandable member when adjusted to a radially expanded position.
Figures 12A-B show perspective views of another circumferential ablation element which includes a plurality of individual ablation electrodes that are spaced circumferentially to form an equatorial band which circumscribes the working length of an expandable member either in an equatorial location or an otherwise circumferential location that is bounded both proximally and distally by the working length, and which are adapted to form a continuous circumferential lesion while the working length is adjusted to a radially expanded position.

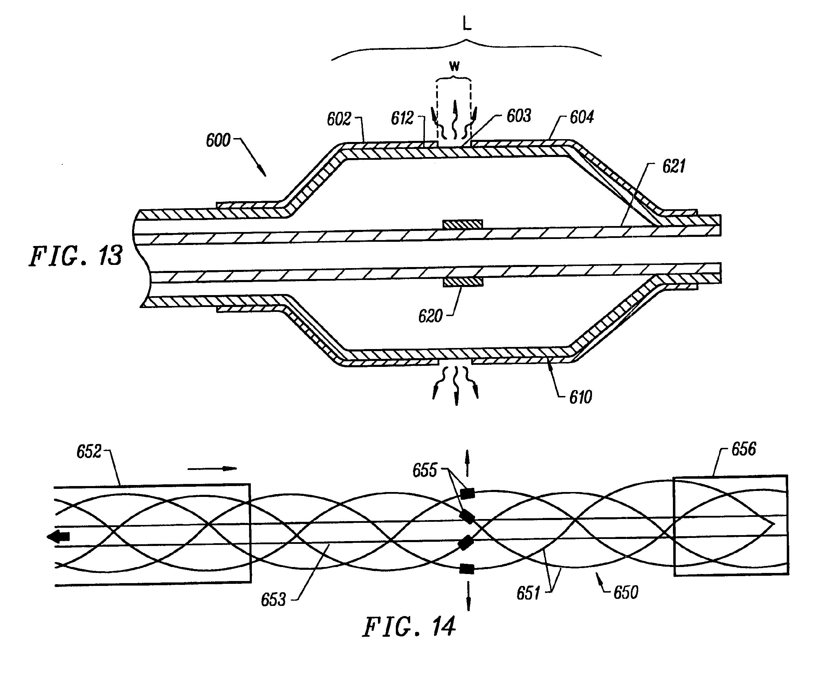

Figure 13 shows a cross-sectional view of another circumferential ablation member for use in the circumferential ablation device assembly according to the present invention, wherein the circumferential ablation element circumscribes an outer surface of an expandable member substantially along its working length and is insulated at both the proximal and the distal ends of the working length to thereby form an uninsulated equatorial band in a middle region of the working length or otherwise circumferential region of the working length which is bounded both proximally and distally by end portions of the working length, which member is adapted to ablate a circumferential path of tissue in a pulmonary wall adjacent to the equatorial band.
Figure 14 shows a perspective view of another circumferential ablation member which is adapted for use in the circumferential ablation device assembly of the present invention, wherein the expandable member is shown to be a cage of coordinating wires which are adapted to be adjusted from a radially collapsed position to a radially expanded position in order to engage electrode elements on the wires about a circumferential pattern of tissue in a pulmonary vein wall.

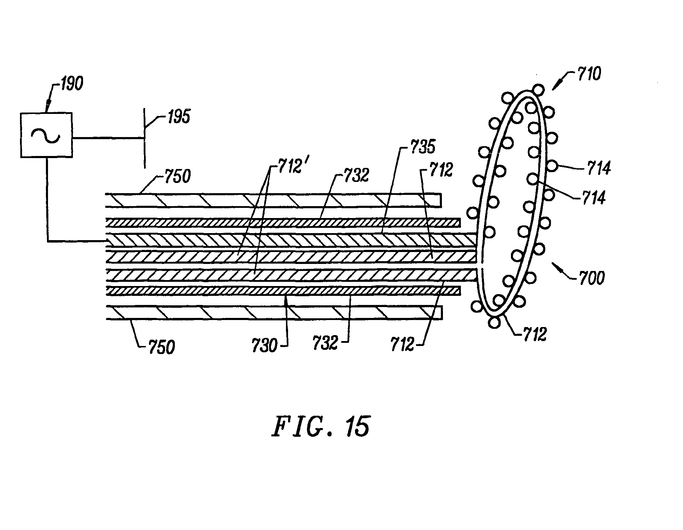

Figure 15 shows a cross-sectional view of another circumferential ablation element which is adapted for use in the circumferential ablation device assembly of the present invention. A superelastic, looped electrode element is shown at the distal end of a pusher and is adapted to circumferentially engage pulmonary vein wall tissue to form a circumferential lesion as a conduction block that circumscribes the pulmonary vein lumen.

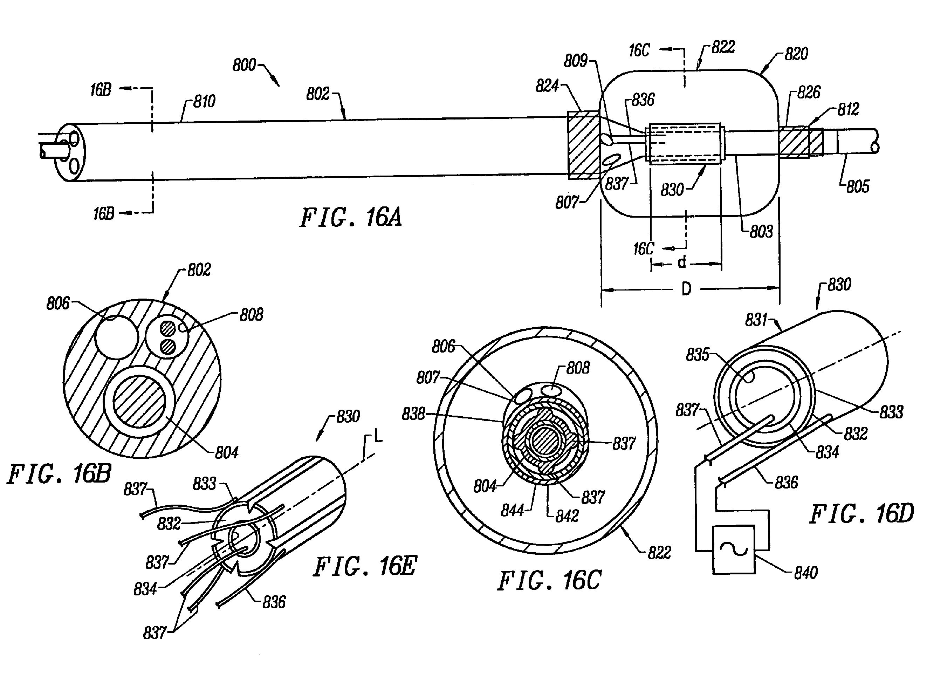

Figure 16A shows a longitudinal cross-sectional view of another circumferential ablation catheter according to the present invention, and shows the ablation element to include a single cylindrical ultrasound transducer which is positioned along an inner member within an expandable balloon which is further shown in a radially expanded condition.
Figure 16B shows a transverse cross-sectional view of the circumferential ablation catheter shown in Figure 16A taken along line 16B-16B shown in Figure 16A.
Figure 16C shows a transverse cross-sectional view of the circumferential ablation catheter shown in Figure 16A taken along line 16C-16C shown in Figure 16A.
Figure 16D shows a perspective view of the ultrasonic transducer of Figure 16A in isolation.
Figure 16E shows a modified version of the ultrasonic transducer of Figure 16D with individually driven sectors.

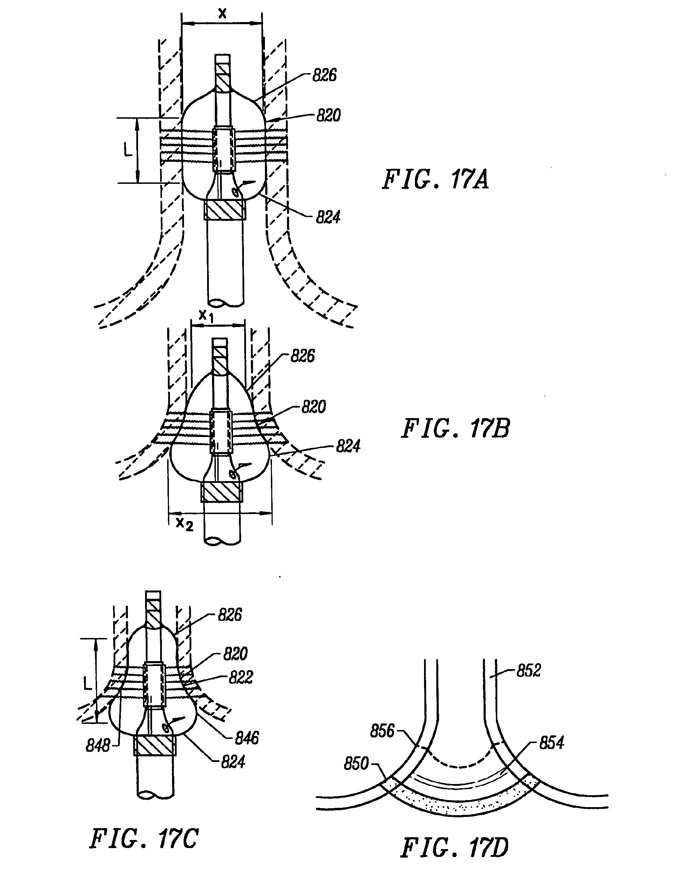

Figure 17A shows a perspective view of a similar circumferential ablation catheter to the catheter shown in Figure 16A, and shows the distal end portion of the circumferential ablation catheter during one mode of use in forming a circumferential conduction block in a pulmonary vein in the region of its ostium along a left atrial wall (shown in cross-section in shadow).
Figure 17B shows a similar perspective and cross-section shadow view of a circumferential ablation catheter and pulmonary vein ostium as that shown in Figure 17A, although shows another circumferential ablation catheter wherein the balloon has a tapered outer diameter.
Figure 17C shows a similar view to that shown in Figures 17A-B, although showing another circumferential ablation catheter wherein the balloon has a "pear"-shaped outer diameter with a contoured surface along a taper which is adapted to seat in the ostium of a pulmonary vein.
Figure 17D shows a cross-sectional view of one circumferential conduction block which may be formed by use of a circumferential ablation catheter such as that shown in Figure 17C.

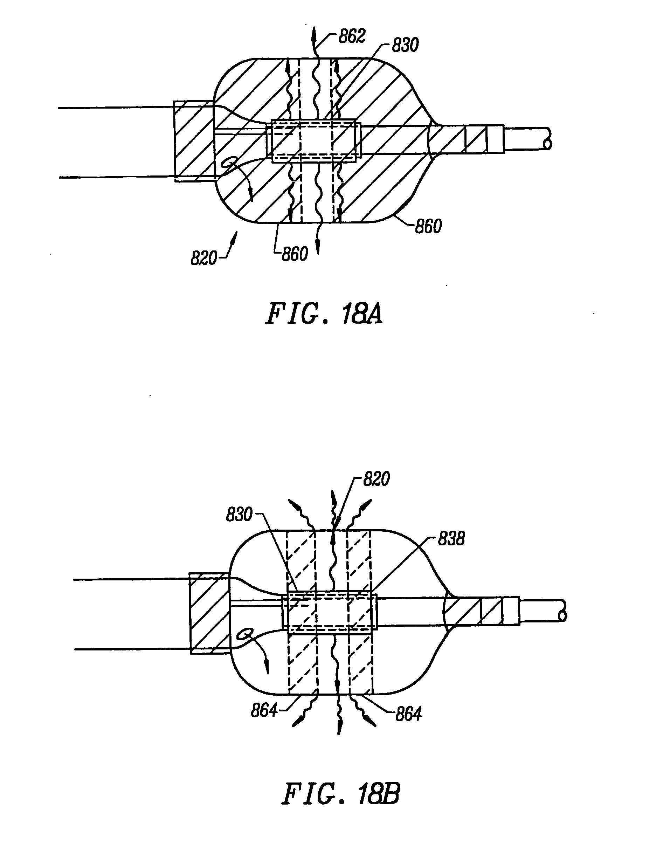

Figure 18A shows a cross-sectional view of the distal end portion of another circumferential ablation catheter according to the present invention, wherein an outer shield or filter is provided along the balloon's outer surface in order to form a predetermined shape for the circumferential ablation element created by sonic transmissions from the inner ultrasound transducer.
Figure 18B shows a similar view as that shown in Figure 18A, although showing the distal end portion of another circumferential ablation catheter which includes a heat sink as an equatorial band within the circumferential path of energy emission from an inner ultrasound transducer.

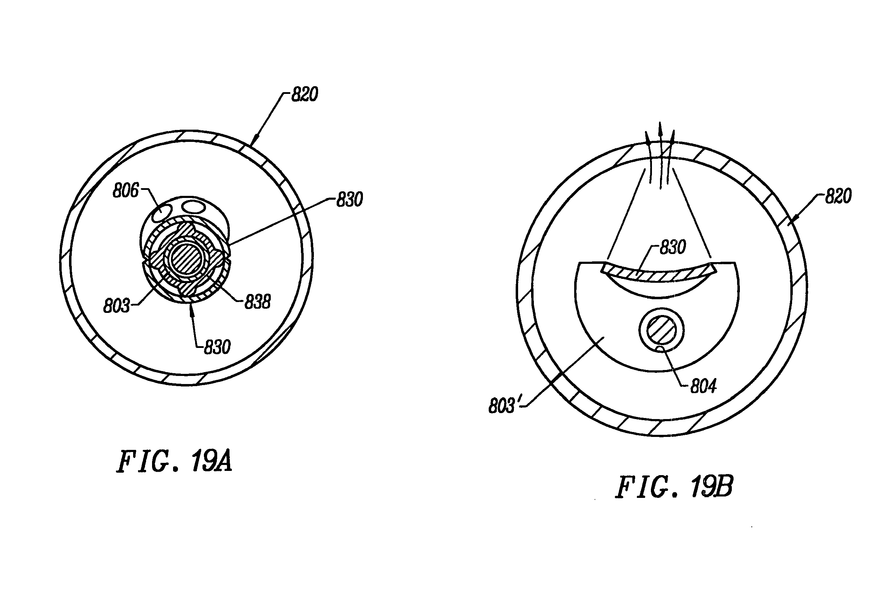

Figure 19A shows a transverse cross-sectional view of an additional circumferential ablation catheter according to the present invention, and shows the ablation element to include a single transducer sector segment which is positioned along an inner member within an expandable balloon which is further shown in a radially expanded condition.
Figure 19B shows a transverse cross-sectional view of a further circumferential ablation catheter according to the present invention, and shows the ablation element to include a single curvilinear section that is mounted so as to position its concave surface facing in a radially outward direction.

### DETAILED DESCRIPTION OF THE PREFERRED EMBODIMENTS

As will be described with reference to the detailed embodiments below, the present invention is a circumferential ablation device assembly which is adapted to treat patients with atrial arrhythmia by forming a circumferential conduction block in a pulmonary vein which blocks electrical conduction along the longitudinal axis of the pulmonary vein wall and into the left atrium. The related method of treatment is further illustrated in diagrammatical form in the flow diagram of Figure 1.

The terms "circumference" or "circumferential", including derivatives thereof, are herein intended to mean a continuous path or line which forms an outer border or perimeter that surrounds and thereby defines an enclosed region of space. Such a continuous path starts at one location along the outer border or perimeter, and translates along the outer border or perimeter until it is completed at the original starting location to enclose the defined region of space. The related term "circumscribe," including derivatives thereof, is herein intended to mean to enclose, surround, or encompass a defined region of space. Therefore, according to these defined terms, a continuous line which is traced around a region of space and which starts and ends at the same location "circumscribes" the region of space and has a "circumference" which is defined by the distance the line travels as it translates along the path circumscribing the space.

Still further, a circumferential path or element may include one or more of several shapes, and may be, for example, circular, oblong, ovular, elliptical, or otherwise planar enclosures. A circumferential path may also be three dimensional, such as, for example, two opposite-facing semi-circular paths in two different parallel or off-axis planes which are connected at their ends by line segments bridging between the planes.

For purpose of further illustration, Figures 2A-D therefore show various circumferential paths *A, B, C*, and *D*, respectively, each translating along a portion of a pulmonary vein wall and circumscribing a defined region of space, shown at *a, b, c*, and *d* also respectively, each circumscribed region of space being a portion of a pulmonary vein lumen. For still further illustration of the three-dimensional circumferential case shown in Figure 2D, Figure 2E shows an exploded perspective view of circumferential path *D* as it circumscribes multiplanar portions of the pulmonary vein lumen shown at *d', d",* and *d*"', which together make up region *d* as shown in Figure 2D.

The term "transect", including derivatives thereof, is also herein intended to mean to divide or separate a region of space into isolated regions. Thus, each of the regions circumscribed by the circumferential paths shown in Figures 2A-D transects the respective pulmonary vein, including its lumen and its wall, to the extent that the respective pulmonary vein is divided into a first longitudinal region located on one side of the transecting region, shown, for example, at region "*X*" in Figure 2A, and a second longitudinal region on the other side of the transecting plane, shown, for example, at region "*Y*" also in Figure 2A.

Therefore, a "circumferential conduction block" according to the present invention is formed along a region of tissue which follows a circumferential path along the pulmonary vein wall, circumscribing the pulmonary vein lumen and transecting the pulmonary vein relative to electrical conduction along its longitudinal axis. The transecting circumferential conduction block therefore isolates electrical conduction between opposite longitudinal portions of the pulmonary wall relative to the conduction block and along the longitudinal axis.

The terms "ablate" or "ablation," including derivatives thereof, are hereafter intended to mean the substantial altering of the mechanical, electrical, chemical, or other structural nature of tissue. In the context of intracardiac ablation applications shown and described with reference to the variations of the illustrative embodiment below, "ablation" is intended to mean sufficient altering of tissue properties to substantially block conduction of electrical signals from or through the ablated cardiac tissue.

The term "element" within the context of "ablation element" is herein intended to mean a discrete element, such as an electrode, or a plurality of discrete elements, such as a plurality of spaced electrodes, which are positioned so as to collectively ablate a region of tissue.

Therefore, an "ablation element" according to the defined terms may include a variety of specific structures adapted to ablate a defined region of tissue. For example, one suitable ablation element for use in the present invention may be formed, according to the teachings of the embodiments below, from an "energy emitting" type which is adapted to emit energy sufficient to ablate tissue when coupled to and energized by an energy source. Suitable "energy emitting" ablation elements for use in the present invention may therefore include, for example: an electrode element adapted to couple to a direct current ("DC") or alternating current ("AC") current source, such as a radiofrequency ("RF") current source; an antenna element which is energized by a microwave energy source; a heating element, such as a metallic element or other thermal conductor which is energized to emit heat such as by convective or conductive heat transfer, by resistive heating due to current flow, or by optical heating with light; a light emitting element, such as a fiber optic element which transmits light sufficient to ablate tissue when coupled to a light source; or an ultrasonic element such as an ultrasound crystal element which is adapted to emit ultrasonic sound waves sufficient to ablate tissue when coupled to a suitable excitation source.

In addition, other elements for altering the nature of tissue may be suitable as "ablation elements" under the present invention when adapted according to the detailed description of the invention below. For example, a cryoablation element adapted to sufficiently cool tissue to substantially alter the structure thereof may be suitable if adapted according to the teachings of the current invention. Furthermore, a fluid delivery element, such as a discrete port or a plurality of ports which are fluidly coupled to a fluid delivery source, may be adapted to infuse an ablating fluid, such as a fluid containing alcohol, into the tissue adjacent to the port or ports to substantially alter the nature of that tissue.

The term "diagnose", including derivatives thereof, is intended to include patients suspected or predicted to have atrial arrhythmia, in addition to those having specific symptoms or mapped electrical conduction indicative of atrial arrhythmia.

Returning to the method as shown in Figure 1, a patient diagnosed with atrial arrhythmia according to diagnosing step (1) is treated with a circumferential conduction block according to treatment step (2). In one aspect, a patient diagnosed according to diagnosis step (1) with multiple wavelet arrhythmia originating from multiple regions along the atrial wall may also be treated in part by forming the circumferential conduction block according to treatment step (2), although as an adjunct to forming long linear regions of conduction block between adjacent pulmonary vein ostia in a less-invasive "maze"-type catheter ablation procedure. More detail regarding this particular aspect of the method is provided below with reference to a combination circumferential-long linear lesion ablation device which is described below with reference to Figures 9A-F.

In another aspect of the method of Figure 1, a patient diagnosed with focal arrhythmia originating from an arrhythmogenic origin or focus in a pulmonary vein is treated according to this method when the circumferential conduction block is formed along a circumferential path of wall tissue that either includes the arrhythmogenic origin or is between the origin and the left atrium. In the former case, the arrhythmogenic tissue at the origin is destroyed by the conduction block as it is formed through that focus. In the latter case, the arrhythmogenic focus may still conduct abnormally, although such aberrant conduction is prevented from entering and affecting the atrial wall tissue due to the intervening circumferential conduction block.

In still a further aspect of the method shown in Figure 1, the circumferential conduction block may be formed in one of several ways according to treatment step (2). In one example not shown, the circumferential conduction block may be formed by a surgical incision or other method to mechanically transect the pulmonary vein, followed by suturing the transected vein back together. As the circumferential injury is naturally repaired, such as through a physiologic scarring response common to the "maze" procedure, electrical conduction will generally not be restored across the injury site. In another example not shown, a circumferential conduction block of one or more pulmonary veins may be performed in an epicardial ablation procedure, wherein an ablation element is either placed around the target pulmonary vein or is translated circumferentially around it while being energized to ablate the adjacent tissue in an "outside-in" approach. This alternative method may be performed during an open chest-type procedure, or may be done using other known epicardial access techniques.

Figure 3 diagrammatically shows the sequential steps of a method for using the circumferential ablation device assembly of the present invention in forming a circumferential conduction block in a pulmonary vein. The circumferential ablation method according to Figure 3 includes: positioning a circumferential ablation element at an ablation region along the pulmonary vein according to a series of detailed steps shown collectively in Figure 3 as positioning step (3); and thereafter ablating a continuous circumferential region of tissue in the PV wall at the ablation region according to ablation step (4).

Further to positioning step (3) according to the method of Figure 3, a distal tip of a guiding catheter is first positioned within the left atrium according to a transeptal access method, which is further described in more detail as follows. The right venous system is first accessed using the "Seldinger" technique, wherein a peripheral vein (such as a femoral vein) is punctured with a needle, the puncture wound is dilated with a dilator to a size sufficient to accommodate an introducer sheath, and an introducer sheath with at least one hemostatic valve is seated within the dilated puncture wound while maintaining relative hemostasis. With the introducer sheath in place, the guiding catheter or sheath is introduced through the hemostatic valve of the introducer sheath and is advanced along the peripheral vein, into the region of the vena cavae, and into the right atrium.

Once in the right atrium, the distal tip of the guiding catheter is positioned against the fossa ovalis in the intraatrial septal wall. A "Brockenbrough" needle or trocar is then advanced distally through the guide catheter until it punctures the fossa ovalis. A separate dilator may also be advanced with the needle through the fossa ovalis to prepare an access port through the septum for seating the guiding catheter. The guiding catheter thereafter replaces the needle across the septum and is seated in the left atrium through the fossa ovalis, thereby providing access for object devices through its own inner lumen and into the left atrium.

It is however further contemplated that other left atrial access methods may be suitable substitutes for using the circumferential ablation device assembly of the present invention. In one alternative variation not shown, a "retrograde" approach may be used, wherein the guiding catheter is advanced into the left atrium from the arterial system. In this variation, the Seldinger technique is employed to gain vascular access into the arterial system, rather than the venous, for example, at a femoral artery. The guiding catheter is advanced retrogradedly through the aorta, around the aortic arch, into the ventricle, and then into the left atrium through the mitral valve.

Subsequent to gaining transeptal access to the left atrium as just described, positioning step (3) according to Figure 3 next includes advancing a guidewire into a pulmonary vein, which is done generally through the guiding catheter seated in the fossa ovalis. In addition to the left atrial access guiding catheter, the guidewire according to this variation may also be advanced into the pulmonary vein by directing it into the vein with a second sub-selective delivery catheter (not shown) which is coaxial within the guiding catheter, such as, for example, by using one of the directional catheters disclosed in US Patent No. 5,575,766 to Swartz. Or, the guidewire may have sufficient stiffness and maneuverability in the left atrial cavity to unitarily subselect the desired pulmonary vein distally of the guiding catheter seated at the fossa ovalis.

Suitable guidewire designs for use in the overall circumferential ablation device assembly of the present invention may be selected from previously known designs, while generally any suitable choice should include a shaped, radiopaque distal end portion with a relatively stiff, torquable proximal portion adapted to steer the shaped tip under X-ray visualization. Guidewires having an outer diameter ranging from .010" to .035" (0.254 to 0.889 mm) may be suitable. In cases where the guidewire is used to bridge the atrium from the guiding catheter at the fossa ovalis, and where no other sub-selective guiding catheters are used, guidewires having an outer diameter ranging from .018" to .035" (0.457 to 0.889 mm) may be required. It is believed that guidewires within this size range may be required to provide sufficient stiffness and maneuverability in order to allow for guidewire control and to prevent undesirable guidewire prolapsing within the relatively open atrial cavity.

Subsequent to gaining pulmonary vein access, positioning step (3) of Figure 3 next includes tracking the distal end portion of a circumferential ablation device assembly over the guidewire and into the pulmonary vein, followed by positioning a circumferential ablation element at an ablation region of the pulmonary vein where the circumferential conduction block is to be desirably formed.

Figures 3-4 further show a circumferential ablation device assembly (100) according to the present invention during use in performing positioning step (3) and ablation step (4) just described with reference to Figure 3. Included in the circumferential ablation device assembly (100) are guiding catheter (101), guidewire (102), and circumferential ablation catheter (103).

More specifically, Figure 4 shows guiding catheter (101) subsequent to performing a transeptal access method according to Figure 3, and also shows guidewire (102) subsequent to advancement and positioning within a pulmonary vein, also according to step (3) of Figure 3. Figure 4 shows circumferential ablation catheter (103) as it tracks coaxially over guidewire (102) with a distal guidewire tracking member, which is specifically shown only in part at first and second distal guidewire ports (142,144) located on the distal end portion (132) of an elongate catheter body (130). A guidewire lumen (not shown) extends between the first and second distal guidewire ports (142,144) and is adapted to slideably receive and track over the guidewire. In the particular variation of Figure 4, the second distal guidewire port (142) is located on a distal end portion (132) of the elongate catheter body (130), although proximally of first distal guidewire port (142).

As would be apparent to one of ordinary skill, the distal guidewire tracking member shown in Figure 4 and just described may be slideably coupled to the guidewire externally of the body in a "backloading" technique after the guidewire is first positioned in the pulmonary vein. Furthermore, there is no need in this guidewire tracking variation for a guidewire lumen in the proximal portions of the elongate catheter body (130), which allows for a reduction in the outer diameter of the catheter shaft in that region. Nevertheless, it is further contemplated that a design which places the second distal guidewire port on the proximal end portion of the elongate catheter body would also be acceptable, as is described below, for example, with reference to the perfusion embodiment of Figures 6A-B.

In addition, the inclusion of a guidewire lumen extending within the elongate body between first and second ports, as provided in Figure 4, should not limit the scope of acceptable guidewire tracking members according to the present invention. Other guidewire tracking members which form a bore adapted to slideably receive and track over a guidewire are also considered acceptable, such as, for example, the structure adapted to engage a guidewire as described in U.S. Patent No. 5,505,702 to Arney.

While the assemblies and methods shown variously throughout the Figures include a guidewire coupled to a guidewire tracking member on the circumferential ablation catheter, other detailed variations may also be suitable for positioning the circumferential ablation element at the ablation region in order to form a circumferential conduction block there. For example, an alternative circumferential ablation catheter not shown may include a "fixed-wire" -type of design wherein a guidewire is integrated into the ablation catheter as one unit. In another alternative assembly, the same type of sub-selective sheaths described above with reference to U.S. Patent No. 5,575,766 to Swartz for advancing a guidewire into a pulmonary vein may also be used for advancing a circumferential ablation catheter device across the atrium and into a pulmonary vein.

Figure 4 also shows circumferential ablation catheter (103) with a circumferential ablation element (160) formed on an expandable member (170). The expandable member (170) is shown in Figure 4 in a radially collapsed position adapted for percutaneous translumenal delivery into the pulmonary vein according to positioning step (3) of Figure 3. However, expandable member (170) is also adjustable to a radially expanded position when actuated by an expansion actuator (175), as shown in Figure 5. Expansion actuator (175) may include, but is not limited to, a pressurizeable fluid source. According to the expanded state shown in Figure 5, expandable member (170) includes a working length *L* relative to the longitudinal axis of the elongate catheter body which has a larger expanded outer diameter *OD* than when in the radially collapsed position. Furthermore, the expanded outer diameter *OD* is sufficient to circumferentially engage the ablation region of the pulmonary vein. Therefore, the terms "working length" are herein intended to mean the length of an expandable member which, when in a radially expanded position, has an expanded outer diameter that is: (a) greater than the outer diameter of the expandable member when in a radially collapsed position; and (b) sufficient to engage a body space wall or adjacent ablation region surrounding the expandable member, at least on two opposing internal sides of the body space wall or adjacent ablation region, with sufficient surface area to anchor the expandable member.

Circumferential ablation element (160) also includes a circumferential band (152) on the outer surface of working length *L* which is coupled to an ablation actuator (190) at a proximal end portion of the elongate catheter body (shown schematically). After expandable member (170) is adjusted to the radially expanded position and at least a portion of working length *L* circumferentially engages the pulmonary vein wall in the ablation region, the circumferential band (152) of the circumferential ablation element (160) is actuated by ablation actuator (190) to ablate the surrounding circumferential path of tissue in the pulmonary vein wall, thereby forming a circumferential lesion that circumscribes the pulmonary vein lumen and transects the electrical conductivity of the pulmonary vein to block conduction in a direction along its longitudinal axis.

Figure 6A shows another circumferential ablation catheter (203) during use also according to the method of Figure 3, wherein a perfusion lumen (260) (shown in phantom in Figure 6B) is formed within the distal end portion (232) of elongate catheter body (230). The perfusion lumen (260) in this example is formed between a distal perfusion port, which in this example is the first distal guidewire port (242), and proximal perfusion port (244). Proximal perfusion port (244) is formed through the wall of the elongate catheter body (230) and communicates with the guidewire lumen (not shown) which also forms the perfusion lumen between the distal and proximal perfusion ports. In the particular design shown, after the guidewire has provided for the placement of the ablation element into the pulmonary vein, the guidewire is withdrawn proximally of the proximal perfusion port (244) (shown schematically in shadow) so that the lumen between the ports is clear for antegrade blood flow into the distal perfusion port (242), proximally along the perfusion lumen, out the proximal perfusion port (244) and into the atrium (perfusion flow shown schematically with arrows).

Further to the perfusion design shown in Figures 6A-B, guidewire (102) is positioned in a guidewire lumen which extends the entire length of the elongate catheter body (230) in an "over-the-wire"-type of design, which facilitates the proximal withdrawal of the guidewire to allow for perfusion while maintaining the ability to subsequently readvance the guidewire distally through the first distal guidewire port (242) for catheter repositioning. In one alternative variation not shown, the guidewire is simply withdrawn and disengaged from the second distal guidewire port (244), in which case the circumferential ablation catheter must generally be withdrawn from the body in order to recouple the distal guidewire tracking member with the guidewire.

In another alternative perfusion variation not shown which is a modification of the embodiment of Figure 6A, a proximal perfusion port is provided as a separate and distinct port positioned between the second distal guidewire port (244) and the expandable member (270), which allows for proximal withdrawal of the guidewire to clear the guidewire lumen and thereby form a perfusion lumen between the first distal guidewire port and the proximal perfusion port. The guidewire of this alternative variation, however, remains engaged within the guidewire lumen between the second distal guidewire port and the proximal perfusion port.

Passive perfusion during expansion of the expandable member is believed to minimize stasis and allow the target pulmonary vein to continue in its atrial filling function during the atrial arrhythmia treatment procedure. Without this perfusion feature, the expandable member when in the radially expanded position during ablation blocks the flow from the vein into the atrium, which flow stasis may result in undesirable thrombogenesis in the pulmonary vein distally to the expandable member. In addition, in cases where the ablation element is adapted to ablate tissue with heat conduction at the ablation region, as described by reference to more detailed embodiments below, the perfusion feature according to the variation of Figures 6A-B may also provide a cooling function in the surrounding region, including in the blood adjacent to the expandable member.

Moreover, in addition to the specific perfusion structure shown and described by reference to Figures 6A-B, it is to be further understood that other structural variants which allow for perfusion flow during expansion of the expandable element may provide suitable substitutes according to one of ordinary skill without departing from the scope of the present invention.

Figure 7 shows pulmonary vein (52) after removing the circumferential ablation device assembly subsequent to forming a circumferential lesion (70) around the ablation region of the pulmonary vein wall (53) according to the use of the circumferential ablation device assembly shown in stepwise fashion in Figures 3-6. Circumferential lesion (70) is shown located along the pulmonary vein adjacent to the pulmonary vein ostium (54), and is shown to also be "transmural," which is herein intended to mean extending completely through the wall, from one side to the other. Also, the circumferential lesion (70) is shown in Figure 7 to form a "continuous" circumferential band, which is herein intended to mean without gaps around the pulmonary vein wall circumference, thereby circumscribing the pulmonary vein lumen.

It is believed, however, that circumferential catheter ablation with a circumferential ablation element according to the present invention may leave some tissue, either transmurally or along the circumference of the lesion, which is not actually ablated, but which is not substantial enough to allow for the passage of conductive signals. Therefore, the terms "transmural" and "continuous" as just defined are intended to have functional limitations, wherein some tissue in the ablation region may be unablated but there are no functional gaps which allow for symptomatically arrhythmogenic signals to conduct through the conduction block and into the atrium from the pulmonary vein.

Moreover, it is believed that the functionally transmural and continuous lesion qualities just described are characteristic of a completed circumferential conduction block in the pulmonary vein. Such a circumferential conduction block thereby transects the vein, isolating conduction between the portion of the vein on one longitudinal side of the lesion and the portion on the other side. Therefore, any foci of originating arrhythmogenic conduction which is opposite the conduction block from the atrium is prevented by the conduction block from conducting down into the atrium and atrial arrhythmic affects are therefore nullified.

Figures 8A-B show a further variation of the present invention, wherein a circumferential ablation member (350) includes a radially compliant expandable member (370) which is adapted to conform to a pulmonary vein ostium (54) at least in part by adjusting it to a radially expanded position while in the left atrium and then advancing it into the ostium. Figure 8A shows expandable member (370) after being adjusted to a radially expanded position while located in the left atrium (50). Figure 8B further shows expandable member (370) after being advanced into the pulmonary vein (52) until at least a portion of the expanded working length *L* of circumferential ablation member (350), which includes a circumferential band (352), engages the pulmonary vein ostium (54). Figure 8C shows a portion of a circumferential lesion (72) which forms a circumferential conduction block in the region of the pulmonary vein ostium (54) subsequent to actuating the circumferential ablation element to form the circumferential lesion.

In addition to conforming to the pulmonary vein ostium, expandable member (370) is also shown in Figure 8B to engage a circumferential path of tissue along the left posterior atrial wall which surrounds ostium (54). Moreover, circumferential bank (352) of the circumferential ablation member is also thereby adapted to engage that atrial wall tissue. Therefore, the circumferential conduction block formed according to the method shown and just described in sequential steps by reference to Figures 8A-B, as shown in-part in Figure 8C, includes ablating the circumferential path of atrial wall tissue which surrounds ostium (54). Accordingly, the entire pulmonary vein, including the ostium, is thereby electrically isolated from at least a substantial portion of the left atrial wall which includes the other of the pulmonary vein ostia, as would be apparent to one of ordinary skill according to the sequential method steps shown in Figures 8A-B and by further reference to the resulting circumferential lesion (72) shown in Figure 8C.

Figures 8D-E show another highly beneficial circumferential ablation device embodiment and use thereof for electrically isolating pulmonary vein and ostium from a substantial portion of the left posterior atrial wall. However, unlike the embodiment previously shown and described by reference to Figures 8A-C, the Figure 8D-E embodiment isolates the pulmonary vein without also ablating tissue along the lumen or lining of the pulmonary vein or ostium, as is apparent by reference to the resulting circumferential conduction block shown in Figure 8f.

In more detail, Figure 8D shows a similar device assembly as that shown in Figures 8A-B, except that circumferential band (352') has a geometry (primarily width) and position along expandable member (370') such that it is adapted to engage only a circumferential path of tissue along the left posterior atrial wall which surrounds the pulmonary vein ostium. In one aspect of this embodiment, the compliant nature of the expandable member may be self-conforming to the region of the ostium such that the circumferential band is placed against this atrial wall tissue merely by way of conformability.

In another variation, a "pear"-shaped expandable member or balloon that includes a contoured taper may be suitable for use according to the Figure 8D embodiment, as is shown by way of example in Figure 8E. Such a pear shape may be preformed into the expandable member or balloon, or the member may be adapted to form this shape by way of controlled compliance as it expands, such as for example by the use of composite structures within the balloon construction. In any case, according to the "pear"-shaped variation, the circumferential band (352') of the ablation member is preferably placed along the surface of the contoured taper which is adapted to face the left posterior atrial wall during use according to the method illustrated by Figure 8D. It is further contemplated that the ablation element may be further extended or alternatively positioned along other portions of the taper, such as is shown by example in shadow at extended band (352") in Figure 8E. Accordingly, the variation shown in Figure 8E to include extended band (352") may also adapt this particular device embodiment for use in forming circumferential conduction blocks also along tissue within the pulmonary vein and ostium, such as according to the previously described method shown in Figures 8A-C.

The method of forming a circumferential conduction block along a circumferential path of tissue along a left posterior atrial wall and which surrounds a pulmonary vein ostium without ablating the tissue of the vein or ostium should not be limited to the particular device embodiments just illustrated by reference to Figures 8D-F. Other device variations may be acceptable substitutes for use according to this method. In one particular example which is believed to be suitable, a "looped" ablation member such as the embodiment illustrated below by reference to Figure 15 may be adapted to form a "looped" ablation element within the left atrium and then be advanced against the left posterior atrial wall such that the loop engages the circumferential path of tissue along the atrial wall and which surrounds a vein ostium. Thereafter, the looped ablation element may be actuated to ablate the engaged tissue, such as for further illustration like a branding iron forming the predetermined pattern around the pulmonary vein os (i.e., ostium). In addition, other device or method variations may also be suitable substitutes according to one of ordinary skill.

Figures 9A-D collectively show a circumferential ablation device assembly according to the present invention as it is used to form a circumferential conduction block adjunctively to the formation of long linear lesions in a less-invasive "maze"-type procedure, as introduced above for the treatment of multiwavelet reentrant type fibrillation along the left atrial wall.

More specifically, Figure 9A diagrammatically shows a summary of steps for performing a "maze"-type procedure by forming circumferential conduction blocks that intersect with long linear conduction blocks formed between the pulmonary veins. A box-like conduction block surrounding an arrhythmogenic atrial wall region bounded by the pulmonary veins may be created by forming long linear lesions between anchors in all pairs of adjacent pulmonary vein ostia, such as is shown in part in steps (5) and (6) of Figure 9A. However, it is further believed that, in some particular applications, such linear lesions may be made sufficiently narrow with respect to the surface area of the pulmonary vein ostia that they may not intersect, thereby leaving gaps between them which may present proarrhythmic pathways for abnormal conduction into and from the box, such as is shown between linear lesions (57,58,59) in Figure 9B. Therefore, by forming the circumferential conduction block according to step (7) of Figure 9A, and as shown by use of circumferential ablation member (450) in Figure 9C, the linear lesions are thereby bridged and the gaps are closed.

In a further variation to the specific embodiments shown in Figures 9B-C, Figure 9D shows another circumferential ablation device assembly which includes both circumferential and linear ablation elements (452,461), respectively. Circumferential ablation member (450) is shown to include an expandable member (470) which is adjusted to a radially expanded position that is asymmetric to the underlying catheter shaft. Linear ablation member (460) extends along the elongate body proximally from the circumferential ablation member (450). When expanded sufficiently to engage the pulmonary vein wall, expandable member (470) provides at least a portion of an anchor for a first end (462) of linear ablation member (460).

A shaped stylet (466) is shown in shadow in Figure 9D within the elongate catheter body in the region of the second end (464) of the linear ablation member (460). Shaped stylet (466) is adapted to push the second end (464) into an adjacent pulmonary vein ostium such that the linear ablation member (460) is adapted to substantially contact the left atrial wall between the adjacent vein ostia to form the linear ablation according to the method of Figure 9A. In addition to the use of shaped stylet (466), it is further contemplated that a second anchor may be used adjacent to second end (464), such as for example an intermediate guidewire tracking member adapted to track over a guidewire engaged to the pulmonary vein, ar shown in Figure. 9E at intermediate guidewire tracking member (466') which is engaged over guidewire (467).

In a yet a further variation to the specific embodiment shown in Figure 9D, Figure 9E shows a circumferential ablation device assembly which includes both circumferential and linear ablation elements (452,460), respectively. Circumferential ablation member (450) is shown to include an expandable member (470) which is adjusted to a radially expanded position that is asymmetric to the underlying catheter shaft Linear ablation member (460) extends along the elongate body proximally from the circumferential ablation member (450). When expanded sufficiently to engage the pulmonary vein wall, expandable member (470) provides at least a portion of an anchor for a first end (462) of linear ablation member (460).

Moreover, the method shown schematically in Figure 9A and also in various detail by reference to Figures 9B-C provides a specific sequence of steps for the purpose of illustration. According to this illustrative sequence, the linear lesions are formed first and then are connected thereafter with the circumferential conduction block. However, a circumferential conduction block may be formed prior to the formation of the linear lesions or conduction blocks, or in any other combination or sub-combination of sequential steps. so long as the resulting combination of lesions allows for the circumferential block to intersect with and connect with the linear lesions. In addition, the circumferential conduction block which connects the linear lesions may also include a circumferential path of tissue which surrounds and electrically isolates the pulmonary vein ostium from the rest of the left posterior atrial wall, such as for example by considering the embodiments just shown and described by reference to Figures 9A-E in view of the embodiment previously shown and described in relation to Figure 8C above.

In addition to the particular embodiments just shown and described by reference to Figures 9A-E, other methods are also contemplated for combining circumferential and linear conduction blocks device assemblies and uses in order to perform a less-invasive "maze"-type procedure. For example, Figure 9F shows one particular lesion pattern which results by combining a circumferential conduction block, formed according to the previous embodiments of Figures 8A-C, with a pair of linear lesions which are formed according to the method illustrated by Figure 9B. In a further example shown in Figure 9G, another lesion pattern is formed by combining the pair of linear lesions of Figure 9B with a circumferential conduction block formed according to the embodiments which are previously illustrated above by reference to Figures 9D-F. While the resulting lesion patterns of Figures 9F and 9G differ slightly as regards the particular geometry and position of the circumferential conduction block formed, the two variations are also similar in that the circumferential conduction block includes a circumferential path of atrial wall tissue. When such circumferential conduction blocks are formed between adjacent pulmonary vein ostia, shorter linear lesions are therefore sufficient to bridge the circumferential lesions during the overall "maze"-type procedure.

To this end, the invention further contemplates one further variation for a less-invasive "maze"-type procedure (not shown) wherein multiple circumferential conduction blocks are formed in atrial wall tissue such that each pulmonary vein ostium is surrounded by and is electrically isolated with one circumferential conduction block. A series of four linear lesions may be formed between the various pairs of adjacent ostia and with just sufficient length to intersect with and bridge the corresponding adjacent circumferential blocks. A box-like conduction block is thereby formed by the four circumferential conduction blocks and the four bridging linear lesions. A fifth linear lesion may be also formed between at least a portion of the box-like conduction block and another predetermined location, such as for example the mitral value annulus.

Figure 9H shows yet a further variation for forming circumferential conduction blocks along atrial wall tissue around the pulmonary vein ostia during a less invasive "maze"-type procedure. According to this further variation, the circumferential conduction block patterns formed around each of two adjacent superior and inferior pulmonary vein ostia are shown in Figure 9H to intersect, thereby alleviating the need for a linear lesion in order to form a conduction block between the ostia. Furthermore, the distances between the inferior and superior ostia, both on the right and left side of the posterior atrial wall, are believed to be significantly shorter than the distances between the two adjacent superior or inferior ostia. Therefore, Figure 9H only shows the overlapping circumferential conduction blocks as just described to be positioned vertically between the inferior-superior pairs of adjacent ostia, and further shows linear lesions which are used to connect the right and left sided ostia of the superior and inferior pairs. In some instances these linear lesions will not be required to cure, treat or prevent a particular atrial arrhythmia condition. However, other combinations of these patterns are further contemplated, such as for example using only overlapping circumferential conduction blocks between all adjacent pairs of ostia in order to form the entire "maze"-type left atrial pattern.

Figure 10 diagrammatically shows a further method for using the circumferential ablation device assembly of the present invention wherein electrical signals along the pulmonary vein are monitored with a sensing element before and after ablation according to steps (8) and (9), respectively. Signals within the pulmonary vein are monitored prior to forming a conduction block, as indicated in step (8) in Figure 10, in order to confirm that the pulmonary vein chosen contains an arrhythmogenic origin for atrial arrhythmia. Failure to confirm an arrhythmogenic origin in the pulmonary vein, particularly in the case of a patient diagnosed with focal arrhythmia, may dictate the need to monitor signals in another pulmonary vein in order to direct treatment to the proper location in the heart. In addition, monitoring the pre-ablation signals may be used to indicate the location of the arrhythmogenic origin of the atrial arrhythmia, which information helps determine the best location to form the conduction block. As such, the conduction block may be positioned to include and therefore ablate the actual focal origin of the arrhythmia, or may be positioned between the focus and the atrium in order to block aberrant conduction from the focal origin and into the atrial wall.

In addition or in the alternative to monitoring electrical conduction signals in the pulmonary vein prior to ablation, electrical signals along the pulmonary vein wall may also be monitored by the sensing element subsequent to circumferential ablation, according to step (9) of the method of Figure 10. This monitoring method aids in testing the efficacy of the ablation in forming a complete conduction block against arrhythmogenic conduction. Arrhythmogenic firing from the identified focus will not be observed during signal monitoring along the pulmonary vein wall when taken below a continuous circumferential and transmural lesion formation, and thus would characterize a successful circumferential conduction block. In contrast, observation of such arrhythmogenic signals between the lesion and the atrial wall characterizes a functionally incomplete or discontinuous circumference (gaps) or depth (transmurality) which would potentially identify the need for a subsequent follow-up procedure, such as a second circumferential lesioning procedure in the ablation region.

A test electrode may also be used in a "post ablation" signal monitoring method according to step (10) of Figure 10. In one particular embodiment not shown, the test electrode is positioned on the distal end portion of an elongate catheter body and is electrically coupled to a current source for firing a test signal into the tissue surrounding the test electrode when it is placed distally or "upstream" of the circumferential lesion in an attempt to simulate a focal arrhythmia. This test signal generally challenges the robustness of the circumferential lesion in preventing atrial arrhythmia from any such future physiologically generated aberrant activity along the suspect vein.

Further to the signal monitoring and test stimulus methods just described, such methods may be performed with a separate electrode or electrode pair located on the catheter distal end portion adjacent to the region of the circumferential ablation element, or may be performed using one or more electrodes which form the circumferential ablation element itself, as will be further developed below.

### Circumferential Ablation Member

The designs for the expandable member and circumferential ablation element for use in the circumferential ablation device assembly of the present invention have been described generically with reference to the embodiments shown in the previous Figures. Examples of more specific expandable member and ablation element embodiments which are adapted for use in the assembly of the present invention are further provided as follows.

Notwithstanding their somewhat schematic detail, the circumferential ablation members shown in the previous figures do illustrate one particular embodiment wherein a circumferential electrode element circumscribes an outer surface of an expandable member. The expandable member of the embodiments shown may take one of several different forms, although the expandable member is generally herein shown as an inflatable balloon that is coupled to an expansion actuator (175) which is a pressurizeable fluid source. The balloon is preferably made of a polymeric material and forms a fluid chamber which communicates with a fluid passageway (not shown in the figures) that extends proximally along the elongate catheter body and terminates proximally in a proximal fluid port that is adapted to couple to the pressurizeable fluid source.

In one expandable balloon variation, the balloon is constructed of a relatively inelastic polymer such as a polyethylene ("PE"; preferably linear low density or high density or blends thereof), polyolefin copolymer ("POC"), polyethylene terepthalate ("PET"), polyimide, or a nylon material. In this construction, the balloon has a low radial yield or compliance over a working range of pressures and may be folded into a predetermined configuration when deflated in order to facilitate introduction of the balloon into the desired ablation location via known percutaneous catheterization techniques. In this variation, one balloon size may not suitably engage all pulmonary vein walls for performing the circumferential ablation methods of the present invention on all needy patients. Therefore, it is further contemplated that a kit of multiple ablation catheters, with each balloon working length having a unique predetermined expanded diameter, may be provided from which a treating physician may chose a particular device to meet a particular patient's pulmonary vein anatomy.

In an alternative expandable balloon variation, the balloon is constructed of a relatively compliant, elastomeric material, such as, for example (but not limited to), a silicone, latex, polyurethane, or mylar elastomer. In this construction, the balloon takes the form of a tubular member in the deflated, non-expanded state. When the elastic tubular balloon is pressurized with fluid such as in the previous, relatively non-compliant example, the material forming the wall of the tubular member elastically deforms and stretches radially to a predetermined diameter for a given inflation pressure. It is further contemplated that the compliant balloon may be constructed as a composite, such as, for example, a latex or silicone balloon skin which includes fibers, such as metal, Kevlar, or nylon fibers, which are embedded into the skin. Such fibers, when provided in a predetermined pattern such as a mesh or braid, may provide a controlled compliance along a preferred axis, preferably limiting longitudinal compliance of the expandable member while allowing for radial compliance.

It is believed that, among other features, the relatively compliant variation may provide a wide range of working diameters, which may allow for a wide variety of patients, or of vessels within a single patient, to be treated with just one or a few devices. Furthermore, this range of diameters is achievable over a relatively low range of pressures, which is believed to diminish a potentially traumatic vessel response that may otherwise be presented concomitant with higher pressure inflations, particularly when the inflated balloon is oversized to the vessel. In addition, the low-pressure inflation feature of this variation is suitable for the present invention because the functional requirement of the expandable balloon is merely to engage the ablation element against a circumferential path along the inner lining of the pulmonary vein wall.

Moreover, a circumferential ablation member is adapted to conform to the geometry of the pulmonary vein ostium, at least in part by providing substantial compliance to the expandable member, as was shown and described previously by reference to Figures 8A-B. Further to this conformability to pulmonary vein ostium as provided in the specific design of Figures 8A-B, the working length L of expandable member (370) is also shown to include a taper which has a distally reducing outer diameter from a proximal end (372) to a distal end (374). In either a compliant or the non-compliant balloon, such a distally reducing tapered geometry adapts the circumferential ablation element to conform to the funneling geometry of the pulmonary veins in the region of their ostia in order to facilitate the formation of a circumferential conduction block there.

Further to the circumferential electrode element embodiment as shown variously throughout the previous illustrative Figures, the circumferential electrode element is coupled to an ablation actuator (190). Ablation actuator (190) generally includes a radiofrequency ("RF") current source (not shown) that is coupled to both the RF electrode element and also a ground patch (195) which is in skin contact with the patient to complete an RF circuit. In addition, ablation actuator (190) preferably includes a monitoring circuit (not shown) and a control circuit (not shown) which together use either the electrical parameters of the RF circuit or tissue parameters such as temperature in a feedback control loop to drive current through the electrode element during ablation. Also, where a plurality of ablation elements or electrodes in one ablation element are used, a switching means may be used to multiplex the RF current source between the various elements or electrodes.

Figures 11A-D show various patterns of electrically conductive, circumferential electrode bands as electrode ablation elements, each circumscribing an outer surface of the working length of an expandable member. Figures 11A-B show circumferential ablation member (550) to include a continuous circumferential electrode band (552) that circumscribes an outer surface of an expandable member (570). Figure 11B more specifically shows expandable member (570) as a balloon which is fluidly coupled to a pressurizeable fluid source (175), and further shows electrode band (circumferential ablation element) (552) electrically coupled via electrically conductive lead (554) to ablation actuator (190). In addition, a plurality of apertures (572) are shown in the balloon skin wall of expandable member (570) adjacent to electrode band (552). The purpose of these apertures (572) is to provide a positive flow of fluid such as saline or ringers lactate fluid into the tissue surrounding the electrode band (552). Such fluid flow is believed to reduce the temperature rise in the tissue surrounding the electrode element during RF ablation.

The shapes shown collectively in Figures 11A-D allow for a continuous electrode band to circumscribe an expandable member's working length over a range of expanded diameters, a feature which is believed to be particularly useful with a relatively compliant balloon as the expandable member. In the particular embodiments of Figures 11A-D, this feature is provided primarily by a secondary shape given to the electrode band relative to the longitudinal axis of the working length of the expandable member. Electrode band (552) is thus shown in Figures 11A-B to take the specific secondary shape of a modified step curve. Other shapes than a modified step curve are also suitable, such as the serpentine or sawtooth secondary shapes shown respectively in Figures 11C-D. Other shapes in addition to those shown in Figures 11A-D and which meet the defined functional requirements are further contemplated within the scope of the present invention.

In addition, the electrode band provided by the circumferential ablation elements shown in Figures 11C-D and also shown schematically in Figures 3-6B has a functional band width w relative to the longitudinal axis of the working length which is only required to be sufficiently wide to form a complete conduction block against conduction along the walls of the pulmonary vein in directions parallel to the longitudinal axis. In contrast, the working length *L* of the respective expandable element is adapted to securely anchor the distal end portion in place such that the ablation element is firmly positioned at a selected region of the pulmonary vein for ablation. Accordingly, the band width w is relatively narrow compared to the working length *L* of the expandable element, and the electrode band may thus form a relatively narrow equatorial band which has a band width that is less than two-thirds or even one-half of the working length of the expandable element. Additionally, it is to be noted here and elsewhere throughout the specification, that a narrow band may be placed at locations other than the equator of the expandable element, preferably as long as the band is bordered on both sides by a portion of the working length *L.*

In another aspect of the narrow equatorial band variation for the circumferential ablation element, the circumferential lesion formed may also be relatively narrow when compared to its own circumference, and may be less than two-thirds or even one-half its own circumference on the expandable element when expanded. In one arrangement which is believed to be suitable for ablating circumferential lesions in the pulmonary veins as conduction blocks, the band width w is less than 1 cm with a circumference on the working length when expanded that is greater than 1.5 cm.

Figures 12A-B show a further variation of a circumferential ablation element which is adapted to maintain a continuous circumferential lesion pattern over a range of expanded diameters and which includes electrode elements that form a relatively narrow equatorial band around the working length of an expandable balloon member. In this variation, a plurality of individual electrode/ablation elements (562) are included in the circumferential ablation element and are positioned in spaced arrangement along an equatorial band which circumscribes an outer surface of the expandable member's working length *L*.

The size and spacing between these individual electrode elements (562), when the balloon is expanded, is adapted to form a substantially continuous circumferential lesion in pulmonary vein wall tissue when in intimal contact adjacent thereto, and is further adapted to form such a lesion over a range of band diameters as the working length is adjusted between a variety of radially expanded positions. Each individual electrode element (562) has two opposite ends (563,564), respectively, along a long axis LA and also has a short axis *SA,* and is positioned such that the long axis *LA* is at an acute angle relative to the longitudinal axis *La* of the elongate catheter body and expandable member (560). At least one of the ends (563,564) along the long axis *LA* overlaps with an end of another adjacent individual electrode element, such that there is a region of overlap along their circumferential aspect, i.e., there is a region of overlap along the circumferential coordinates. The terms "region of overlap along their circumferential coordinate" are herein intended to mean that the two adjacent ends each are positioned along the working length with a circumferential and also a longitudinal coordinate, wherein they share a common circumferential coordinate. In this arrangement, the circumferential compliance along the working length which accompanies radial expansion of the expandable member also moves the individual electrode elements apart along the circumferential axis. However, the spaced, overlapping arrangement described allows the individual ablation elements to maintain a certain degree of their circumferential overlap, or at least remain close enough together, such that a continuous lesion may be formed without gaps between the elements.

The construction for suitable circumferential electrode elements in the RF variation of the present invention, such as the various electrode embodiments described with reference to Figures 11A-12B, may comprise a metallic material deposited on the outer surface of the working length using conventional techniques, such as by plasma depositing, sputter coating, chemical vapor deposition, other known techniques which are equivalent for this purpose, or otherwise affixing a metallic shaped member onto the outer surface of the expandable member such as through known adhesive bonding techniques. Other RF electrode arrangements are also considered within the scope of the present invention, so long as they form a circumferential conduction block as previously described. For example, a balloon skin may itself be metallized, such as by mixing conductive metal, including but not limited to gold, platinum, or silver, with a polymer to form a compounded, conductive matrix as the balloon skin.

Still further to the RF electrode embodiments, another circumferential ablation member variation (not shown) may also include an expandable member, such as an inflatable balloon, that includes a porous skin that is adapted to allow fluid, such as hypertonic saline solution, to pass from an internal chamber defined by the skin and outwardly into surrounding tissues. Such a porous skin may be constructed according to several different methods, such as by forming holes in an otherwise contiguous polymeric material, including mechanically drilling or using laser energy, or the porous skin may simply be an inherently porous membrane. In any case, by electrically coupling the fluid within the porous balloon skin to an RF current source (preferably monopolar), the porous region of the expandable member serves as an RF electrode wherein RF current flows outwardly through the pores via the conductive fluid. In addition, it is further contemplated that a porous outer skin may be provided externally of another, separate expandable member, such as a separate expandable balloon, wherein the conductive fluid is contained in a region between the porous outer skin and the expandable member contained therein. Various other "fluid electrode" designs than those specifically herein described may also be suitable according to one of ordinary skill upon review of this disclosure.

In the alternative, or in addition to the RF electrode variations just described, the circumferential ablation element may also include other ablative energy sources or sinks, and particularly may include a thermal conductor that circumscribes the outer circumference of the working length of an expandable member. Examples of suitable thermal conductor arrangements include a metallic element which may, for example, be constructed as previously described for the more detailed RF embodiments above. However, in the thermal conductor embodiment such a metallic element would be generally either resistively heated in a closed loop circuit internal to the catheter, or conductively heated by a heat source coupled to the thermal conductor. In the latter case of conductive heating of the thermal conductor with a heat source, the expandable member may be, for example, a polymeric balloon skin which is inflated with a fluid that is heated either by a resistive coil or by bipolar RF current. In any case, it is believed that a thermal conductor on the outer surface of the expandable member is suitable when it is adapted to heat tissue adjacent thereto to a temperature between 40deg and 80deg Celsius.

Further to the thermal conduction variation for the circumferential ablation element, the perfusion balloon embodiment as shown in Figures 6A-B may be particularly useful in such a design. It is believed that ablation through increased temperatures, as provided by example above may also enhance coagulation of blood in the pulmonary vein adjacent to the expandable member, which blood would otherwise remain stagnant without such a perfusion feature.

One further circumferential ablation element design which is believed to be highly useful in performing the methods according to the present invention is shown in Figure 13 to include a circumferential ablation member(600) with two insulators (602,604) that encapsulate the proximal and distal ends, respectively, of the working length *L* of an expandable member (610). In the particular embodiment shown, the insulators (602,604) are thermal insulators, such as a thermal insulator comprising a Teflon material. Expandable member (610) is an inflatable balloon which has a balloon skin (612) that is thermally conductive to surrounding tissue when inflated with a heated fluid which may contain a radiopaque agent, saline fluid, ringers lactate, combinations thereof, other known biocompatible fluids having acceptable heat transfer properties for these purposes, further to the thermal conductor embodiments previously described. By providing these spaced insulators, a circumferential ablation element is formed as an equatorial band (603) of uninsulated balloon skin is located between the opposite insulators. In this configuration, the circumferential ablation element is able to conduct heat externally of the balloon skin much more efficiently at the uninsulated equatorial band (603) than at the insulated portions, and thereby is adapted to ablate only a circumferential region of tissue in a pulmonary vein wall which is adjacent to the equatorial band. It is further noted that this embodiment is not limited to an "equatorial" placement of the ablation element. Rather, a circumferential band may be formed anywhere along the working length of the expandable member and circumscribing the longitudinal axis of the expandable member as previously described.

Figure 13 further shows use of a radiopaque marker (620) to identify the location of the equatorial band (603) in order to facilitate placement of that band at a selected ablation region of a pulmonary vein via X-ray visualization. Radiopaque marker (620) is opaque under X-ray, and may be constructed, for example, of a radiopaque metal such as gold, platinum, or tungsten, or may comprise a radiopaque polymer such as a metal loaded polymer. Figure 13 shows radiopaque marker (620) positioned coaxially over an inner tubular member (621) which is included in a coaxial catheter design as would be apparent to one of ordinary skill. The present invention contemplates the combination of such a radiopaque marker additionally in the other embodiments herein shown and described. To note, when the circumferential ablation member which forms an equatorial band includes a metallic electrode element, such electrode may itself be radiopaque and may not require use of a separate marker as just described.

The thermal insulator embodiment just described by reference to Figure 13 is illustrative of a broader embodiment, wherein a circumferential ablation member has an ablating surface along the entire working length of an expandable member, but is shielded from releasing ablative energy into surrounding tissues except for along an unshielded or uninsulated equatorial band. As such, the insulator embodiment contemplates other ablation elements, such as the RF embodiments previously described above, which are provided along the entire working length of an expandable member and which are insulated at their ends to selectively ablate tissue only about an uninsulated equatorial band.

In a further example using the insulator embodiment in combination with a circumferential RF electrode embodiment, a metallized balloon which includes a conductive balloon skin may have an electrical insulator, such as a polymeric coating, at each end of the working length and thereby selectively ablate tissue with electricity flowing through the uninsulated equatorial band. In this and other insulator embodiments, it is further contemplated that the insulators described may be only partial and still provide the equatorial band result. For instance, in the conductive RF electrode balloon case, a partial electrical insulator will allow a substantial component of current to flow through the uninsulated portion due to a "shorting" response to the lower resistance in that region.

In still a further example of an insulator combined with an RF ablation electrode, a porous membrane comprises the entire balloon skin of an expandable member. By insulating the proximal and distal end portions of the working length of the expandable member, only the pores in the unexposed equatorial band region are allowed to effuse the electrolyte which carries an ablative RF current.

Further to the expandable member design for use in a circumferential ablation element according to the present invention, other expandable members than a balloon are also considered suitable. For example, in one expandable cage embodiment shown in Figure 14, cage (650) comprises coordinating wires (651) and is expandable to engage a desired ablation region in a pulmonary vein.

The radial expansion of cage (650) is accomplished as follows. Sheath (652) is secured around the wires proximally of cage (650). However, core (653), which may be a metallic mandrel such as stainless steel, extends through sheath (652) and distally within cage (650) wherein it terminates in a distal tip (656). Wires (651) are secured to distal tip (656), for example, by soldering, welding, adhesive bonding, heat shrinking a polymeric member over the wires, or any combination of these methods. Core (653) is slideable within sheath (652), and may, for example, be housed within a tubular lumen (not shown) within sheath (652), the wires being housed between a coaxial space between the tubular lumen and sheath (652). By moving the sheath (652) relative to core (653) and distal tip (656)(shown by arrows in Figure 14), the cage (650) is collapsible along its longitudinal axis in order to force an outward radial bias (also shown with arrows in Figure 14) to wires (651) in an organized fashion to formed a working length of cage (650) which is expanded (not shown).

Further to the particular expandable cage embodiment shown in Figure 14, a plurality of ablation electrodes (655) is shown, each being positioned on one of wires (651) and being similarly located along the longitudinal axis of the cage (650). The radial bias given to wires (651) during expansion, together with the location of the ablation electrodes (655), serves to position the plurality of ablation electrodes/elements (655) along a circumferential, equatorial band along the expanded working length of cage (650). The wires forming a cage according to this embodiment may also have another predetermined shape when in the radially expanded position. For example, a taper similar to that shown for expandable member (370) in Figures 8A-B may be formed by expanding cage (650), wherein the ablation element formed by ablation electrodes (655) may be positioned between the proximal end and the distal end of the taper.

Further to the construction of the embodiment shown in Figure 14, wires (651) are preferably metal, and may comprise stainless steel or a superelastic metal alloy, such as an alloy of nickel and titanium, or a combination of both. Regarding the case of nickel and titanium construction for wires (655), a separate electrical conductor may be required in order to actuate ablation electrodes (655) to efficiently emit ablative current into surrounding tissues. In the case where wires (651) are constructed of stainless steel, they may also serve as electrical conductors for ablation electrodes (655). Further to the stainless steel design, the wires (651) may be coated with an electrical insulator to isolate the electrical flow into surrounding tissues at the site of the ablation electrodes (655). Moreover, the ablation electrodes (655) in the stainless steel wire variation may be formed simply by removing electrical insulation in an isolated region to allow for current to flow into tissue only from that exposed region.

In a further cage embodiment (not shown) to that shown in Figure 14, a circumferential strip of electrodes may also be secured to the cage (650) such that the strip circumscribes the cage at a predetermined location along the cage's longitudinal axis. By expanding cage (650) as previously described, the strip of electrodes are adapted to take a circumferential shape according to the shape of the expanded cage (650). Such an electrode strip is preferably flexible, such that it may be easily reconfigured when the cage is adjusted between the radially collapsed and expanded positions and such that the strip may be easily advanced and withdrawn with the cage within the delivery sheath. Furthermore, the electrode strip may be a continuous circumferential electrode such as a conductive spring coil, or may be a flexible strip which includes several separate electrodes along its circumferential length. In the latter case, the flexible strip may electrically couple all of the electrodes to a conductive lead that interfaces with a drive circuit, or each electrode may be separately coupled to one or more such conductive leads.

Another circumferential ablation element adapted for use in the circumferential conduction block assembly according to the present invention is shown in Figure 15, wherein circumferential ablation member (700) includes a looped member (710) attached, preferably by heat shrinking, to a distal end of a pusher (730). Looped member (710) and pusher (730) are slideably engaged within delivery sheath (750) such that looped member (710) is in a first collapsed position when positioned and radially confined within delivery sheath (750), and expands to a second expanded position when advanced distally from delivery sheath (750).

Looped member (710) is shown in more detail in Figure 15 to include a core (712) which is constructed of a superelastic metal alloy such as a nickel-titanium alloy and which has a looped portion with shape memory in the looped configuration. This looped configuration is shown in Figure 15 to be in a plane which is off-axis, preferably perpendicular, to the longitudinal axis of the pusher (730). This off-axis orientation of the loop is adapted to engage a circumferential path of tissue along a pulmonary vein wall which circumscribes the pulmonary vein lumen when the looped member (710) is delivered from the delivery sheath (750) when the delivery sheath is positioned within the vein lumen parallel to its longitudinal axis. An ablation electrode (714) is also shown in Figure 15 as a metallic coil which is wrapped around core (712) in its looped portion.

Pusher (730) is further shown in Figure 15 to include a tubular pusher member (732) which is heat shrunk over two ends (712') of core (712) which extend proximally of looped member (710) through pusher (730) in the particular variation shown. While in this embodiment core (712) extends through the pusher in order to provide stiffness to the composite design for the pusher, it is further contemplated that the superelastic metal of the core may be replaced or augmented in the pusher region with another different mandrel or pusher core (not shown), such as a stiffer stainless steel mandrel. Also shown within pusher (730) is an electrically conductive lead (735) which is coupled to the ablation electrode (714) and which is also adapted in a proximal region of the pusher (not shown) to couple to an ablation actuator (190) such as an RF current source (shown schematically).

### Ultrasound Circumferential Ablation Member

Figures 16A-19B show various specific embodiments of a broader circumferential ablation device assembly which utilizes an ultrasonic energy source to ablate tissue. The present circumferential ablation device has particular utility in connection with forming a circumferential lesion within or about a pulmonary vein ostium or within the vein itself in order to form a circumferential conductive block. This application of the present ablation device, however, is merely exemplary, and it is understood that those skilled in the art can readily adapt the present ablation device for applications in other body spaces.

As common to each of the following embodiments, a source of acoustic energy is provided a delivery device that also includes an anchoring mechanism. In one mode, the anchoring device comprises an expandable member that also positions the acoustic energy source within the body; however, other anchoring and positioning devices may also be used, such as, for example, a basket mechanism. In a more specific form, the acoustic energy source is located within the expandable member and the expandable member is adapted to engage a circumferential path of tissue either about or along a pulmonary vein in the region of its ostium along a left atrial wall. The acoustic energy source in turn is acoustically coupled to the wall of the expandable member and thus to the circumferential region of tissue engaged by the expandable member wall by emitting a circumferential and longitudinally collimated ultrasound signal when actuated by an acoustic energy driver. The use of acoustic energy, and particularly ultrasonic energy, offers the advantage of simultaneously applying a dose of energy sufficient to ablate a relatively large surface area within or near the heart to a desired heating depth without exposing the heart to a large amount of current. For example, a collimated ultrasonic transducer can form a lesion, which has about a 1.5 mm width, about a 2.5 mm diameter lumen, such as a pulmonary vein and of a sufficient depth to form an effective conductive block. It is believed that an effective conductive block can be formed by producing a lesion within the tissue that is transmural or substantially transmural. Depending upon the patient as well as the location within the pulmonary vein ostium, the lesion may have a depth of 1 millimeter to 10 millimeters. It has been observed that the collimated ultrasonic transducer can be powered to provide a lesion having these parameters so as to form an effective conductive block between the pulmonary vein and the posterior wall of the left atrium.

With specific reference now to the embodiment illustrated in Figures 16A through 16D, a circumferential ablation device assembly (800) includes an elongate body (802) with proximal and distal end portions (810,812), an expandable balloon (820) located along the distal end portion (812) of elongate body (802), and a circumferential ultrasound transducer (830) which forms a circumferential ablation member which is acoustically coupled to the expandable balloon (820). In more detail, Figures 16A-C variously show elongate body (802) to include guidewire lumen (804), inflation lumen (806), and electrical lead lumen (808). The ablation device, however, can be of a self steering type rather than an over-the-wire type device.

Each lumen extends between a proximal port (not shown) and a respective distal port, which distal ports are shown as distal guidewire port (805) for guidewire lumen (804), distal inflation port (807) for inflation lumen (806), and distal lead port (809) for electrical lead lumen (808). Although the guidewire, inflation and electrical lead lumens are generally arranged in a side-by-side relationship, the elongate body (802) can be constructed with one or more of these lumens arranged in a coaxial relationship, or in any of a wide variety of configurations that will be readily apparent to one of ordinary skill in the art.

In addition, the elongate body (802) is also shown in Figures 16A and 16C to include an inner member (803) which extends distally beyond distal inflation and lead ports (807,809), through an interior chamber formed by the expandable balloon (820), and distally beyond expandable balloon (820) where the elongate body terminates in a distal tip. The inner member (803) forms the distal region for the guidewire lumen (804) beyond the inflation and lead ports, and also provides a support member for the cylindrical ultrasound transducer (830) and for the distal neck of the expansion balloon, as described in more detail below.

One more detailed construction for the components of the elongate body (802) which is believed to be suitable for use in transeptal left atrial ablation procedures is as follows. The elongate body (802) itself may have an outer diameter provided within the range of from about 5 French (1.65 mm) to about 10 French (3.3 mm) and more preferable from about 7 French (2.31 mm) to about 9 French (2.97 mm). The guidewire lumen preferably adapted to slideably receive guidewires ranging from about 0.010 inch (0.254 mm) to about 0.038 inch (0.965 mm) in diameter and preferably is adapted for use with guidewires ranging from about 0.018 inch (0.457 mm) to about 0.035 inch (0.889 mm) in diameter. Where a 0.035 (0.889 mm) inch guidewire is to be used, the guidewire lumen preferably has an inner diameter of 0.040 inch (1.02 mm) to about 0.042 inch (1.07 mm). In addition the inflation lumen preferably has an inner diameter of about 0.020 inch (0.508 mm) in order to allow for rapid deflation times, although may vary based upon the viscosity of inflation medium used, length of the lumen, and other dynamic factors relating to fluid flow and pressure.

In addition to providing the requisite lumens and support members for the ultrasound transducer assembly, the elongate body (802) of the present embodiment must also be adapted to be introduced into the left atrium such that the distal end portion with balloon and transducer may be placed within the pulmonary vein ostium in a percutaneous translumenal procedure, and even more preferably in a transeptal procedure as otherwise herein provided. Therefore, the distal end portion (812) is preferably flexible and adapted to track over and along a guidewire seated within the targeted pulmonary vein. In one further more detailed construction which is believed to be suitable, the proximal end portion is adapted to be at least 30% more stiff than the distal end portion. According to this relationship, the proximal end portion may be suitably adapted to provide push transmission to the distal end portion while the distal end portion is suitably adapted to track through bending anatomy during *in vivo* delivery of the distal end portion of the device into the desired ablation region.

Notwithstanding the specific device constructions just described, other delivery mechanisms for delivering the ultrasound ablation member to the desired ablation region are also contemplated. For example, while the Figure 16A variation is shown as an "over-the-wire" catheter construction, other guidewire tracking designs may be suitable substitutes, such as, for example, catheter devices which are known as "rapid exchange" or "monorail" variations wherein the guidewire is only housed coaxially within a lumen of the catheter in the distal regions of the catheter. In another example, a deflectable tip design may also be a suitable substitute and which is adapted to independently select a desired pulmonary vein and direct the transducer assembly into the desired location for ablation. Further to this latter variation, the guidewire lumen and guidewire of the Figure 16A variation may be replaced with a "pullwire lumen and associated fixed pullwire which is adapted to deflect the catheter tip by applying tension along varied stiffness transitions along the catheter's length. Still further to this pullwire variation acceptable pullwires may have a diameter within the range from about 0.008 inch (0.203 mm) to about 0.020 inch (0.508 mm), and may further include a taper, such as for example, a tapered outer diameter from about 0.020 inch (0.508 mm) to about 0.008 inch (0.203 mm).

More specifically regarding expandable balloon (820) as shown in varied detail between Figures 16A and 16C, a central region (822) is generally coaxially disposed over the inner member (803) and is bordered at its end neck regions by proximal and distal adaptions (824,826). The proximal adaption (824) is sealed over elongate body (802) proximally of the distal inflation and the electrical lead ports (807,809), and the distal adaption (826) is sealed over inner member (803). According to this arrangement, a fluid tight interior chamber is formed within expandable balloon (820). This interior chamber is fluidly coupled to a pressurizeable fluid source (not shown) via inflation lumen (806). In addition to the inflation lumen (806), electrical lead lumen (808) also communicates with the interior chamber of expandable balloon (820) so that the ultrasound transducer (830), which is positioned within that chamber and over the inner member (803), may be electrically coupled to an ultrasound drive source or actuator, as will be provided in more detail below.

The expandable balloon (820) may be constructed from a variety of known materials, although the balloon (820) preferably is adapted to conform to the contour of a pulmonary vein ostium. For this purpose, the balloon material can be of the highly compliant variety, such that the material elongates upon application of pressure and takes on the shape of the body lumen or space when fully inflated. Suitable balloon materials include elastomers, such as, for example, but without limitation, Silicone, latex, or low durometer polyurethane (for example, a durometer of about 80A).

In addition or in the alternative to constructing the balloon of highly compliant material, the balloon (820) can be formed to have a predefined fully inflated shape (i.e., be preshaped) to generally match the anatomic shape of the body lumen in which the balloon is inflated. For instance, as described below in greater detail, the balloon can have a distally tapering shape to generally match the shape of a pulmonary vein ostium, and/or can include a bulbous proximal end to generally match a transition region of the atrium posterior wall adjacent to the pulmonary vein ostium. In this manner, the desired seating within the irregular geometry of a pulmonary vein or vein ostium can be achieved with both compliant and non-compliant balloon variations.

Notwithstanding the alternatives which may be acceptable as just described, the balloon (820) is preferably constructed to exhibit at least 300% expansion at 3 atmospheres (3.04x10⁵Pa) of pressure, and more preferably to exhibit at least 400% expansion at that pressure. The term "expansion" is herein intended to mean the balloon outer diameter after pressurization divided by the balloon inner diameter before pressurization, wherein the balloon inner diameter before pressurization is taken after the balloon is substantially filled with fluid in a taught configuration. In other words, "expansion" is herein intended to relate to charge in diameter that is attributable to the material compliance in a stress strain relationship. In one more detailed construction which is believed to be suitable for use in most conduction block procedures in the region of the pulmonary veins, the balloon is adapted to expand under a normal range of pressure such that its outer diameter may be adjusted from a radially collapsed position of about 5 millimeters to a radially expanded position of about 2.5 centimeters (or approximately 500% expansion ratio).

The ablation member, which is illustrated in Figures 16A-D, takes the form of annular ultrasonic transducer (830). In the illustrated embodiment, the annular ultrasonic transducer (830) has a unitary cylindrical shape with a hollow interior (i.e., is tubular shaped); however, the transducer applicator (830) can have a generally annular shape and be formed of a plurality of segments. For instance, the transducer applicator (830) can be formed by a plurality of tube sectors that together form an annular shape. The tube sectors can also be of sufficient arc lengths so as when joined together, the sectors assembly forms a "clover-leaf" shape. This shape is believed to provide overlap in heated regions between adjacent elements. The generally annular shape can also be formed by a plurality of planar transducer segments which are arranged in a polygon shape (e.g., hexagon). In addition, although in the illustrated embodiment the ultrasonic transducer comprises a single transducer element, the transducer applicator can be formed of a multi-element array, as described in greater detail below.

As is shown in detail in Figure 16D, cylindrical ultrasound transducer (830) includes a tubular wall (831) which includes three concentric tubular layers. The central layer (832) is a tubular shaped member of a piezoceramic or piezoelectric crystalline material. The transducer preferably is made of type PZT-4, PZT-5 or PZT-8, quartz or Lithium-Niobate type piezoceramic material to ensure high power output capabilities. These types of transducer materials are commercially available from Stavely Sensors, Inc. of East Hartford, Connecticut, or from Valpey-Fischer Corp. of Hopkinton, Massachusetts.

The outer and inner tubular members (833,834) enclose central layer (832) within their coaxial space and are constructed of an electrically conductive material. In the illustrated embodiment, these transducer electrodes (833, 834) comprise a metallic coating, and more preferably a coating of nickel, copper, silver, gold, platinum, or alloys of these metals.

One more detailed construction for a cylindrical ultrasound transducer for use in the present application is as follows. The length of the transducer (830) or transducer assembly (e.g., multi-element array of transducer elements) desirably is selected for a given clinical application. In connection with forming circumferential condition blocks in cardiac or pulmonary vein wall tissue, the transducer length can fall within the range of approximately 2 mm up to greater than 10 mm, and preferably equals about 5 mm to 10 mm. A transducer accordingly sized is believed to form a lesion of a width sufficient to ensure the integrity of the formed conductive block without undue tissue ablation. For other applications, however, the length can be significantly longer.

Likewise, the transducer outer diameter desirably is selected to account for delivery through a particular access path (e.g., percutaneously and transeptally), for proper placement and location within a particular body space, and for achieving a desired ablation effect. In the given application within or proximate of the pulmonary vein ostium, the transducer (830) preferably has an outer diameter within the range of about 1.8 mm to greater than 2.5 mm. It has been observed that a transducer with an outer diameter of about 2 mm generates acoustic power levels approaching 20 Watts per centimeter radiator or greater within myocardial or vascular tissue, which is believed to be sufficient for ablation of tissue engaged by the outer balloon for up to about 2 cm outer diameter of the balloon. For applications in other body spaces, the transducer applicator (830) may have an outer diameter within the range of about 1 mm to greater than 3-4 mm (e.g., as large as 1 to 2 cm for applications in some body spaces).

The central layer (832) of the transducer (830) has a thickness selected to produce a desired operating frequency. The operating frequency will vary of course depending upon clinical needs, such as the tolerable outer diameter of the ablation and the depth of heating, as well as upon the size of the transducer as limited by the delivery path and the size of the target site. As described in greater detail below, the transducer (830) in the illustrated application preferably operates within the range of about 5 MHz to about 20 MHz, and more preferably within the range of about 7 MHz to about 10 MHz. Thus, for example, the transducer can have a thickness of approximately 0.3 mm for an operating frequency of about 7 MHz (i.e., a thickness generally equal to ½ the wavelength associated with the desired operating frequency).

The transducer (830) is vibrated across the wall thickness and to radiate collimated acoustic energy in the radial direction. For this purpose, as best seen in Figures 16A and 16D, the distal ends of electrical leads (836,837) are electrically coupled to outer and inner tubular members or electrodes (833,834), respectively, of the transducer (830), such as, for example, by soldering the leads to the metallic coatings or by resistance welding. In the illustrated embodiment, the electrical leads are 4-8 mil (0.004 to 0.008 inch diameter or 0.1016 mm to 0.2032 diameter) silver wire or the like.

The proximal ends of these leads are adapted to couple to an ultrasonic driver or actuator (840), which is schematically illustrated in Figure 16D. Figures 16A-D further show leads (836,837) as separate wires within electrical lead lumen (808), in which configuration the leads must be well insulated when in close contact. Other configurations for leads (836,837) are therefore contemplated. For example, a coaxial cable may provide one cable for both leads which is well insulated as to inductance interference. Or, the leads may be communicated toward the distal end portion (812) of the elongate body through different lumens which are separated by the catheter body.

The transducer also can be sectored by scoring or notching the outer transducer electrode (833) and part of the central layer (832) along lines parallel to the longitudinal axis L of the transducer (830), as illustrated in Figure 16E. A separate electrical lead connects to each sector in order to couple the sector to a dedicated power control that individually excites the corresponding transducer sector. By controlling the driving power and operating frequency to each individual sector, the ultrasonic driver (840) can enhance the uniformity of the ultrasonic beam around the transducer (830), as well as can vary the degree of heating (i.e., lesion control) in the angular dimension.

The ultrasound transducer just described is combined with the overall device assembly according to the present embodiment as follows. In assembly, the transducer (830) desirably is "air-backed" to produce more energy and to enhance energy distribution uniformity, as known in the art. In other words, the inner member (803) does not contact an appreciable amount of the inner surface of transducer inner tubular member (834). This is because the piezoelectric crystal which forms central layer (832) of ultrasound transducer (830) is adapted to radially contract and expand (or radially "vibrate") when an alternating current is applied from a current source and across the outer and inner tubular electrodes (833,834) of the crystal via the electrical leads (836,837). This controlled vibration emits the ultrasonic energy which is adapted to ablate tissue and form a circumferential conduction block according to the present embodiment. Therefore, it is believed that appreciable levels of contact along the surface of the crystal may provide a dampening effect which would diminish the vibration of the crystal and thus limit the efficiency of ultrasound transmission.

For this purpose, the transducer (830) seats coaxially about the inner member (803) and is supported about the inner member (803) in a manner providing a gap between the inner member (803) and the transducer inner tubular member (834). That is, the inner tubular member (834) forms an interior bore (835) which loosely receives the inner member (803). Any of a variety of structures can be used to support the transducer (830) about the inner member (803). For instance, spacers or splines can be used to coaxially position the transducer (830) about the inner member (803) while leaving a generally annular space between these components. In the alternative, other conventional and known approaches to support the transducer can also be used. For instance, O-rings that circumscribe the inner member (803) and lie between the inner member (803) and the transducer (830) can support the transducer (830) in a manner similar to that illustrated in U.S. Patent No. 5,606,974, issued March 4, 1997, and entitled "Catheter Having Ultrasonic Device." More detailed examples of the alternative transducer support structures just described are respectfully disclosed in the following references: U.S. Patent No. 5,620,479 to Diederich, issued April 15, 1997, and entitled "Method and Apparatus for Thermal Therapy of Tumors," and U.S. Patent No. 5,606,974 to Castellano, issued March 4, 1997, and entitled "Catheter Having Ultrasonic Device."

In the illustrated embodiment, a stand-off (838) is provided in order to ensure that the transducer (830) has a radial separation from the inner member (803) to form a gap filled with air and/or other fluid. In one preferred mode shown in Figure 16C, stand-off (838) is a tubular member with a plurality of circumferentially spaced outer splines (839) which hold the majority of the transducer inner surface away from the surface of the stand-off between the splines, thereby minimizing dampening affects from the coupling of the transducer to the catheter. The tubular member which forms a stand-off such as stand-off (838) in the Figure 16C embodiment may also provide its inner bore as the guidewire lumen in the region of the ultrasound transducer, in the alternative to providing a separate stand-off coaxially over another tubular member which forms the inner member, such as according to the Figure 16C embodiment.

In a further mode, the elongate body (802) can also include additional lumens which lie either side by side to or coaxial with the guidewire lumen (804) and which terminate at ports located within the space between the inner member (803) and the transducer (830). A cooling medium can circulate through space defined by the stand-off (838) between the inner member (803) and the transducer (830) via these additional lumens. By way of example, carbon dioxide gas, circulated at a rate of 5 liters per minute, can be used as a suitable cooling medium to maintain the transducer at a lower operating temperature. It is believed that such thermal cooling would allow more acoustic power to transmit to the targeted tissue without degradation of the transducer material.

The transducer (830) desirably is electrically and mechanically isolated from the interior of the balloon (820). Again, any of a variety of coatings, sheaths, sealants, tubings and the like may be suitable for this purpose, such as those described in U.S. Patent Nos. 5,620,479 and 5,606,974. In the illustrated embodiment, as best illustrated in Figure 16C, a conventional, flexible, acoustically compatible, and medical grade epoxy (842) is applied over the transducer (830). The epoxy (842) may be, for example, Epotek 301, Epotek 310, which is available commercially from Epoxy Technology, or Tracon FDA-8. In addition, a conventional sealant, such as, for example, General Electric Silicon II gasket glue and sealant, desirably is applied at the proximal and distal ends of the transducer (830) around the exposed portions of the inner member (803), wires (836, 837) and stand-off (838) to seal the space between the transducer (830) and the inner member (803) at these locations.

An ultra thin-walled polyester heat shrink tubing (844) or the like then seals the epoxy coated transducer. Alternatively, the epoxy covered transducer (830), inner member (803) and stand-off (838) can be instead inserted into a tight thin wall rubber or plastic tubing made from a material such as Teflon®, polyethylene, polyurethane, silastic or the like. The tubing desirably has a thickness of 0.0005 to 0.003 inches (0.0127 to 0.0762 mm).

When assembling the ablation device assembly, additional epoxy is injected into the tubing after the tubing is placed over the epoxy coated transducer (830). As the tube shrinks, excess epoxy flows out and a thin layer of epoxy remains between the transducer and the heat shrink tubing (844). These layers (842, 844) protect the transducer surface, help acoustically match the transducer (830) to the load, makes the ablation device more robust, and ensures air-tight integrity of the air backing.

Although not illustrated in Figure 16A in order to simplify the drawing, the tubing (844) extends beyond the ends of transducer (830) and surrounds a portion of the inner member (803) on either side of the transducer (830). A filler (not shown) can also be used to support the ends of the tubing (844). Suitable fillers include flexible materials such as, for example, but without limitation, epoxy, Teflon® tape and the like.

The ultrasonic actuator (840) generates alternating current to power the transducer (830). The ultrasonic actuator (840) drives the transducer (830) at frequencies within the range of about 5 to about 20 MHz, and preferably for the illustrated application within the range of about 7 MHz to about 10 MHz. In addition, the ultrasonic driver can modulate the driving frequencies and/or vary power in order to smooth or unify the produced collimated ultrasonic beam. For instance, the function generator of the ultrasonic actuator (840) can drive the transducer at frequencies within the range of 6.8 MHz and 7.2 MHz by continuously or discretely sweeping between these frequencies.

The ultrasound transducer (830) of the present embodiment sonically couples with the outer skin of the balloon (820) in a manner which forms a circumferential conduction block in a pulmonary vein as follows. Initially, the ultrasound transducer is believed to emit its energy in a circumferential pattern which is highly collimated along the transducer's length relative to its longitudinal axis *L* (see Figure 16D). The circumferential band therefore maintains its width and circumferential pattern over an appreciable range of diameters away from the source at the transducer. Also, the balloon is preferably inflated with fluid which is relatively ultrasonically transparent, such as, for example, degassed water. Therefore, by actuating the transducer (830) while the balloon (820) is inflated, the circumferential band of energy is allowed to translate through the inflation fluid and ultimately sonically couple with a circumferential band of balloon skin which circumscribes the balloon (820). Moreover, the circumferential band of balloon skin material may also be further engaged along a circumferential path of tissue which circumscribes the balloon, such as, for example, if the balloon is inflated within and engages a pulmonary vein wall, ostium, or region of atrial wall. Accordingly, where the balloon is constructed of a relatively ultrasonically transparent material, the circumferential band of ultrasound energy is allowed to pass through the balloon skin and into the engaged circumferential path of tissue such that the circumferential path of tissue is ablated.

Further to the transducer-balloon relationship just described, the energy is coupled to the tissue largely via the inflation fluid and balloon skin. It is believed that, for *in vivo* uses of the present invention, the efficiency of energy coupling to the tissue, and therefore ablation efficiency, may significantly diminish in circumstances where there is poor contact and conforming interface between the balloon skin and the tissue. Accordingly, it is contemplated that several different balloon types may be provided for ablating different tissue structures so that a particular shape may be chosen for a particular region of tissue to be ablated.

In one particular balloon-transducer combination shown in Figure 16A and also in Figure 18A, the ultrasound transducer preferably has a length such that the ultrasonically coupled band of the balloon skin, having a similar length *d* according to the collimated ultrasound signal, is shorter than the working length *D* of the balloon. According to this aspect of the relationship, the transducer is adapted as a circumferential ablation member which is coupled to the balloon to form an ablation element along a circumferential band of the balloon, therefore forming a circumferential ablation element band which circumscribes the balloon. Preferably, the transducer has a length which is less than two-thirds the working length of the balloon, and more preferably is less than one-half the working length of the balloon. By sizing the ultrasonic transducer length d smaller than the working length D of the balloon (820) - and hence shorter than a longitudinal length of the engagement area between the balloon (820) and the wall of the body space (e.g., pulmonary vein ostium) - and by generally centering the transducer (830) within the balloon's working length D, the transducer (830) operates in a field isolated from the blood pool. A generally equatorial position of the transducer (830) relative to the ends of the balloon's working length also assists in the isolation of the transducer (830) from the blood pool. It is believed that the transducer placement according to this arrangement may be preventative of thrombus formation which might otherwise occur at a lesion sight, particularly in the left atrium.

The ultrasound transducer described in various levels of detail above has been observed to provide a suitable degree of radiopacity for locating the energy source at a desired location for ablating the conductive block. However, it is further contemplated that the elongate body (802) may include an additional radiopaque marker or markers (not shown) to identify the location of the ultrasonic transducer (830) in order to facilitate placement of the transducer at a selected ablation region of a pulmonary vein via X-ray visualization. The radiopaque marker is opaque under X-ray, and can be constructed, for example, of a radiopaque metal such as gold, platinum, or tungsten, or can comprise a radiopaque polymer such as a metal loaded polymer. The radiopaque marker is positioned coaxially over an inner tubular member (803), in a manner similar to that described in connection with the embodiment of Figure 13.

The present circumferential ablation device is introduced into a pulmonary vein of the left atrium in a manner similar to that described above. Once properly positioned within the pulmonary vein or vein ostium, the pressurized fluid source inflates the balloon (820) to engage the lumenal surface of the pulmonary vein ostium. Once properly positioned, the ultrasonic driver (840) is energized to drive the transducer (830). It is believed that by driving the ultrasonic transducer 830 at 20 acoustical watts at an operating frequency of 7 megahertz, that a sufficiently sized lesion can be formed circumferentially about the pulmonary vein ostium in a relatively short period of time (e.g., 1 to 2 minutes or less). It is also contemplated that the control level of energy can be delivered, then tested for lesion formation with a test stimulus in the pulmonary vein, either from an electrode provided at the tip area of the ultrasonic catheter or on a separate device such as a guidewire through the ultrasonic catheter. Therefore, the procedure may involve ablation at a first energy level in time, then check for the effective conductive block provided by the resulting lesion, and then subsequent ablations and testing until a complete conductive block is formed. In the alternative, the circumferential ablation device may also include feedback control, for example, if thermocouples are provided at the circumferential element formed along the balloon outer surface. Monitoring temperature at this location provides indicia for the progression of the lesion. This feedback feature may be used in addition to or in the alternative to the multi-step procedure described above.

Figures 17A-C show various alternative embodiments of the present invention for the purpose of illustrating the relationship between the ultrasound transducer and balloon of the present invention just described above. More specifically, Figure 17A shows the balloon (820) having "straight" configuration with a working length L and a relatively constant diameter X between proximal and distal tapers (824, 826). As is shown in Figure 17A, this variation is believed to be particularly well adapted for use in forming a circumferential conduction block along a circumferential path of tissue which circumscribes and transects a pulmonary vein wall. However, unless the balloon is constructed of a material having a high degree of compliance and conformability, this shape may provide for gaps in contact between the desired circumferential band of tissue and the circumferential band of the balloon skin along the working length of the balloon (820).

The balloon (820) in Figure 17A is also concentrically positioned relative to the longitudinal axis of the elongate body (802). It is understood, however, that the balloon can be asymmetrically positioned on the elongate body, and that the ablation device can include more than one balloon.

Figure 17B shows another assembly according to the invention, although this assembly includes a balloon (820) which has a tapered outer diameter from a proximal outer diameter X₂ to a smaller distal outer diameter X₁. (Like reference numerals have been used in each of these embodiments in order to identify generally common elements between the embodiments.) According to this mode, this tapered shape is believed to conform well to other tapering regions of space, and may also be particularly beneficial for use in engaging and ablating circumferential paths of tissue along a pulmonary vein ostium.

Figure 17C further shows a similar shape for the balloon as that just illustrated by reference to Figure 17B, except that the Figure 17C embodiment further includes a balloon (820) and includes a bulbous proximal end (846). In the illustrated embodiment, the proximate bulbous end (846) of the central region (822) gives the balloon (820) a "pear"-shape. More specifically, a contoured surface (848) is positioned along the tapered working length L and between proximal shoulder (824) and the smaller distal shoulder (826) of balloon (820). As is suggested by view of Figure 17C, this pear shaped embodiment is believed to be beneficial for forming the circumferential conduction block along a circumferential path of atrial wall tissue which surrounds and perhaps includes the pulmonary vein ostium. For example, the device shown in Figure 17C is believed to be suited to form a similar lesion to that shown at circumferential lesion (850) in Figure 17D. Circumferential lesion (850) electrically isolates the respective pulmonary vein (852) from a substantial portion of the left atrial wall. The device shown in Figure 17C is also believed to be suited to form an elongate lesion which extends along a substantial portion of the pulmonary vein ostium (854), e.g., between the proximal edge of the illustrated lesion (850) and the dashed line (856) which schematically marks a distal edge of such an exemplary elongate lesion (850).

As mentioned above, the transducer (830) can be formed of an array of multiple transducer elements that are arranged in series and coaxial. The transducer can also be formed to have a plurality of longitudinal sectors. These modes of the transducer have particular utility in connection with the tapering balloon designs illustrated in Figures 17B and 17C. In these cases, because of the differing distances along the length of the transducer between the transducer and the targeted tissue, it is believed that a non-uniform heating depth could occur if the transducer were driven at a constant power. In order to uniformly heat the targeted tissue along the length of the transducer assembly, more power may therefore be required at the proximal end than at the distal end because power falls off as 1/radius from a source (i.e., from the transducer) in water. Moreover, if the transducer (830) is operating in an attenuating fluid, then the desired power level may need to account for the attenuation caused by the fluid. The region of smaller balloon diameter near the distal end thus requires less transducer power output than the region of larger balloon diameter near the proximal end. Further to this premise, in a more specific embodiment transducer elements or sectors, which are individually powered, can be provided and produce a tapering ultrasound power deposition. That is, the proximal transducer element or sector can be driven at a higher power level than the distal transducer element or sector so as to enhance the uniformity of heating when the transducer lies skewed relative to the target site.

The circumferential ablation device (800) can also include additional mechanisms to control the depth of heating. For instance, the elongate body (802) can include an additional lumen which is arranged on the body so as to circulate the inflation fluid through a closed system. A heat exchanger can remove heat from the inflation fluid and the flow rate through the closed system can be controlled to regulate the temperature of the inflation fluid. The cooled inflation fluid within the balloon (820) can thus act as a heat sink to conduct away some of the heat from the targeted tissue and maintain the tissue below a desired temperature (e.g., 90 decrees C), and thereby increase the depth of heating. That is, by maintaining the temperature of the tissue at the balloon/tissue interface below a desired temperature, more power can be deposited in the tissue for greater penetration. Conversely, the fluid can be allowed to warm. This use of this feature and the temperature of the inflation fluid can be varied from procedure to procedure, as well as during a particular procedure, in order to tailor the degree of ablation to a given application or patient.

The depth of heating can also be controlled by selecting the inflation material to have certain absorption characteristics. For example, by selecting an inflation material with higher absorption than water, less energy will reach the balloon wall, thereby limiting thermal penetration into the tissue. It is believed that the following fluids may be suitable for this application: vegetable oil, silicone oil and the like.

Uniform heating can also be enhanced by rotating the transducer within the balloon. For this purpose, the transducer (830) may be mounted on a torquable member which is movably engaged within a lumen that is formed by the elongate body (802).

Another aspect of the balloon-transducer relationship of the present embodiment is also illustrated by reference to Figures 18A-B. In general as to the variations embodied by those figures, the circumferential ultrasound energy signal is modified at the balloon coupling level such that a third order of control is provided for the tissue lesion pattern (the first order of control is the transducer properties affecting signal emission, such as length, width, shape of the transducer crystal; the second order of control for tissue lesion pattern is the balloon shape, per above by reference to Figures 17A-C).

More particularly, Figure 18A shows balloon (820) to include a filter (860) which has a predetermined pattern along the balloon surface and which is adapted to shield tissue from the ultrasound signal, for example, by either absorbing or reflecting the ultrasound signal. In the particular variation shown in Figure 18A, the filter (860) is patterned so that the energy band which is passed through the balloon wall is substantially more narrow than the band which emits from the transducer (830) internally of the balloon (820). The filter (860) can be constructed, for example, by coating the balloon (820) with an ultrasonically reflective material, such as with a metal, or with an ultrasonically absorbent material, such as with a polyurethane elastomer. Or, the filter (860) can be formed by varying the balloon's wall thickness such that a circumferential band (862), which is narrow in the longitudinal direction as compared to the length of the balloon, is also thinner (in a radial direction) than the surrounding regions, thereby preferentially allowing signals to pass through the band (862). The thicker walls of the balloon (820) on either side of the band (862) inhibit propagation of the ultrasonic energy through the balloon skin at these locations.

For various reasons, the "narrow pass filter" embodiment of Figure 18A may be particularly well suited for use in forming circumferential conduction blocks in left atrial wall and pulmonary vein tissues with the device according to the present invention. It is believed that the efficiency of ultrasound transmission from a piezoelectric transducer is limited by the length of the transducer, which limitations are further believed to be a function of the wavelength of the emitted signal. Thus, for some applications a transducer (830) may be required to be longer than the length which is desired for the lesion to be formed. Many procedures intending to form conduction blocks in the left atrium or pulmonary veins, such as, for example, less-invasive "maze"-type procedures, require only enough lesion width to create a functional electrical block and to electrically isolate a tissue region. In addition, limiting the amount of damage formed along an atrial wall, even in a controlled ablation procedure, pervades as a general concern. However, a transducer that is necessary to form that block, or which may be desirable for other reasons, may require a length which is much longer and may create lesions which are much wider than is functionally required for the block. A "narrow pass" filter along the balloon provides one solution to such competing interests.

Figure 18B shows another variation of the balloon-transducer relationship in an ultrasound ablation assembly according to the present invention. Unlike the variation shown in Figure 18A, Figure 18B shows placement of an ultrasonically absorbent band (864) along balloon (820) and directly in the central region of the emitted energy signal from transducer (830). According to this variation, the ultrasonically absorbent band (864) is adapted to heat to a significant temperature rise when sonically coupled to the transducer via the ultrasound signal. It is believed that some ablation methods may benefit from combining ultrasound/thermal conduction modes of ablation in a targeted circumferential band of tissue. In another aspect of this variation, ultrasonically absorbent band (864) may operate as an energy sink as an aid to control the extent of ablation to a less traumatic and invasive level than would be reached by allowing the raw ultrasound energy to couple directly to the tissue. In other words, by heating the absorbent band (864) the signal is diminished to a level that might have a more controlled depth of tissue ablation. Further to this aspect, absorbent band (864) may therefore also have a width which is more commensurate with the length of the transducer, as is shown in an alternative mode in shadow at absorbent band (864).

In each of the embodiments illustrated in Figures 16A through 18B, the ultrasonic transducer had an annular shape so as to emit ultrasonic energy around the entire circumference of the balloon. The present circumferential ablation device, however, can emit a collimated beam of ultrasonic energy in a specific angular exposure. For instance, as seen in Figure 19A, the transducer can be configured to have only a single active sector (e.g., 180 degree exposure). The transducer can also have a planar shape. By rotating the elongate body (802), the transducer (830) can be swept through 360 degrees in order to form a circumferential ablation. For this purpose, the transducer (830) may be mounted on a torquable member (803), in the manner described above.

Figure 19B illustrates another type of ultrasonic transducer which can be mounted to a torquable member (803) within the balloon (820). The transducer (830) is formed by curvilinear section and is mounted on the inner member (803) with its concave surface facing in a radially outward direction. The inner member (803) desirably is formed with recess that substantially matches a portion of the concave surface of the transducer (830). The inner member (803) also includes longitudinal ridges on the edges of the recess that support the transducer above the inner member such that an air gap is formed between the transducer and the inner member. In this manner, the transducer is "air-backed." This spaced is sealed and closed in the manner described above in connection with the embodiment of Figures 16A-E.

The inverted transducer section produces a highly directional beam pattern. By sweeping the transducer through 360 degrees of rotation, as described above, a circumferential lesion can be formed while using less power than would be required with a planar or tubular transducer.

It is to be further understood that the various modes of the ultrasound-balloon embodiments just illustrated by reference to Figures 16A-19B may be used according to several different particular methods such as those methods otherwise set forth throughout this disclosure. For example, any of the ultrasound transducer embodiments may be used to form a conduction block in order to prevent or treat focal arrhythmia arising from a specific pulmonary vein, or may alternatively or additionally be used for joining adjacent linear lesions in a less-invasive "maze"-type procedure.

While particular detailed description has been herein provided for particular embodiments and variations according to the present invention, it is further understood that various modifications and improvements may be made by one of ordinary skill according to this disclosure and without departing from the scope of the invention as defined in the appended set of claims.

## Claims

1. A tissue ablation device assembly (100) for the treatment of atrial arrhythmia comprising a circumferential ablation member and a delivery assembly (101) that cooperates with the circumferential ablation member to enable intervascular delivery of the circumferential ablation member into a patient's heart,
the delivery assembly (101) including an elongated body (f.i. 802) having a distal end portion and being for placing the circumferential ablation member at a location where a pulmonary vein (52) extends from a left atrium of the patient's heart; and
the circumferential ablation member (160, 350, 450, 550, 600, 700, 800) being for ablating a circumferential region of tissue at the location, the circumferential ablation member including,
an expandable member (170, 270, 370, 470, 570, 610) having a working length (L) with a longitudinal dimension defined between ends of the working length along a longitudinal axis of the elongated body and which is adjustable between a radially collapsed position and a radially expanded position with an expanded outer diameter (OD) which is larger than a radially collapsed diameter, the working length (L) also being positionable along the location and being adapted to engage the circumferential region of tissue when in the radially expanded position, and
an ablation element (f.i. 562) being engaged to the distal end portion (f.i. 812) of the elongated body (802) in a fixed position relative to the working length (L), the ablation element being adapted to ablatively couple to a circumferential area surrounding at least a portion of the working length (L) of the expandable member (f.i. 562) so as to ablate tissue within the circumferential area when the working length of the expandable member is engaged with the circumferential region of tissue in the radially expanded position.

2. A tissue ablation device assembly as in Claim 1, wherein
the expandable member comprising an inflatable balloon (f.i. 820) and the working length (L) of the inflatable balloon being defined between first and second ends and having a first end portion, a second end portion, and an intermediate portion positioned between the first and second end portions, the working length (L) also having an expandable outer diameter when in the radially expanded position which is sufficient along the intermediate portion to engage the circumferential region of tissue; and
the ablation element has a circumferential band (f.i., 352) that is located along the longitudinal axis at a position that generally coincides with the intermediate portion and that is substantially spaced from the first and second ends, the circumferential band also having a band width (W) along the longitudinal axis which is shorter than the working length (L) and being adapted to ablatively couple to at least a substantial portion of the circumferential area surrounding only the intermediate portion such that tissue ablation principally occurs along the area of the circumferential region of tissue which is engaged by the intermediate portion and is spaced from the first and second ends of the working length (L) in the radially expanded position.

3. A tissue ablation device assembly as in Claim 1, wherein the expandable member comprises an inflatable balloon (820), and the ablation element has a single circumferential band disposed along the longitudinal axis at a position generally coinciding with the working length (L) of the inflatable balloon, the circumferential band (352) being adapted to couple to and be actuated by an ablation actuator (190) such that the ablation element ablatively couples to at least a substantial portion of the circumferential area when the working length (L) is radially expanded and engaged to the circumferential region of tissue.

4. A tissue ablation device assembly as in Claim 1, wherein the working length (L) of the expandable member is adjustable between a radially collapsed position and a plurality of radially expanded positions which correspond to a range of outer diameters wherein the working length (L) is adapted to engage each of multiple circumferential regions of tissue, respectively; and the ablation element is adapted in each of the radially expanded positions to ablatively couple to at least a substantial portion of the circumferential area and is therefore also adapted to ablate a substantially continuous, transmural circumferential pattern in each of the multiple circumferential regions of tissue, respectively, when engaged by the working length (L).

5. A tissue ablation device assembly as in Claim 1, wherein the ablation element is substantially spaced from the ends of the working length (L).

6. A tissue ablation device assembly as in Claims 2, wherein the ablation element has a circumferential band (352) that is located along the longitudinal axis at a position that generally coincides with the intermediate portion and that is substantially spaced from the first and second ends, the circumferential band also having a band width (W) along the longitudinal axis which is shorter than the working length (L).

7. A tissue ablation device assembly as in Claim 3, wherein the circumferential band (352) having a band width (W) along the longitudinal axis which is shorter than the working length (L).

8. A tissue ablation device assembly as in Claims 2 or 3, wherein the inflatable balloon (820) when adjusted to at least one radially expanded position is adapted to conform to a pulmonary vein (52).

9. A tissue ablation device assembly as in Claims 4 or 5, wherein the expandable member when adjusted to at least one radially expanded position is adapted to conform to a pulmonary vein (52).

10. A tissue ablation device assembly as in Claims 8 or 9, wherein the working length (L) in at least one radially expanded position has a proximal region (372) and a distal region (374) and a shape having a taper with a distally reducing outer diameter from the proximal region (372) to the distal region (374).

11. A tissue ablation device assembly as in any one of the proceeding claims, wherein the working length (L) of the expandable member when adjusted to the radially expanded position is adapted to engage a pulmonary vein ostium (54).

12. A tissue ablation device assembly of Claim 11, wherein the working length (L) of the expandable member in the radially expanded position has a proximal region and a distal region and also has a tapered shape with a distally reducing outer diameter from the proximal region to the distal region.

13. A tissue ablation device assembly as in any one of Claims 2, 3, or 8, wherein the inflatable balloon is constructed of a compliant balloon material.

14. A tissue ablation device assembly as in Claims 9 or 11, wherein the expandable member comprises an inflatable balloon (820) that forms a portion of a balloon chamber which is adapted to fluidly couple to a pressurizeable fluid source (175), and the inflatable balloon (820) comprises a compliant balloon material.

15. A tissue ablation device assembly as in Claim 14, wherein the inflatable balloon (820) is adapted to conform to the pulmonary vein ostium (54) when said inflatable balloon is advanced in the radially expanded position retrogradedly from a left atrium into the pulmonary vein (52) and at least partially against the pulmonary vein ostium (54).

16. A tissue ablation device assembly as in any of Claims 2, 3, 8,13,14 and 15, wherein the balloon material is selected from the group of materials consisting of silicone, latex rubber, polyvinyl chloride, polyurethane and blends and combinations thereof.

17. A tissue ablation device assembly as in Claims 4 or 5, wherein the expandable member (f.i. 470) comprises a basket.

18. A tissue ablation device assembly as in Claims 4 or 5, wherein the expandable member (f.i. 470) comprises a coil.

19. A tissue ablation device assembly as in an one of the proceeding claims, wherein the ablation element comprises an ablation electrode (655) which is adapted to electrically couple to an electrical current source (190) and also to couple to at least a substantial portion of the circumferential area.

20. A tissue ablation device assembly as in Claim 19, wherein the ablation electrode (655) further comprises at least one electrode element positioned on an outer surface of the working length (L) and has a shape which is adapted to form the circumferential band (152, 352).

21. A tissue ablation device assembly as in Claim 19, wherein
the expandable member forms an outer skin of a conductive fluid chamber that is adapted to couple to a pressurizeable source (195) of electrically conductive fluid;
the circumferential band (352) is adapted to ablatively couple the electrically conductive fluid within the conductive fluid chamber and the circumferential region of tissue engaged to the circumferential band (352) in the radially expanded position; and
the ablation electrode (655) is adapted to electrically couple to the electrically conductive fluid within the conductive fluid chamber and therefore also to electrically couple to at least a substantial portion of the circumferential region of tissue engaged by the circumferential band (152, 352).

22. The tissue ablation device assembly of Claim 21, wherein the circumferential band (352) further comprising a porous membrane which is adapted to pass the electrically conductive fluid from the conductive fluid chamber and into the circumferential region of tissue engaged by the circumferential band (352).

23. A tissue ablation device assembly as in Claims 19 or 20, wherein the circumferential band (352) further comprises a secondary shape relative to the longitudinal axis and having a circumferential band length relative to the longitudinal axis, the secondary shape being adapted such that the circumferential band width (W) shortens as the expanded outer diameter of the circumferential band (352) increases when the working length (L) is expanded from a first radially expanded position to a second radially expanded position.

24. A tissue ablation device assembly as in any of Claims 2-18, wherein the ablation element further comprises a thermal conductor which is adapted to couple to a thermal ablation actuator and also to the circumferential band (352), such that thermal energy flows from the thermal conductor and into at least a substantial portion of the circumferential area of tissue engaged by the circumferential band (352).

25. A tissue ablation device assembly as in Claim 24, wherein the thermal conductor further comprises an electrical conductor which is adapted to electrically couple to an electrical current source (190) and also to resistively heat and emit thermal energy into the circumferential region of tissue engaged by the circumferential band (352).

26. A tissue ablation device assembly as in Claim 24, wherein the thermal conductor is adapted to couple to a thermal energy source and also to be conductively heated and thereby emit thermal energy into the circumferential region of tissue engaged by the circumferential band (352).

27. A tissue ablation device assembly as in Claim 24, wherein the circumferential ablation member further comprises a chamber that is adapted to fluidly couple to a pressurizeable source (195) of thermally conductive fluid, and a heater element adapted to thermally couple to the thermally conductive fluid and also to the circumferential region of tissue.

28. A tissue ablation device assembly as in any one of Claims 2-18, wherein the ablation element further comprising a microwave antenna.

29. A tissue ablation device assembly as in any one or Claims 2-18, wherein the ablation element further comprising a cryogenic element.

30. A tissue ablation device assembly as in any one of Claims 2-18, wherein the ablation element further comprises a porous membrane.

31. A tissue ablation device assembly as in any one of Claims 2-18, the ablation element further comprising an ultrasonic energy applicator (840).

32. A tissue ablation device assembly as in any of Claims 19 and 28-31, wherein the circumferential ablation member further comprises:
a first shield, a second shield, and a middle region extending between the first and second shields relative to the longitudinal axis and which encompasses at least in part the ablation element,
the first and second shields being adapted to engage first and second adjacent regions of tissue on respective opposite sides of the circumferential area of tissue and to shield the first and second adjacent regions of tissue from coupling to the ablation element to thereby isolate ablative energy from the middle region and to the circumferential region of tissue.

33. A tissue ablation device assembly as in Claims 31 or 32, wherein the ultrasonic energy applicator (840) and the expandable member are sized to be inserted into a pulmonary vein ostium (54).

34. A tissue ablation device assembly as in Claims 31, 32 or 33, wherein said ultrasonic energy applicator (840) further comprises a single cylindrical ultrasound transducer (830).

35. A tissue ablation device assembly as in any of the preceding claims, wherein the a length of the ablation element along the longitudinal axis is less than two-thirds of the working length (L).

36. A tissue ablation device assembly as in any of the preceding claims, wherein the ablation element in the radially expanded position has a circumference and the length of the ablation element along the longitudinal axis is less than two-thirds of the circumference of the ablation element

37. A tissue ablation device assembly as in Claim 1, wherein the circumferential ablation member further comprises an anchor that is adapted to secure the circumferential ablation member at the location.

38. A tissue ablation device assembly as in Claim 1, wherein the circumferential ablation member has a looped member with a shape that is adapted to contact the circumferential region of tissue, and an ablation element positioned along the shaped region.

39. A tissue ablation device assembly as in Claim 38, wherein the looped member is collapsible within a catheter delivery passageway of the delivery assembly (101).

40. The tissue ablation device assembly as in Claims 38 or 39, wherein the looped member is adjustable from a first configuration, which is adapted to be delivered through a catheter delivery passageway of the delivery assembly (101) into the left atrium, to a second configuration which is adapted to contact at least a substantial portion of the circumferential region of tissue.

41. A tissue ablation device assembly as in Claim 1, wherein the distal end portion of the elongate body includes a guidewire tracking member which is adapted to slideably engage and track over a guidewire (102) of the deliver assembly which is positioned at least partially within the pulmonary vein (52) and through the pulmonary vein ostium (54) such that the circumferential ablation member may be positioned within the pulmonary vein (52) and the ablation element may be ablatively coupled to at least a substantial portion of the circumferential region of tissue.

42. A tissue ablation device assembly as in Claims 1 or 41, wherein the delivery assembly (101) additionally comprises a guiding catheter subassembly having a guiding catheter with an inner lumen terminating at a distal tip which is adapted to be positioned within the left atrium and to deliver the distal end portion of the elongate body including the circumferential ablation member into the left atrium through the inner lumen.

43. A tissue ablation device assembly as in Claim 42, wherein the guiding catheter assembly is adapted to position the guiding catheter (101) within a right atrium and across a fossa ovalis such that the distal tip of the guiding catheter (101) is within the left atrium and further such that the guiding catheter (101) is adapted to deliver the distal end portion of the elongate body including the circumferential ablation member transeptally into the left atrium.

44. A tissue ablation device assembly as in any one of Claims 1, 42 or 43, wherein the elongated body includes a fixed-wire integrated into the body of the catheter.

45. A tissue ablation device assembly as in any one of Claims 1, 42, 43, or 44, wherein the elongated body includes a pullwire lumen and an associated fixed pullwire which is adapted to deflect the catheter tip by applying tension along varied stiffness transitions along the length of the elongated body.

46. A tissue ablation device assembly as in any one of Claims 1 and 41 through wherein the elongated body includes a perfusion lumen (260) located at the distal end portion and defined between a distal end port (242) and a proximal end port (244), the distal end port located distally of the ablation element and the proximal end port located proximally of the ablation element,

47. A tissue ablation device assembly as in any one of Claims 1 and 41 through 45, wherein the distal end portion of the elongated body (802) further comprises a shaped region with a shape that is adapted to position the circumferential ablation member along the circumferential area of tissue.

48. A tissue ablation device assembly as in Claim 47, wherein the shaped region is adjustable from a first configuration, which is substantially straight and unshaped, to a second configuration having the shape.

49. A tissue ablation device assembly as in Claim 47, wherein the delivery member (101) further comprises a deflection member coupled to the shaped region and which is adapted to adjust the shaped region between the first and second configurations.

50. A tissue ablation device assembly as in any of Claims 41-49, additionally **characterized by** a linear ablation member (460) secured to the distal end portion of the elongate member and having a linear ablation element that is adapted to couple to an ablation actuator (190), and the linear ablation member being adapted to engage a region of atrial wall tissue extending from the pulmonary vein such that the linear ablation element (460) may be coupled to and ablate a continuous lesion along the region of atrial wall tissue.

51. A kit including a plurality of circumferential ablation device assemblies as in any of the preceding claims, further **characterized by** each circumferential ablation device assembly including an expandable member with a different expanded outer diameter than the other expandable members of the other circumferential ablation device assemblies within the kit.

## Patentansprüche

1. Gewebeablationsvorrichtung (100) zur Behandlung von Vorhofarrhythmie, umfassend ein Teil zur zirkumferenziellen Ablation und ein Förderorgan (101), das mit dem Teil zur zirkumferenziellen Ablation zusammenwirkt, um eine intervaskuläre Beförderung des Teils zur zirkumferenziellen Ablation in das Herz eines Patienten zu ermöglichen, wobei das Förderorgan (101) einen gestreckten Körper (z. B. 802) einschließt, der einen distalen Endabschnitt aufweist und der Platzierung des Teils zur zirkumferenziellen Ablation an einer Stelle dient, wo eine Lungenvene (52) sich von einem linken Vorhof des Herzens des Patienten weg erstreckt; und das Teil zur zirkumferenziellen Ablation (160, 350, 450, 550, 600, 700, 800) der Ablation einer umgrenzenden Geweberegion an der Stelle dient, wobei das Teil zur zirkumferenziellen Ablation ein aufweitbares Teil (170, 270, 370, 470, 570, 610) mit einer Arbeitslänge (L) einschließt, deren Längsabmessung zwischen Enden der Arbeitslänge entlang einer Längsachse des gestreckten Körpers definiert ist und das zwischen einer radial zusammengefallenen Stellung und einer radial aufgeweiteten Stellung mit einem aufgeweiteten Außendurchmesser (OD) verstellbar ist, der größer ist als ein radial zusammengefallener Durchmesser, wobei die Arbeitslänge (L) auch entlang der Stelle positionierbar und geeignet ist, in der radial aufgeweiteten Stellung an der umgrenzenden Geweberegion anzugreifen, und ein Ablationselement (z. B. 562), das an dem distalen Endabschnitt (z. B. 812) des gestreckten Körpers (802) in einer festen Stellung bezüglich der Arbeitslänge (L) gebunden ist, wobei das Ablationselement geeignet ist, ablativ an eine Umfangsfläche zu koppeln, die zumindest einen Abschnitt der Arbeitslänge (L) des aufweitbaren Teils (z. B. 562) umgibt, um Gewebe innerhalb der Umfangsfläche abzutragen, wenn die Arbeitslänge des aufweitbaren Teils in der radial aufgeweiteten Stellung mit der umgrenzenden Geweberegion im Eingriff ist.

2. Gewebeablationsvorrichtung nach Anspruch 1, worin das aufweitbare Teil einen aufblasbaren Ballon (z. B. 820) umfasst und die Arbeitslänge (L) des aufblasbaren Ballons zwischen dem ersten und zweiten Ende definiert ist und einen ersten Endabschnitt, einen zweiten Endabschnitt und einen zwischen dem ersten und dem zweiten Endabschnitt angeordneten Zwischenabschnitt aufweist, wobei die Arbeitslänge (L) auch in der radial aufgeweiteten Stellung einen erweiterbaren Außendurchmesser aufweist, der entlang dem Zwischenabschnitt zum Erfassen der umgrenzenden Geweberegion ausreicht; und das Ablationselement ein Umfangsband (z. B. 352) aufweist, das entlang der Längsachse an einer Position festgelegt ist, die allgemein mit dem Zwischenabschnitt zusammenfällt und im festen Abstand von dem ersten und zweiten Ende angeordnet ist, wobei das Umfangsband auch eine Bandbreite (W) entlang der Längsachse aufweist, die kürzer ist als die Arbeitslänge (L), und geeignet ist, ablativ an zumindest einen wesentlichen Abschnitt der Umfangsfläche zu koppeln, die nur den Zwischenabschnitt umgibt, so dass die Gewebeablation hauptsächlich entlang der Fläche der umgrenzenden Geweberegion stattfindet, die von dem Zwischenabschnitt erfasst ist und in der radial aufgeweiteten Stellung vom ersten und zweite Ende der Arbeitslänge (L) unterteilt ist.

3. Gewebeablationsvorrichtung nach Anspruch 1, worin das aufweitbare Teil einen aufblasbaren Ballon (820) umfasst und das Ablationselement ein einzelnes Umfangsband aufweist, das entlang der Längsachse an einer allgemein mit der Arbeitslänge (L) des aufblasbaren Ballons zusammenfallenden Position angeordnet ist, wobei das Umfangsband (352) geeignet ist, an ein Ablationsbetätigungselement (190) zu koppeln und davon betätigt zu werden, so dass das Ablationselement ablativ an zumindest einen wesentlichen Abschnitt der Umfangsfläche koppelt, wenn die Arbeitslänge (L) radial gedehnt und an die umgrenzende Geweberegion gebunden ist.

4. Gewebeablationsvorrichtung nach Anspruch 1, worin die Arbeitslänge (L) des aufweitbaren Teils zwischen einer radial zusammengefallenen Stellung und einer Vielzahl von radial aufgeweiteten Stellungen verstellbar ist, die einer Reihe von Außendurchmessern entsprechen, wobei die Arbeitslänge (L) geeignet ist, jeweils jede von vielfachen umgrenzenden Geweberegionen zu erfassen; und das Ablationselement in jeder der radial aufgeweiteten Stellungen geeignet ist, ablativ an zumindest einen wesentlichen Abschnitt der Umfangsfläche zu koppeln und daher auch geeignet ist, jeweils ein weitgehend durchgehendes, transmurales Umfangsmuster in jeder der vielfachen umgrenzenden Geweberegionen abzutragen, wenn sie von der Arbeitslänge (L) erfasst ist.

5. Gewebeablationsvorrichtung nach Anspruch 1, worin das Ablationselement im festen Abstand von den Enden der Arbeitslänge (L) angeordnet ist.

6. Gewebeablationsvorrichtung nach Anspruch 2, worin das Ablationselement ein Umfangsband (352) aufweist, das entlang der Längsachse an einer Position festgelegt ist, die allgemein mit dem Zwischenabschnitt zusammenfällt und im festen Abstand von dem ersten und zweiten Ende angeordnet ist, wobei das Umfangsband auch eine Bandbreite (W) entlang der Längsachse aufweist, die kürzer ist als die Arbeitslänge (L).

7. Gewebeablationsvorrichtung nach Anspruch 3, worin das Umfangsband (352) eine Bandbreite (W) entlang der Längsachse aufweist, die kürzer ist als die Arbeitslänge (L).

8. Gewebeablationsvorrichtung nach Anspruch 2 oder 3, worin der aufblasbare Ballon (820) bei Einstellung auf zumindest eine radial aufgeweitete Stellung geeignet ist, sich an eine Lungenvene (52) anzupassen.

9. Gewebeablationsvorrichtung nach Anspruch 4 oder 5, worin das aufweitbare Teil bei Einstellung auf zumindest eine radial aufgeweitete Stellung geeignet ist, sich an eine Lungenvene (52) anzupassen.

10. Gewebeablationsvorrichtung nach Anspruch 8 oder 9, worin die Arbeitslänge (L) in zumindest einer radial aufgeweiteten Stellung einen proximalen Bereich (372) und einen distalen Bereich (374) sowie eine Gestaltung mit abnehmendem Querschnitt aufweist, wobei sich der Außendurchmesser vom proximalen Bereich (372) zum distalen Bereich (374) distal verjüngt.

11. Gewebeablationsvorrichtung nach einem der vorhergehenden Ansprüche, worin die Arbeitslänge (L) des aufweitbaren Teils bei Einstellung auf die radial aufgeweitete Stellung geeignet ist, ein Lungenvenenostium (54) zu erfassen.

12. Gewebeablationsvorrichtung nach Anspruch 11, worin die Arbeitslänge (L) des aufweitbaren Teils in der radial aufgeweiteten Stellung einen proximalen Bereich und einen distalen Bereich aufweist und auch eine trichterförmige Gestaltung mit einem sich distal verjüngenden Außendurchmesser vom proximalen Bereich zum distalen Bereich aufweist.

13. Gewebeablationsvorrichtung nach einem der Ansprüche 2, 3 oder 8, worin der aufblasbare Ballon aus einem nachgiebigen Ballonmaterial hergestellt ist.

14. Gewebeablationsvorrichtung nach Anspruch 9 oder 11, worin das aufweitbare Teil einen aufblasbaren Ballon (820) umfasst, der einen Abschnitt einer Ballonkammer bildet, die zur fließfähigen Verbindung mit einer druckbelüftbaren Fluidquelle (175) geeignet ist, und der aufblasbare Ballon (820) ein nachgiebiges Ballonmaterial umfasst.

15. Gewebeablationsvorrichtung nach Anspruch 14, worin der aufblasbare Ballon (820) zur Anpassung an das Lungenvenenostium (54) geeignet ist, wenn der aufblasbare Ballon in der radial aufgeweiteten Stellung retrograd von einem linken Vorhof in die Lungenvene (52) und zumindest teilweise gegen das Lungenvenenostium (54) vorgeschoben wird.

16. Gewebeablationsvorrichtung nach einem der Ansprüche 2, 3, 8, 13, 14 und 15, worin das Ballonmaterial ausgewählt ist aus der Gruppe von Werkstoffen bestehend aus Silikon, Latexgummi, Polyvinylchlorid, Polyurethan und Blends und Kombinationen davon.

17. Gewebeablationsvorrichtung nach Anspruch 4 oder 5, worin das aufweitbare Teil (z. B. 470) einen Korb umfasst.

18. Gewebeablationsvorrichtung nach Anspruch 4 oder 5, worin das aufweitbare Teil (z. B. 470) eine Spirale umfasst.

19. Gewebeablationsvorrichtung nach einem der vorhergehenden Ansprüche, worin das Ablationselement eine Ablationselektrode (655) umfasst, die zur elektrischen Verbindung mit einer elektrischen Stromquelle (190) und auch zur Verbindung mit zumindest einem wesentlichen Abschnitt der Umfangsfläche geeignet ist.

20. Gewebeablationsvorrichtung nach Anspruch 19, worin die Ablationselektrode (655) ferner zumindest ein Elektrodenelement umfasst, das an einer Außenfläche der Arbeitslänge (L) angeordnet ist und eine Gestaltung aufweist, die zur Bildung des Umfangsbandes (152, 352) geeignet ist.

21. Gewebeablationsvorrichtung nach Anspruch 19, worin das aufweitbare Teil eine Außenhaut einer leitenden Fluidkammer bildet, die zur Verbindung mit einer druckbelüftbaren Quelle (195) von elektrisch leitender Flüssigkeit geeignet ist; das Umfangsband (352) zur ablativen Kopplung der elektrisch leitenden Flüssigkeit innerhalb der leitenden Fluidkammer und der umgrenzenden Geweberegion geeignet ist, die in der radial aufgeweiteten Stellung an das Umfangsband (352) gebunden ist; und die Ablationselektrode (655) zur elektrischen Verbindung mit der elektrisch leitenden Flüssigkeit innerhalb der leitenden Fluidkammer und damit auch zur elektrischen Verbindung mit zumindest einem wesentlichen Abschnitt der von dem Umfangsband (152, 352) erfassten umgrenzenden Geweberegion geeignet ist.

22. Gewebeablationsvorrichtung nach Anspruch 21, worin das Umfangsband (352) ferner eine poröse Membran umfasst, die zum Durchlassen der elektrisch leitenden Flüssigkeit von der leitenden Fluidkammer und in die von dem Umfangsband (352) erfasste umgrenzende Geweberegion geeignet ist.

23. Gewebeablationsvorrichtung nach Anspruch 19 oder 20, worin das Umfangsband (352) ferner eine sekundäre Gestaltung bezüglich der Längsachse umfasst und mit einer Umfangsbandlänge bezüglich der Längsachse, wobei die sekundäre Gestaltung derart angepasst ist, dass sich die Umfangsbandbreite (W) vermindert, während der aufgeweitete Außendurchmesser des Umfangsbandes (352) zunimmt, wenn die Arbeitslänge (L) von einer ersten radial aufgeweiteten Stellung auf eine zweite radial aufgeweitete Stellung gedehnt wird.

24. Gewebeablationsvorrichtung nach einem der Ansprüche 2-18, worin das Ablationselement ferner einen Wärmeleiter umfasst, der zur Verbindung mit einem Wärme-Ablationsbetätigungselement und auch dem Umfangsband (352) geeignet ist, so dass Wärmeenergie von dem Wärmeleiter weg und in zumindest einen wesentlichen Abschnitt der von dem Umfangsband (352) erfassten umgrenzenden Gewebefläche strömt.

25. Gewebeablationsvorrichtung nach Anspruch 24, worin der Wärmeleiter ferner einen elektrischen Leiter umfasst, der zur elektrischen Verbindung mit einer elektrischen Stromquelle (190) und auch zum resistiven Erwärmen und Abgeben von Wärmeenergie in die von dem Umfangsband (352) erfasste umgrenzende Geweberegion geeignet ist.

26. Gewebeablationsvorrichtung nach Anspruch 24, worin der Wärmeleiter zur Verbindung mit einer Wärmeenergiequelle und auch zur leitenden Erwärmung und **dadurch** zum Abgeben von Wärmeenergie in die von dem Umfangsband (352) erfasste umgrenzende Geweberegion geeignet ist.

27. Gewebeablationsvorrichtung nach Anspruch 24, worin das Teil zur zirkumferenziellen Ablation ferner eine Kammer umfasst, die zur fließfähigen Verbindung mit einer druckbelüftbaren Quelle (195) von wärmeleitfähiger Flüssigkeit geeignet ist, und ein Heizelement umfasst, das zur Wärmeverbindung mit der wärmeleitfähigen Flüssigkeit und auch der umgrenzenden Geweberegion geeignet ist.

28. Gewebeablationsvorrichtung nach einem der Ansprüche 2-18, worin das Ablationselement ferner eine Mikrowellenantenne umfasst.

29. Gewebeablationsvorrichtung nach einem der Ansprüche 2-18, worin das Ablationselement ferner ein Kryoelement umfasst.

30. Gewebeablationsvorrichtung nach einem der Ansprüche 2-18, worin das Ablationselement ferner eine poröse Membran umfasst.

31. Gewebeablationsvorrichtung nach einem der Ansprüche 2-18, wobei das Ablationselement ferner einen Ultraschallenergieapplikator (840) umfasst.

32. Gewebeablationsvorrichtung nach einem der Ansprüche 19 und 28-31, worin das Teil zur zirkumferenziellen Ablation ferner eine erste Abschirmung, eine zweite Abschirmung und eine Mittelregion umfasst, die sich zwischen der ersten und der zweiten Abschirmung bezüglich der Längsachse erstreckt und das Ablationselement zumindest zum Teil umschließt, wobei die erste und zweite Abschirmung geeignet sind, erste und zweite angrenzende Geweberegionen auf jeweiligen Gegenseiten der umgrenzenden Gewebefläche zu erfassen und die ersten und zweiten angrenzenden Geweberegionen vor der Verbindung mit dem Ablationselement abzuschirmen, um **dadurch** ablative Energie von der Mittelregion und zu der umgrenzenden Geweberegion zu isolieren.

33. Gewebeablationsvorrichtung nach Anspruch 31 oder 32, worin der Ultraschallenergieapplikator (840) und das aufweitbare Teil so bemessen sind, dass sie in ein Lungenvenenostium (54) eingeführt werden können.

34. Gewebeablationsvorrichtung nach Anspruch 31, 32 oder 33, worin der Ultraschallenergieapplikator (840) ferner einen einzelnen zylindrischen Ultraschallwandler (830) umfasst.

35. Gewebeablationsvorrichtung nach einem der vorhergehenden Ansprüche, worin eine Länge des Ablationselementes entlang der Längsachse weniger als zwei Drittel der Arbeitslänge (L) beträgt.

36. Gewebeablationsvorrichtung nach einem der vorhergehenden Ansprüche, worin das Ablationselement in der radial aufgeweiteten Stellung einen Kreisumfang aufweist und die Länge des Ablationselementes entlang der Längsachse weniger als zwei Drittel des Kreisumfangs des Ablationselementes beträgt.

37. Gewebeablationsvorrichtung nach Anspruch 1, worin das Teil zur zirkumferenziellen Ablation ferner einen Anker umfasst, der zur Sicherung des Teils zur zirkumferenziellen Ablation an der Stelle geeignet ist.

38. Gewebeablationsvorrichtung nach Anspruch 1, worin das Teil zur zirkumferenziellen Ablation ein Schlingenteil mit einer zum Kontaktieren der umgrenzenden Geweberegion geeigneten Gestaltung und ein entlang der geformten Region angeordnetes Ablationselement aufweist.

39. Gewebeablationsvorrichtung nach Anspruch 38, worin das Schlingenteil innerhalb eines Katheterförderdurchlasses des Förderorgans (101) zusammenlegbar ist.

40. Gewebeablationsvorrichtung nach Anspruch 38 oder 39, worin das Schlingenteil von einer ersten Gestalt, die zur Beförderung durch einen Katheterförderdurchlass des Förderorgans (101) in den linken Vorhof geeignet ist, auf eine zweite Gestalt verstellbar ist, die zum Kontaktieren zumindest eines wesentlichen Abschnitts der umgrenzenden Geweberegion geeignet ist.

41. Gewebeablationsvorrichtung nach Anspruch 1, worin der distale Endabschnitt des länglichen Körpers ein Führungsdraht-Nachführteil einschließt, das zum verschiebbaren Eingriff und zur Führung über einen Führungsdraht (102) des Förderorgans geeignet ist, das zumindest teilweise innerhalb der Lungenvene (52) und durch das Lungenvenenostium (54) hindurch positioniert ist, so dass das Teil zur zirkumferenziellen Ablation innerhalb der Lungenvene (52) angeordnet werden kann und das Ablationselement mit zumindest einem wesentlichen Abschnitt der umgrenzenden Geweberegion ablativ verbunden werden kann.

42. Gewebeablationsvorrichtung nach Anspruch 1 oder 41, worin das Förderorgan (101) zusätzlich eine Führungskatheter-Unteranordnung umfasst, die einen Führungskatheter mit einem Innenlumen aufweist, das an einer distalen Spitze endet, die zum Positionieren innerhalb des linken Vorhofs und Befördern des distalen Endabschnitts des länglichen Körpers einschließlich des Teils zur zirkumferenziellen Ablation in den linken Vorhof durch das Innenlumen hindurch geeignet ist.

43. Gewebeablationsvorrichtung nach Anspruch 42, worin die Führungskatheteranordnung geeignet ist, den Führungskatheter (101) innerhalb eines rechten Vorhofs und über eine Fossa ovalis derart zu positionieren, dass die distale Spitze des Führungskatheters (101) sich innerhalb des linken Vorhofs befindet und ferner derart, dass der Führungskatheter (101) geeignet ist, den distalen Endabschnitt des länglichen Körpers einschließlich des Teils zur zirkumferenziellen Ablation quer in den linken Vorhof zu befördern.

44. Gewebeablationsvorrichtung nach einem der Ansprüche 1, 42 oder 43, worin der gestreckte Körper einen in den Körper des Katheters integrierten Festdraht einschließt.

45. Gewebeablationsvorrichtung nach einem der Ansprüche 1, 42, 43 oder 44, worin der gestreckte Körper ein Handzuglumen und einen zugehörigen ortsfesten Handzug umfasst, der geeignet ist, die Katheterspitze durch Anlegen von Zugkraft entlang mannigfacher Steifigkeitsübergänge entlang der Länge des gestreckten Körpers abzubiegen.

46. Gewebeablationsvorrichtung nach einem der Ansprüche 1 und 41 bis einschließlich 45, worin der gestreckte Körper ein Perfusionslumen (260) einschließt, das an dem distalen Endabschnitt festgelegt und zwischen einem distalen Enddurchlass (242) und einem proximalen Enddurchlass (244) definiert ist, wobei der distale Enddurchlass distal vom Ablationselement liegt und der proximale Enddurchlass proximal vom Ablationselement liegt.

47. Gewebeablationsvorrichtung nach einem der Ansprüche 1 und 41 bis einschließlich 45, worin der distale Endabschnitt des gestreckten Körpers (802) ferner eine geformte Region mit einer Gestaltung umfasst, die zur Anordnung des Teils zur zirkumferenziellen Ablation entlang der umgrenzenden Gewebefläche geeignet ist.

48. Gewebeablationsvorrichtung nach Anspruch 47, worin die geformte Region von einer ersten Gestalt, die weitgehend gerade und unverformt ist, auf eine zweite Gestalt mit der Gestaltung verstellbar ist.

49. Gewebeablationsvorrichtung nach Anspruch 47, worin das Förderteil (101) ferner ein Ablenkteil umfasst, das mit der geformten Region verbunden und geeignet ist, die geformte Region zwischen der ersten und der zweiten Gestalt zu verstellen.

50. Gewebeablationsvorrichtung nach einem der Ansprüche 41-49, zusätzlich **gekennzeichnet durch** ein lineares Ablationsteil (460), das am distalen Endabschnitt des länglichen Teils befestigt ist und ein lineares Ablationselement aufweist, das zur Verbindung mit einem Ablationsbetätigungselement (190) geeignet ist, und wobei das lineare Ablationsteil geeignet ist, eine Vorhofwand-Geweberegion, die sich von der Lungenvene weg erstreckt, derart zu erfassen, dass das lineare Ablationselement (460) mit einer durchgehenden Läsion entlang der Vorhofwand-Geweberegion verbunden werden und diese abtragen kann.

51. Kit mit einer Vielzahl von Vorrichtungen zur zirkumferenzielle Ablation nach einem der vorhergehenden Ansprüche, ferner **dadurch gekennzeichnet, dass** jede Vorrichtung zur zirkumferenziellen Ablation ein aufweitbares Teil mit einem verschieden aufgeweiteten Außendurchmesser als die anderen aufweitbaren Teile der anderen Vorrichtungen zur zirkumferenziellen Ablation innerhalb des Kits einschließt.

## Revendications

1. Ensemble de dispositifs d'ablation de tissu (100) pour le traitement d'une arythmie atriale, comprenant un élément d'ablation circonférentielle et un ensemble de libération (101) qui coopère avec l'élément d'ablation circonférentielle pour permettre une libération intravasculaire de l'élément d'ablation circonférentielle dans le coeur d'un patient,
l'ensemble de libération (101) comprenant un corps allongé (f.i. 802) ayant une portion d'extrémité distale et étant pour placer l'élément d'ablation circonférentielle en un endroit où une veine pulmonaire (52) s'étend à partir d'un atrium gauche du coeur d'un patient ; et
l'élément d'ablation circonférentielle (160, 350, 450, 550, 600, 700, 800) étant pour ablater une région circonférentielle de tissu en l'endroit, l'élément d'ablation circonférentielle comprenant
un élément expansible (170, 270, 370, 470, 570, 610) ayant une longueur utile (L) avec une dimension longitudinale définie entre des extrémités de la longueur utile le long d'un axe longitudinal du corps allongé et qui est ajustable entre une position radialement rétractée et une position radialement expansée avec un diamètre extérieur (OD) expansé qui est plus grand qu'un diamètre radialement rétracté, la longueur utile (L) étant aussi positionnable le long de l'endroit et étant adaptée pour engager la région circonférentielle de tissu quand elle est dans la position radialement expansée, et
un élément d'ablation (f.i. 562) étant engagé dans la portion d'extrémité distale (f.i. 812) du corps allongé (802) dans une position fixe par rapport à la longueur utile (L), l'élément d'ablation étant adaptée pour se coupler de manière ablative à une zone circonférentielle entourant au moins une portion de la longueur utile (L) de l'élément expansible (f.i. 562) de manière à ablater du tissu dans la zone circonférentielle quand la longueur utile de l'élément expansible est engagée avec la région circonférentielle du tissu dans la position radialement expansée.

2. Ensemble de dispositifs d'ablation de tissu selon la revendication 1, dans lequel
l'élément expansible comprend un ballonnet gonflable (f.i. 820) et la longueur utile (L) du ballonnet gonflable est définie entre des première et seconde extrémités et ayant une première portion d'extrémité, une seconde portion d'extrémité, et une portion intermédiaire positionnée entre les première et seconde portions, la longueur utile (L) ayant aussi un diamètre extérieur expansible quand elle est dans une position radialement expansée qui est suffisant le long de la portion intermédiaire pour engager la région circonférentielle de tissu ; et
l'élément d'ablation a une bande circonférentielle (f.i. 352) qui est située le long de l'axe longitudinal en une position qui généralement coïncide avec la portion intermédiaire et qui est sensiblement espacée des première et seconde extrémités, la bande circonférentielle ayant aussi une largeur de bande (W) le long de l'axe longitudinal qui est plus courte que la longueur utile (L) et qui est adaptée pour se coupler de manière ablative à au moins une portion substantielle de la zone circonférentielle entourant seulement la portion intermédiaire de sorte que l'ablation de tissu se produit principalement le long de la zone de la région circonférentielle de tissu qui est engagée par la portion intermédiaire et est espacée des première et seconde extrémités de la longueur utile (L) dans la portion radialement expansée.

3. Ensemble de dispositifs d'ablation de tissu selon la revendication 1, dans lequel l'élément expansible comprend un ballonnet gonflable (820), et l'élément d'ablation a une seule bande circonférentielle disposée le long de l'axe longitudinal en une position coïncidant généralement avec la longueur utile (L) du ballonnet gonflable, la bande circonférentielle (352) étant adaptée pour se coupler à et être actionnée par un actionneur d'ablation (190) de sorte que l'élément d'ablation se couple de manière ablative à au moins une portion substantielle de la zone circonférentielle quand la longueur utile (L) est expansée radialement et engagée dans la région circonférentielle du tissu.

4. Ensemble de dispositifs d'ablation de tissu selon la revendication 1, dans lequel la longueur utile (L) de l'élément expansible est ajustable entre une position radialement rétractée et une pluralité de positions radialement expansées qui correspondent à une gamme de diamètres où la longueur utile (L) est adaptée pour engager chacune des multiples régions circonférentielles de tissu, respectivement ; et l'élément d'ablation est adapté dans chacune des positions radialement expansées pour se coupler de manière ablative à au moins une portion substantielle de la zone circonférentielle et est en conséquence adapté pour ablater un schéma circonférentiel transmural sensiblement continu dans chacune des multiples régions circonférentielles de tissu, respectivement, quand il est engagé par la longueur utile (L).

5. Ensemble de dispositifs d'ablation de tissu selon la revendication 1, dans lequel l'élément d'ablation est sensiblement espacé des extrémités de la longueur utile (L).

6. Ensemble de dispositifs d'ablation de tissu selon la revendication 2, dans lequel l'élément d'ablation a une bande circonférentielle (352) qui est située le long de l'axe longitudinal en une position qui coïncide généralement avec la portion intermédiaire et qui est sensiblement espacée des première et seconde extrémités, la bande circonférentielle ayant aussi une largeur de bande (W) le long de l'axe longitudinal qui est plus courte que la longueur utile (L).

7. Ensemble de dispositifs d'ablation de tissu selon la revendication 3, dans lequel la bande circonférentielle (352) a une largeur de bande (W) le long de l'axe longitudinal qui est plus courte que la longueur utile (L).

8. Ensemble de dispositifs d'ablation de tissu selon les revendications 2 ou 3, dans lequel le ballonnet gonflable (820) quand il est ajusté sur au moins une position radialement expansée est adapté pour se conformer à une veine pulmonaire (52).

9. Ensemble de dispositifs d'ablation de tissu selon les revendications 4 ou 5, dans lequel l'élément expansible quand il est ajusté sur au moins une position radialement expansée est adapté pour se conformer à une veine pulmonaire (52).

10. Ensemble de dispositifs d'ablation de tissu selon les revendications 8 ou 9, dans lequel la longueur utile (L) dans au moins une position radialement expansée a une région proximale (372) et une région distale (374) et une forme ayant un cône avec un diamètre extérieur se réduisant distalement depuis la région proximale (372) vers la région distale (374).

11. Ensemble de dispositifs d'ablation de tissu selon l'une quelconque des revendications précédentes, dans lequel la longueur utile (L) de l'élément expansible quand il est ajusté sur la position radialement expansée est adapté pour engager un ostium de veine pulmonaire (54).

12. Ensemble de dispositifs d'ablation de tissu selon la revendication 11, dans lequel la longueur utile (L) de l'élément expansible dans la position radialement expansée a une région proximale et une région distale et a aussi une forme conique avec un diamètre extérieur se réduisant distalement depuis la région proximale vers la région distale.

13. Ensemble de dispositifs d'ablation de tissu selon l'une quelconque des revendications 2, 3 ou 8, dans lequel le ballonnet gonflable est construit dans un matériau de ballonnet compliant.

14. Ensemble de dispositifs d'ablation de tissu selon les revendications 9 ou 11, dans lequel l'élément expansible comprend un ballonnet gonflable (820) qui forme une portion d'une chambre de ballonnet qui est adaptée pour se coupler de manière fluidique à une source de fluide pouvant être pressurisé (175), et le ballonnet gonflable (820) comprend un matériau de ballonnet compliant.

15. Ensemble de dispositifs d'ablation de tissu selon la revendication 14, dans lequel le ballonnet gonflable (820) est adapté pour se conformer à l'ostium d'une veine pulmonaire (54) quand ledit ballonnet gonflable est avancé dans la position radialement expansée de manière rétrograde depuis un atrium gauche dans la veine pulmonaire (52) et au moins partiellement contre l'ostium de veine pulmonaire (54).

16. Ensemble de dispositifs d'ablation de tissu selon l'une quelconque des revendications 2, 3, 8, 13, 14 et 15, dans lequel le matériau de ballonnet est choisi dans le groupe de matériaux se composant de silicone, caoutchouc de latex, polychlorure de vinyle, polyuréthane et des mélanges et combinaisons de ceux-ci.

17. Ensemble de dispositifs d'ablation de tissu selon les revendications 4 ou 5, dans lequel l'élément expansible (f.i. 470) comprend un panier.

18. Ensemble de dispositifs d'ablation de tissu selon les revendications 4 ou 5, dans lequel l'élément expansible (f.i. 470) comprend un ressort.

19. Ensemble de dispositifs d'ablation de tissu selon l'une quelconque des revendications précédentes, dans lequel l'élément d'ablation comprend une électrode d'ablation (655) qui est adaptée pour se coupler électriquement à une source de courant électrique (190) et aussi pour se coupler à au moins une portion substantielle de la zone circonférentielle.

20. Ensemble de dispositifs d'ablation de tissu selon la revendication 19, dans lequel l'électrode d'ablation (655) comprend en outre au moins un élément d'électrode positionné sur une surface extérieure de la longueur utile (L) et a une forme qui est adaptée pour former la bande circonférentielle (152, 352).

21. Ensemble de dispositifs d'ablation de tissu selon la revendication 19, dans lequel :
l'élément expansible forme une enveloppe extérieure d'une chambre de fluide conducteur qui est adaptée pour se coupler à une source pouvant être pressurisée (195) d'un fluide électriquement conducteur ;
la bande circonférentielle (352) est adaptée pour se coupler de manière ablative au fluide électriquement conducteur dans la chambre de fluide conducteur et à la région circonférentielle de tissu engagée dans la bande circonférentielle (352) dans la position radialement expansée ; et
l'électrode d'ablation (655) est adaptée pour se coupler électriquement au fluide électriquement conducteur dans la chambre de fluide conducteur et en conséquence pour aussi se coupler électriquement à au moins une portion substantielle de la région circonférentielle de tissu engagée par la bande circonférentielle (152, 352).

22. Ensemble de dispositifs d'ablation de tissu selon la revendication 21, dans lequel la bande circonférentielle (352) comprend en outre une membrane poreuse qui est adaptée pour passer le fluide électriquement conducteur depuis la chambre de fluide conducteur et dans la région circonférentielle de tissu engagée par la bande circonférentielle (352).

23. Ensemble de dispositifs d'ablation de tissu selon les revendications 19 ou 20, dans lequel la bande circonférentielle (352) comprend en outre une forme secondaire par rapport à l'axe longitudinal et a une longueur de bande circonférentielle par rapport à l'axe longitudinal, la forme secondaire étant adaptée de sorte que la largeur de bande circonférentielle (W) raccourcit alors que le diamètre extérieur expansé de la bande circonférentielle (352) augmente quand la longueur utile (L) est expansée depuis une première position radialement expansée vers une seconde position radialement expansée.

24. Ensemble de dispositifs d'ablation de tissu selon l'une quelconque des revendications 2 à 18, dans lequel l'élément d'ablation comprend en outre un conducteur thermique qui est adapté pour se coupler à un actionneur d'ablation thermique et aussi à la bande circonférentielle (352), de sorte que l'énergie thermique circule depuis le conducteur thermique et dans au moins une portion substantielle de la région circonférentielle de tissu engagée par la bande circonférentielle (352).

25. Ensemble de dispositifs d'ablation de tissu selon la revendication 24, dans lequel le conducteur thermique comprend en outre un conducteur électrique qui est adapté pour se coupler électriquement à une source de courant électrique (190) et aussi pour chauffer et émettre de manière résistive une énergie thermique dans la région circonférentielle de tissu engagée par la bande circonférentielle (352).

26. Ensemble de dispositifs d'ablation de tissu selon la revendication 24, dans lequel le conducteur thermique est adapté pour se coupler à une source d'énergie thermique et aussi pour être chauffé de manière conductive et de cette manière émettre une énergie thermique dans la région circonférentielle de tissu engagée par la bande circonférentielle (352).

27. Ensemble de dispositifs d'ablation de tissu selon la revendication 24, dans lequel l'élément d'ablation circonférentielle comprend en outre une chambre qui est adaptée pour se coupler de manière fluidique à une source pouvant être pressurisée (195) d'un fluide thermiquement conducteur, et un élément chauffant adapté pour se coupler thermiquement au fluide thermiquement conducteur et aussi à la région circonférentielle de tissu.

28. Ensemble de dispositifs d'ablation de tissu selon l'une quelconque des revendications 2 à 18, dans lequel l'élément d'ablation comprend en outre une antenne à hyperfréquences.

29. Ensemble de dispositifs d'ablation de tissu selon l'une quelconque des revendications 2 à 18, dans lequel l'élément d'ablation comprend en outre un élément cryogénique.

30. Ensemble de dispositifs d'ablation de tissu selon l'une quelconque des revendications 2 à 18, dans lequel l'élément d'ablation comprend en outre une membrane poreuse.

31. Ensemble de dispositifs d'ablation de tissu selon l'une quelconque des revendications 2 à 18, dans lequel l'élément d'ablation comprend en outre une applicateur d'énergie ultrasonore (840).

32. Ensemble de dispositifs d'ablation de tissu selon l'une quelconque des revendications 19 et 28 à 31, dans lequel l'élément d'ablation circonférentielle comprend en outre :
un premier blindage, un second blindage et une région de milieu s'étendant entre les premier et second blindages par rapport à l'axe longitudinal et qui englobe au moins en partie l'élément d'ablation,
les premier et second blindages étant adaptés pour engager des première et seconde régions de tissu adjacentes sur des côtés opposés de la zone circonférentielle de tissu et pour protéger les première et seconde régions de tissu adjacentes d'un couplage à l'élément d'ablation pour de cette manière isoler l'énergie ablative de la région de milieu et à la région circonférentielle de tissu.

33. Ensemble de dispositifs d'ablation de tissu selon les revendications 31 ou 32, dans lequel l'applicateur d'énergie ultrasonore (840) et l'élément expansible ont une taille adaptée pour être insérés dans un ostium de veine pulmonaire (54).

34. Ensemble de dispositifs d'ablation de tissu selon les revendications 31, 32 ou 33, dans lequel l'applicateur d'énergie ultrasonore (840) comprend en outre un seul transducteur ultrasonore cylindrique (830).

35. Ensemble de dispositifs d'ablation de tissu selon l'une quelconque des revendications précédentes, dans lequel une longueur de l'élément d'ablation le long de l'axe longitudinal est inférieure à deux tiers de la longueur utile (L) .

36. Ensemble de dispositifs d'ablation de tissu selon l'une quelconque des revendications précédentes, dans lequel l'élément d'ablation dans la position radialement expansée a une circonférence et la longueur de l'élément d'ablation le long de l'axe longitudinal est inférieure à deux tiers de la circonférence de l'élément d'ablation.

37. Ensemble de dispositifs d'ablation de tissu selon la revendication 1, dans lequel l'élément d'ablation circonférentielle comprend en outre une ancre qui est adaptée pour fixer l'élément d'ablation circonférentielle en l'endroit.

38. Ensemble de dispositifs d'ablation de tissu selon la revendication 1, dans lequel l'élément d'ablation circonférentielle a un élément en boucle avec une forme qui est adaptée pour entrer en contact avec la région circonférentielle de tissu, et un élément d'ablation positionné le long de la région formée.

39. Ensemble de dispositifs d'ablation de tissu selon la revendication 38, dans lequel l'élément en boucle peut se rétracter dans un passage de libération de cathéter de l'ensemble de libération (101).

40. Ensemble de dispositifs d'ablation de tissu selon les revendications 38 ou 39, dans lequel l'élément en boucle est ajustable depuis une première configuration, qui est adaptée pour être libérée à travers un passage de libération de cathéter de l'ensemble de libération (101) dans l'atrium gauche, vers une seconde configuration qui est adaptée pour entrer en contact avec au moins une portion substantielle de la région circonférentielle de tissu.

41. Ensemble de dispositifs d'ablation de tissu selon la revendication 1, dans lequel la portion d'extrémité distale du corps allongé comprend un élément de guidage de fil-guide qui est adapté pour engager de manière coulissante et guider sur un fil-guide (102) de l'ensemble de libération qui est positionné au moins partiellement dans la veine pulmonaire (52) et à travers l'ostium de veine pulmonaire (54) de sorte que l'élément d'ablation circonférentielle puisse être positionné dans la veine pulmonaire (52) et que l'élément d'ablation puisse être couplé de manière ablative à au moins une portion substantielle de la région circonférentielle de tissu.

42. Ensemble de dispositifs d'ablation de tissu selon les revendications 1 ou 41, dans lequel l'ensemble de libération (101) comprend en outre un sous-ensemble de cathéter de guidage ayant un cathéter de guidage avec une lumière intérieure se terminant en un bout distal qui est adapté pour être positionné dans l'atrium gauche et pour libérer la portion d'extrémité distale du corps allongé comprenant l'élément d'ablation circonférentielle dans l'atrium gauche à travers la lumière intérieure.

43. Ensemble de dispositifs d'ablation de tissu selon la revendication 42, dans lequel l'ensemble de cathéter de guidage est adapté pour positionner le cathéter de guidage (101) dans un atrium droit et d'un côté à l'autre d'une fossa ovalis de sorte que le bout distal du cathéter de guidage (101) est dans l'atrium gauche et en outre de sorte que le cathéter de guidage (101) est adapté pour libérer la portion d'extrémité distale du corps allongé comprenant l'élément d'ablation circonférentielle de manière transseptale dans l'atrium gauche.

44. Ensemble de dispositifs d'ablation de tissu selon l'une quelconque des revendications 1, 42 ou 43, dans lequel le corps allongé comprend un fil fixe intégré dans le corps du cathéter.

45. Ensemble de dispositifs d'ablation de tissu selon l'une quelconque des revendications 1, 42, 43 ou 44, dans lequel le corps allongé comprend une lumière de fil de traction et un fil de traction fixe associé qui est adaptée pour déformer le bout de cathéter en appliquant une tension le long de transitions de raideur variée le long de la longueur du corps allongé.

46. Ensemble de dispositifs d'ablation de tissu selon l'une quelconque des revendications 1 et 41 à 45, dans lequel le corps allongé comprend une lumière de perfusion (260) située en la portion d'extrémité distale et définie entre un orifice d'extrémité distale (242) et un orifice d'extrémité proximale (244), l'orifice d'extrémité distale situé distalement par rapport à l'élément d'ablation et l'orifice d'extrémité proximale situé proximalement par rapport à l'élément d'ablation.

47. Ensemble de dispositifs d'ablation de tissu selon l'une quelconque des revendications 1 et 41 à 45, dans lequel la portion d'extrémité distale du corps allongé (802) comprend en outre une région formée avec une forme qui est adaptée pour positionner l'élément d'ablation circonférentielle le long de la zone circonférentielle de tissu.

48. Ensemble de dispositifs d'ablation de tissu selon la revendication 47, dans lequel la région formée est ajustable depuis une première configuration, qui est sensiblement droite et non formée, vers une seconde configuration ayant la forme.

49. Ensemble de dispositifs d'ablation de tissu selon la revendication 47, dans lequel l'élément de libération (101) comprend en outre un élément déflecteur couplé à la région formée et qui est adapté pour ajuster la région formée entre les première et seconde configurations.

50. Ensemble de dispositifs d'ablation de tissu selon l'une quelconque des revendications 41 à 49, **caractérisé en outre par** un élément d'ablation linéaire (460) fixé à la portion d'extrémité distale de l'élément allongé et ayant un élément d'ablation linéaire qui est adapté pour se coupler à un actionneur d'ablation (190), et l'élément d'ablation linéaire est adapté pour engager une région de tissu de paroi atriale s'étendant depuis la veine pulmonaire de sorte que l'élément d'ablation linéaire (460) peut être couplé à et ablater une lésion continue le long de la région de tissu de paroi atriale.

51. Kit comprenant une pluralité d'ensembles de dispositifs d'ablation circonférentielle comme dans l'une quelconque des revendications précédentes, **caractérisé en outre en ce que** chaque ensemble de dispositifs d'ablation de tissu comprend un élément expansible avec un diamètre extérieur expansé différent des autres éléments expansibles des autres ensembles de dispositifs d'ablation circonférentielle de tissu dans le kit.
